(19) Europäisches Patentamt
European Patent Office
Office européen des brevets

(11) Publication number: **0 356 021 A2**

# (12) EUROPEAN PATENT APPLICATION

(21) Application number: **89307672.9**

(51) Int. Cl.⁴: **C12Q 1/68 , //C07H21/00**

(22) Date of filing: **27.07.89**

(30) Priority: **28.07.88 GB 8818020**

(43) Date of publication of application: **28.02.90 Bulletin 90/09**

(84) Designated Contracting States: **AT BE CH DE ES FR GB GR IT LI LU NL SE**

(71) Applicant: **IMPERIAL CHEMICAL INDUSTRIES PLC**
**Imperial Chemical House Millbank**
**London SW1P 3JF(GB)**

(72) Inventor: **Markham, Alexander Fred**
**25 Booth Bed Lane**
**Goostrey Crewe Cheshire(GB)**
Inventor: **Smith, John Craig**
**14 Ascol Drive**
**Plumley Nr Knutsford Cheshire(GB)**
Inventor: **Anwar, Rashida**
**34, Shelley Avenue**
**Wincham Northwich Cheshire(GB)**

(74) Representative: **Mack, John Richard et al**
**Imperial Chemical Industries PLC Legal Department: Patents PO Box 6 Bessemer Road**
**Welwyn Garden City Hertfordshire AL7 1HD(GB)**

## (54) A method for the amplification of nucleotide sequences.

(57) A method for the amplification of a nucleic acid fragment containing unknown sequence and kits therefor are provided which enable long nucleotide sequences to be rapidly and efficiently sequenced. The method comprises the formation of target nucleic acid fragment/vectorette units by cleavage of target nucleic acid followed by ligation. One of the nucleic acid fragments will contain an initiating priming region of known sequence for hybridisation with an initiating primer and target nucleic acid fragment/vectorette units will contain a vectorette priming region of known sequence for hybridisation with a vectorette primer. Amplification is effected by primer extension of an initiating primer hybridised to the initiating priming region of the target nucleic acid fragment/vectorette unit.

EP 0 356 021 A2

# A METHOD FOR THE AMPLIFICATION OF NUCLEOTIDE SEQUENCES

The present invention relates to a method for the amplification of nucleotide sequences and kits therefor. Such a method is of particular interest in relation to the amplification of sequences only a portion of which is known and enables long nucleotide sequences to be rapidly and efficiently sequenced. The method avoids the recombinant DNA cloning procedures hitherto necessary for the sequencing of unknown nucleotide sequences. By so doing it also allows polymorphisms between nucleotide sequences of different alleles at a genetic locus to be detected as well as simultaneous analysis of alleles at a particular locus in different individuals.

Nucleotides may exist as individual nucleotides or base pairs (hereinafter also referred to as bp) or in strands of nucleotides, each strand containing up to $10^8$ bp or even more. The human genome, for example is believed to contain approximately 3 x $10^9$ bp, a single chromosome containing approximately $10^7$-$10^8$ bp. Techniques for sequencing relatively short nucleotide sequences have existed for many years and include the method of Maxam and Gilbert [Maxam A.M. Gilbert W (1977) "A new method for sequencing DNA". Proc. Natl.Acad. Sci., USA 74, 560-564 and "Sequencing end-labelled DNA with base-specific chemical cleavages" Methods in Enzymology 65, 499-560 (1980)] in which single-stranded DNA radiolabelled at one end, for example with $^{32}P$, is subjected to several chemical cleavage protocols (for example with dimethylsulphate or hydrazine) that selectively make breaks on one side of a particular nucleoside. The fragments obtained are separated according to size by electrophoresis on acrylamide gels and identified by autoradiography. Relatively short nucleotide sequences may also be determined by the enzymatic "dideoxy" technique of Sanger et al [Proc.Natl.Acad.Sci, USA 74, 5463-7, (1977)] in which the Klenow fragment of DNA polymerase I or T7 DNA polymerase or Taq DNA polymerase is used to synthesize a complementary copy of the single-stranded target sequence in the presence of the four deoxynucleoside triphosphates, one or more of which is radiolabelled, for example with $^{32}P$ and in four separate incubation mixes containing a low concentration of one each of the four dideoxynucleoside triphosphates. A population of truncated radioactive DNA molecules, each having a common 5′ end, but each varying in length to a base-specific 3′-end is thus obtained in each reaction. Following appropriate incubation the DNA in each mixture may be denatured, electrophoresed side by side, and the radioactive bands of single-stranded DNA detected by autoradiography. The sequence of the target DNA may then be read off directly from the autoradiograph. Furthermore E.I. Du Pont de Nemours & Company (DuPont) have recently placed on the market an automated instrument for sequencing DNA strands which embodies a modification of the above-mentioned "dideoxy" technique. This modification involves the use of the four dideoxynucleoside triphosphate terminators tagged with fluorescent dyes, each dideoxy chain terminator emitting light of a slightly different wavelength then exoited by an argon laser [Science, 238, 336 (1987). All four dideoxynucleoside triphosphate terminators may be used in the same pot since the terminators can be distinguished by their emission spectra. The resulting mixture of DNA fragments may be electrophoresed in a single lane of a polyacrylamide gel. The sequence of the target DNA may then be read automatically since the identity of the nucleotide terminating each band on the gel is determined by its characteristic fluorescent emission.

It has been estimated that the DuPont sequencer is capable of identifying about 10,000 nucleotides a day under optimum conditions in contrast to an average of about 50,000 nucleotides a year which is estimated for a skilled worker using the unmodified manual "dideoxy" technique. Whilst this is a substantial improvement in the speed and efficiency of determining relatively short nucleotide sequences, the rate determining step in the determination of relatively long sequences, for example genomic sequences, is dependent on the step colloquially known as "chromosome walking". "Chromosome walking" is a technique which comprises the sequential isolation of clones carrying overlapping fragments to span a segment of for example a chromosome that is larger than can be carried in a phage or a cosmid or a yeast artificial chromosome (YAC) vector. This technique thus allows isolation of a locus of interest for which no probe is available and is of particular use where the locus of interest is known to be linked to a locus which has been identified and cloned such as a gene or DNA marker. The locus which has been identified may then be employed as a probe to screen a genomic library and will hybridise with any fragment containing complementary nucleotide sequences and which therefore represent overlapping clones. Such overlapping sequences may be in either the 5′ or the 3′-sense. A fragment identified as representing an overlapping clone may then itself be used as a probe to rescreen the genomic library and hybridise with any fragments containing complementary nucleotide sequences which thus represent further overlapping clones. By repetition of this process the nucleotide sequences of regions further and further away from the originally identified locus may be determined until eventually the locus of interest is encountered.

The technique of "chromosome walking" involves a number of potential difficulties as is exemplified by the time taken from discovery of a marker for a genetic disorder to discovery of the specific genetic lesion responsible for the disorder. Thus, for example, a linked genetic marker for Huntington's Chorea (D4S1) was discovered in 1983, but still today the specific genetic lesion responsible for this disorder is not known. Similar comments apply to many other genetic disorders.

The technique of "chromosome walking" particularly suffers from the disadvantage that cloning of genomic DNA is a prerequisite. In a number of circumstances cloning may prove impossible or at least very difficult and in such situations the "chromosome walk" comes to a premature end; A R Wyman and K F Wertman, in Methods in Enzymology, Vol 152, Guide to Molecular Cloning Techniques, S L Berger and A R Kummel, editors Academic Press, San Diego, 1987 pages 173-180. Moreover the analysis of the fragments identified as representing overlapping clones is complex in view of inter alia the number of such fragments which may be located in any one screening of the genomic library and the fact that the overlapping sequences may be in either the 5′ or the 3′ sense.

A further serious practical problem with "chromosome walking" is the widespread occurrence of repetitive sequences within the human and other genomes. Thus if the identified locus contains such repetitive elements all such elements within the human genome may be identified incorrectly as overlapping clones. Analysis then becomes extremely complex.

Furthermore any overlapping clones obtained by "chromosome walking" will, when sequenced as hereinbefore described, generate sequence information derived from a single cloned allele from the identified locus. In the context of studying the important genetic differences between individual members of a species and their relation to an individual's phenotype it would be advantageous if more than a single allele could be analysed and characterised simultaneously.

The present invention is based upon the discovery of a method which obviates, at least in part, the above-mentioned difficulties by amplifying fragments obtained via cleavage of target DNA at specific sites outside known sequences, thus overcoming the need for cloning.

Thus according to one feature of the present invention there is provided a method for the amplification of a nucleic acid fragment, comprising unknown sequence, by primer extension which method comprises cleaving a target nucleic acid to obtain target nucleic acid fragments, one of said fragments containing an initiating priming region of known nucleotide sequence for hybridisation with an initiating primer, preparing target nucleic acid fragment/vectorette units from the target nucleic acid fragments by ligation each unit having a vectorette priming region of known sequence for hybridisation with a vectorette primer, and treating the target nucleic acid fragment/vectorette units, together or sequentially, with appropriate nucleoside triphosphates and an agent for polymerisation of the nucleoside triphosphates under hybridising conditions, such that an extension product of an initiating primer is synthesised complementary to a single stranded target nucleic acid/vectorette unit having an initiating priming region to which is hybridised an initiating primer selected so as to be substantially complementary to the initiating priming region, whereas no such extension product is synthesised complementary to single stranded target nucleic acid fragment/vectorette units having no such initiating priming region.

If desired the said extension product may be subjected to amplification in the presence of a vectorette primer which is selected so as to be substantially complementary to the vectorette priming region.

The target nucleic acid fragment/vectorette units are thus treated with initiating primer and, if the initiating primer extension product is to be amplified for example as described by R.K. Saiki et al, Science 239, 487-491 (1987), additionally treated with vectorette primer. Where no vectorette primer is used, arithmetical or linear amplifcation (hereinafter referred to as linear amplification) may be achieved by hybridisation of the initiating primer to the initiating priming region followed by primer extension in the presence of appropriate nucleoside triphosphates and an agent for polymerisation of the nucleoside triphosphates, under hybridising conditions and denaturation. This process of priming, primer extension and denaturation may be repeated as many times as appropriate to achieve the desired level of amplification. Preferably, however, amplification is effected in the presence of both initiating and vectorette primers by the use of the PCR technique referred to above and as hereinafter defined.

According to a further feature of the present invention there is provided a kit for the amplification of a nucleic acid fragment of unknown sequence by primer extension, which kit comprises:-

1) means for cleaving a target nucleic acid at a specific site to obtain a target nucleic acid fragment;

2) a vectorette adapted for ligation to a target nucleic acid fragment obtained by use of the means for cleaving a target nucleic acid defined in (1) whereby to form in use a target nucleic acid fragment/vectorette unit said vectorette having a vectorette priming region of known sequence for hybridisation with a vectorette primer;

3) each of four different nucleoside triphosphates; and

4) an agent for polymerisation of the nucleoside triphosphates in (3).

A vectorette priming region may be present or absent from the vectorette portion of a target nucleic acid fragment/vectorette unit as hereinafter defined. Thus the vectorette (2) in the kit of the present invention may itself contain no vectorette priming region provided that in use a target nucleic acid fragment/vectorette unit is formed in which the vectorette portion thereof contains a vectorette priming region. Thus such units may for example either have a vectorette priming region in the vectorette portion of the target nucleic acid fragment/vectorette unit as formed by ligation or have a vectorette priming region which only arises as a result of primer extension of an initiating primer as described hereinafter. Reference throughout the specification to "target nucleic acid fragment/vectorette unit" (also termed "vectorette unit") as hereinafter defined are to be so understood.

Preferably the kit will additionally comprise a vectorette primer having a nucleotide sequence substantially complementary to the vectorette priming region of the target nucleic acid fragment/vectorette unit. The presence of such a vectorette primer in the kit of the present invention will enable amplification of the target nucleic acid fragment/vectorette unit to be effected by PCR techniques (as hereinafter defined) if this is desired.

The kit may more preferably also contain a series of "nested vectorette primers (as hereinafter defined) which may be used, for example, for secondary amplification reactions where necessary or desired and/or for direct sequencing of the products of amplification as has been described in Proc Natl. Acad. Sci. USA 85, 7652-7656 (1988) by U B Gyllenstein and H Ehrlich. It will be appreciated that all such vectorette primers may well be useful as direct sequencing primers for the distal ends of fragments obtained by linear amplification using an initiating primer alone.

Since the target nucleic acid to be investigated will normally be peculiar to the use of the kit, initiating primer(s) will not normally be present in the kit, but may be prepared by the user of the kit.

If desired however the kit of the present invention may additionally contain initiating primer(s) and also, if desired, nested initiating primer(s).

The kit of the present invention may also advantageously contain buffers for performing the method of the invention, a particular feature of the kit being for example the presence of buffers for varying the potassium, magnesium and nucleoside triphosphate(s) concentrations of the reaction mixture. These latter buffers may be desirable for determining the optimum conditions for subsequent cycles of the method of the invention.

Advantageously the kit will comprise more than one (a plurality of) means for cleaving a target nucleic acid each at specific sites. Where a plurality of such means are present, the kit will normally comprise a different vectorette for each such means present in order to permit formation of target nucleic acid fragment/vectorette units in respect of each set of target nucleic acid fragments which may be obtained. Although the invention is not limited to this application, the plurality of different vectorettes will advantageously share sequences complementary to a vectorette primer. In such cases a single vectorette primer will provide amplification from a plurality of target nucleic acid cleavage sites.

Thus in a preferred embodiment the kit of the present invention additionally contains one or more selected from the following:- initiating primer(s), nested vectorette primer(s), nested initiating primer(s), sequencing primers, buffers for performing the method of the present invention, and buffers for varying the magnesium, potassium and nucleoside triphosphate concentrations.

According to a further feature of the present invention there is provided a vectorette library kit for the amplification of a nucleic acid fragment of unknown sequence by primer extension, which kit comprises:-

1) at least one vectorette library each library comprising a set of target nucleic acid fragment/vectorette units obtained from nucleotide sequences of an individual member of a species of animal, plant or microorganism; and

2) an initiating primer or primers for hybridisation to the initiating priming region of the target nucleic acid fragment/vectorette units.

The vectorette library kit will preferably comprise a plurality of vectorette libraries.

The target nucleic acid fragment/vectorette units may be prepared for example either directly from the desired species or indirectly from such a species after initial cloning in plasmid, phage, cosmid or yeast artificial chromosome (YAC) vectors. The species of animal, plant or microorganism employed is preferably human, but may be any other animal species, plant or microorganism such as bacteria, viruses, yeast or parasites. The nucleotide sequences are preferably from genomic DNA, but may be from sorted chromosomes or clones.

The vectorette units employed may be either derived from a single cleavage method and/or multiple vectorette units derived from multiple cleavage methods, which together or separately may constitute a vectorette library or plurality of vectorette libraries (as hereinafter defined).

The target nucleic acid fragments employed may be derived from a number of different sources. Thus for example the target nucleic acid fragments may be obtained from single individuals of a species of animal, plant or microorganism, for example from individuals typical of their species. The target nucleic acid fragments may also be obtained from single individuals known to be heterozygous for a given genetic locus for example a locus causing cystic fibrosis or other inherited disease. The target nucleic acid fragments may also be obtained from single individuals known to be homozygous for a given genetic locus for example cystic fibrosis or other inherited disease. The target nucleic acid fragments may also be obtained from single individuals known to be normal homozygotes for a given genetic locus for example cystic fibrosis or other inherited disease. The target nucleic acid fragments may also be obtained from groups of individuals (as opposed to single individuals) with a shared phenotype(s). The nucleic acid or tissue from each member of a group which shares a phenotype may if desired by pooled. Each group of individuals will consist of at least 2 and advantageously less than 1000, for example 50-500. Vectorette units may be prepared from the target nucleic acid fragments and the vectorette units pooled or used separately to form vectorette libraries. The shared phenotype may if desired be a disease or disease predisposition, obligate carriage or an inherited disease or a normal state with no evidence of the disease or disease predisposition.

Comparison of nucelotide sequences obtained using the method of the invention will identify any common genetic variants in the population which are "associated' with for example a given disease or disease predisposition. It will be appreciated that this extends considerably the scope for detailed analysis over and above that previously attempted using RFLP technology.

The vectorette library kit of the present invention preferably additionally contains vectorette primer(s), and advantageously one or more selected from nested intitiating primer(s), nested vectorette primer(s) and sequencing primers. The vectorette library kit may also conveniently contain each of four different nucleoside triphosphates and an agent for polymerisation of the nucleoside triphosphates. If desired the vectorette library kit may further contain buffers for performing the invention and/or buffers for varying the magnesium, potassium and nucleoside triphosphate concentrations. These latter buffers may be desirable for determining the optimum conditions for subsequent cycles of the method of the invention.

The present invention is of wide applicability. Thus for example the present invnetion may be employed to identify nucleotide sequences characteristic of a particular microorganism such as a microorganism responsible for disease in plants or animals, particularly humans, such microorganism being for example a fungus, yeast, bacterium or virus as well as a parasite (such as plasmodium (malaria) or trypanosome (sleeping sickness)). The method of the present invention may also be employed to identify nucleotide sequences responsible for drug, for example antibiotic, resistance in a given organism. Thus the present invention enables the production of probes diagnostic of disease in animals or plants and additionally enables the production of probes diagnostic of drug, for example antibiotic, resistance.

The present invention may also be employed for example 1) to detect nucleotide sequence variations, for example point mutations, responsible for genetic disorders in plants, but especially in animals, particularly humans; 2) to detect nucleotide sequence variations, for example deletions, responsible for neoplastic disease in animals, particularly humans; 3) to detect nucleotide sequence variations responsible for predispositions to disease or disorders in plants but especially in animals, particularly humans; and 4) to detect nucleotide sequence variations responsible for a specific characteristic such as a desirable characteristic, for example in plants such as flower colour, crop yield or herbicide resistance.

The present invention may also be employed in animal studies, for example, in the monitoring of transgenic animals.

Furthermore, the present invention is of particular interest in the sequencing of an animal genome, particularly the human genome for example to identify hitherto unknown polypeptides capable of production in vivo particularly in the human and which polypeptides may for example have therapeutic interest. The present invention may also be of interest in relation to the production of pharmaceutical proteins for example for sequencing regulatory regions of genes or expression systems.

In the context of sequencing large genomes, for example the human genome, it is widely accepted that the major currently perceived limitation is the preparation of physical maps comprising overlapping "contigs" of isolated cosmid clones. The current strategies for such mapping projects have been reviewed in "Mapping our Genes" Genome Projects: How Big, How Fast?" Congress of the United States, Office of Technology Assessment, The Johns Hopkins University Press, Baltimore and London (1988). Further detail is provided in "Mapping the Human Genome: Experimental Approaches for Cloning and Ordering DNA Fragments", R M Myer, Department of Physiology, University of California, San Francisco, USA in "Mapping our Genes Contractor Reports, Vol 2, Order No. PB 88-162 806/AS available under the auspices of the US OTA from the National Technical Information Service (NTIS) 5285 Port Royal Road, Springfield, VA22161, USA. An approach to mapping the much smaller E.coli genome is taught in C L Smith et al -

(Science, 236, 1448-1453. 1987) and Y Kohara et al (Cell, 50, 495-508, 1987).

The present invention may also be employed for example to sequence large nucleotide fragments purified by cloning in cosmids and YACs (yeast artificial chromosomes). Cosmids can be used to clone nucleotide fragments of for example up to about 45kb and YACs may be used to clone fragments of an average size of over 300 kb (Nucleic Acids Research 17, 3425-3433 (1989) R Anand et al). The applicability of the present invntion ot the sequencing of such large nucleotide fragments enables the rapid sequencing of very large nucleotide sequences.

A particularly advantageous aspect of the present invention is that sequence analysis performed using the method of the invention does not involve cloning of the target nucleic acid. In practice this means that all alleles present in the target nucleic acid sample may be analysed at the same time. The advantages of this approach for the identification of heterozygotes as well as homozygous normals and mutants have been described previously where the direct sequencing of PCR products has been used to analyse mutations (for example point mutations) causing inherted diseases. (Nucleic Acids Res., 16, 8233-8243 (1988) by C R Newton et al). A point mutation is easily visualised in the heterozygote because comigrating bands corresponding to the 2 different bases appear in two lanes of "dideoxy" sequencing gel. The homozygotes (normal or mutant) at this base pair show a single band corresponding to one base or the other at this position on a sequencing gel.

Previously, a prerequisite for this type of PCR-based sequence analysis has been that the sequence of the region of interest has been determined in advance by methods known per se. This allows PCR primers to be designed for amplification and subsequent repetitive sequencing of the region of interest. The present invention allows such analysis to be undertaken with target nucleic acid fragments whose sequence is not already fully established. Sequencing as performed using the method of the invention on for example human genomic DNA from an individual reveals the full extent of polymorphic differences between the diploid chromosomes. If the individual is known to be an obligate heterozygote for an unknown mutation at a given genetic locus then sequencing at both alleles (preferably simultaneous sequencing) by the method of the invention will inevitably reveal all the polymorphic bases present which could therefore be causes of an observed phenotype. The importance of individual polymorphic base changes may be confirmed or disproved by subsequent study of the same target nucleic acid sequences in known homozygous normals and mutants (as well as in other obligate heterozygotes). It will furthermore be appreciated that the present invention represents a considerable advance over current RFLP (restriction fragment length polymorphism) based approaches to the study of variations between chromosomes. In that case, only those base changes which infrequently create or destroy a restriction endonuclease recognition site are detected. Techniques are available for the facile analysis of point mutations which do not result in RFLP (Nucleic Acids Res., 17 No 7 pages 2503-2516 (1989) by C R Newton et al).

A further feature of the present invention is its utility in the analysis of polygenetic or multifactoral diseases. Thus, for example, it would be advantageous to establish whether particular polymorphic variants at certain genetic loci are indicative of a predisposition to develop such multifactoral polygenetic diseases as atherosclerosis, hypertension, diabetes, schizophrenia and the like. Attempts to approach this problem previously using RFLP techniques have proved generally unsuccessful. This is probably not surprising as such studies have relied on the examination of particular polymorphisms displayed on large numbers of labour-intensive Southern blots by individual candidate gene probes. Thus the range of the total polymorphism in the human genome actually analysed for linkage to disease predisposition has been in fact vanishingly small. (see "Molecular Approaches to Human Polygenic Disease", Ciba Foundation Symposium 130, John Wiley, Chichester 1987). Using the present invention it becomes possible to mix target nucleic acids from a number of individuals with a given disease, disease predisposition or other phenotype. Vectorette libraries as hereinafter defined may be constructed from such pooled target nucleic acids, for example pooled human genomic DNA. The present invention allows this pooled genomic DNA to be sequenced in a processive manner. Common polymorphisms will become apparent in a similar fashion to the observations with a single heterozygote. Again the lack of any need for recombinant DNA cloning means that all alleles present in the pooled genomic material may be analysed simultaneously. The number of individual samples present in the pool is not limited. Use of the method of the present invention simply reveals a concensus genotype plus polymorphism. A comparison of results thus obtained with similar results obtained by analysis of pooled genomic DNA from individuals who do not manifest the given disease, disease predisposition or other phenotype will allow polymorphisms associated with the disease or phenotype (or conversely with a lack of the disease or phenotype) to be identified. The analysis is not limited to the small area of the human genome close to candidate genes. This type of analysis using the method of the invention may be further facilitated by combining the amplified products obtained with a given initiating primer and a particular vectorette library made with target nucleic acid from either a

"normal" or an "affected" pooled population of individuals. If there are indeed gross polymorphic differences between the populations then mixing the amplified products, denaturing and re-annealing would generate double-stranded amplification products with mismatched base pairs. Such mismatches may be revealed using reagents such as OsO4 or hydroxylamine as taught by (R G H Cotton, N R Rodrigues and R D Campbell PNAS, 4397-4401 1988)

A glossary of certain terms used herein is set out below in order to assist the reader of the present specification.

The term "target nucleic acid" refers to a nucleotide sequence, generally genomic DNA, for example plant or animal genomic DNA such as human DNA or bacterial DNA. Such a "target nucleic acid" for use in the method of the present invention will normally comprise a portion (generally a small portion) of known sequence and generally a much larger portion of unknown sequence.

The term "target nucleic acid fragment" is used herein to mean a fragment of a target nucelic acid obtained by cleaving (as hereinafter defined) target nucleic acid. The term "target nucleic acid fragment" therefore is not limited to such a fragment obtained by the use of a restriction endonuclease. Furthermore such a fragment may for example comprise a portion of known sequence and generally a larger portion of unknown sequence, or the fragment may be of unknown sequence. Where the fragment is of unknown sequence, cleavage and generation of target nucelic acid fragment/vectorette units may be performed as described hereinafter. In such circumstances random DNA probes may be generated from the much larger genomic fragment by linear amplification with a "random" initiating primer or amplification with a random initiating primer plus vectorette primer. Specific amplification will generate fragments at random which can be purified and used in genetic mapping as in Cell 51, 319-337 (1987) H Donis-Keller et al.

The term "amplification" is used herein to refer to the replication of a nucleotide sequence and/or its complementary sequence by non-biological means and thus includes amplification by the use of an initiating primer alone such primer being hybridised to the initiating priming region of a target nucleic acid fragment/vectorette unit, primer extension being effected in the presence of appropriate nucleoside triphosphates and an agent for the polymerisation of the nucleoside triphosphates under hybridising conditions, such primer extension being followed by denaturation, this process of priming, primer extension and denaturation being repeated as many times as appropriate to achieve a desired level of amplification. The term "amplification" also includes replication by polymerase chain reaction (PCR) techniques as for example described by R.K. Saiki et al, in Science 239, 487-491 (1987) and in U.S. Patents Nos 4,683,195 and 4,683,202 using an initiating primer and a vectorette primer as hereinafter defined and the expression polymerase chain reaction technique or PCR technique as used herein to refer to such techniques as described in these references.

The term "non-biological" is used herein only to exclude amplification by direct cloning and propagation of bacterial colonies. It will therefore be appreciated that the term amplification is used herein to include amplification processes such as those described in PCT Patent Publication WO 87/06270 (or Biotechnology Vol 6, October 1988), PCT Patent Publication WO88/10315 or PCT Patent Publication WO 89/01050.

The term "cleaving " is used herein to refer to cleavage of a nucleic acid at a specific site. Such cleavage is conveniently effected using a restriction endonuclease, preferably a 6bp cutter, having a known recognition sequence and cleavage pattern and thus the characteristic of cleaving DNA at specific sites.

In this regard the position of these "specific sites" in a given target nucleic acid will not normally be known relative to the position of another known element in the target nucleic acid, but the sequence of the specific sites will be known as will their cleavage patterns. Thus the terminal sequences of the restriction fragments obtained will be known. Thus for example the restriction endonuclease EcoRI recognises the sequence:-

```
-G|AATTC-              -G            +  AATTC-
-CTTAA|G-  ---->       -CTTAA               G-
```

and cleaves such a sequence as indicated to yield restriction fragments having the cohesive ends indicated. Since the sequence of the cohesive end is known it is possible to produce target nucleic acid fragment/vectorette units on the basis of this knowledge as described hereinafter in relation to the expression "target nucleic acid fragment/vectorette unit". Means for cleaving a target nucleic acid at a specific site other than restriction endonucleases may be employed, but the use of a restriction endonuclease is preferred. Any convenient restriction endonuclease may be used. Examples of individual restriction endonuclease include those detailed in Nucleic Acids Research, Sequences Supplement, Volume

16, 1988 page r271-r313 and in "Current Protocols in Molecular Biology 1987-1988, Edited by Ausubel F.M., Brent R., Kingston R.E., Moor D.D, Smith J.A., Seidman J.G. and Struhl K., Wiley Interscience, Section 3, Table 3.1-1. Restriction endonucleases capable of producing fragments with cohesive ends such as EcoRI, Hind III and XbaI are convenient regardless of whether the cohesive ends have 5′ or 3′ overhangs. It will also be appreciated that as well as by using restriction endonucleases capable of producing fragments with either a 3′ or a 5′ overhanging cohesive end the production of target nucleic acid fragment/vectorette units is also possible using restriction endonucleases producing blunt-ended fragments in conjunction with appropriate blunt-ended vectorettes as hereinafter defined. Means for cleaving a target nucleic acid at (a) specific site(s) other than by standard restriction endonuclease digestion are known in the art and include, for example, the use of adapters-primers and Class-IIS restriction enzymes (S C Kim et al, Science, 240, 504-506 (1988); W Szybalski, Gene, 40, 169 (1985); A J Podhajska and W Szybalski, Gene, 40, 175 (1985)) as well as various chemical approaches (B L Iverson and P B Dervan, J. Amer. Chem. Soc., 109, 1241-1243 (1987); G B Dreyer and P B Dervan, Proc. Natl. Acad. Sci USA, 82, 968 (1985); V V Vlassov et al, Nucleic Acids Res., 14, 4065 (1986); H E Moser and P B Dervan, Science, 238, 645 (1987); D R Corey and P G Schultz, Science, 238, 1401 (1987); J P Sluka et al, Science, 238, 1129 (1987)).

Furthermore, it is possible to render target nucleic acid fragments blunt-ended, either by DNA polymerase mediated fill-in /repair of any 5′ overhanging cohesive ends or by removal of 3′ and/or 5′ overhangs using S1 nuclease mediated single strand digestion, irrespective of whether they are generated by restriction enzyme digestion or other (eg chemical)means. All such blunt-ended fragments may again be converted into target nucleic acid fragment/vectorette units by attachment of the appropriate blunt-ended vectorette.

The expression "initiating priming region" as used herein means that portion of a cleaved, for example restriction enzyme digested, target nucleic acid fragment which is of known nucleotide sequence and to which in use an initiating primer and if desired nested initiating primer(s) (as hereinafter defined) for example overlapping nested initiating primer(s) may hybridise. In general the method of the present invention will be effected such that only one of the target nucleic acid fragments present will have an initiating priming region. An exception would be the use of a mixture of a plurality of a different vectorette libraries as hereinafter defined.

The expression "vectorette priming region" as used herein means that portion of the target nucleic acid fragment/vectorette unit which is of known nucleotide sequence as defined by the vectorette itself and to which in use the vectorette primer and, if desired nested vectorette primer(s) (as hereinafter defined) for example overlapping nested vectorette primer(s), may hybridise. The vectorette priming region is present in the strand complementary to the strand containing the initiating priming region. In this regard the "vectorette priming region" to which in use the vectorette primer and if desired nested vectorette primer(s) will hybridise may be present either in the target nucleic acid fragment/vectorette unit prepared by ligation or in a target nucleic acid fragment/vectorette unit prepared by primer extension of an initiating primer or in both. Thus it will be appreciated that the vectorette primer and if desired nested vectorette primer(s) to be used in the method of the present invention may be ] selected for hybridisation with a vectorette priming region which may, for example, not be generated until primer extension of an initiating primer and if desired nested initiating primer(s). A consequence of this is that, for example the vectorette itself need not be a totally self-complementary double-stranded DNA fragment.

The term "primer" as used herein refers to an oligonucleotide, whether occurring naturally as in a purified restriction digest or produced synthetically, which is capable of acting as a point of initiation of synthesis when placed under conditions in which synthesis of a primer extension product which is complementary to a nucleic acid strand is induced, i.e., in the presence of appropriate nucleoside triphosphates and an agent for polymerisation such as DNA polymerase in an appropriate buffer ("buffer" includes pH, ionic strength, cofactors, etc.) and at a suitable temperature.

The primer is preferably single stranded for maximum efficiency in extension, but may alternatively be double stranded. If double stranded, the primer is first treated to separate its strands before being used to prepare extension products. Preferably, the primer is an oligodeoxyribonucleotide. The primer must be sufficiently long to prime the synthesis of extension products in the presence of the agent for polymerization. The exact lengths of the primers will depend on many factors, including temperature and source of primer and use of the method. For example, depending on the complexity of the target sequence, the initiating and vectorette primers will typically contain 15-35 nucleotides, although they may contain more or fewer nucleotides. Short primer molecules generally require lower temperatures to form sufficiently stable hybrid complexes with the template.

The term "initiating primer" is used herein to refer to a primer capable of hybridisation with the initiating priming region as hereinbefore defined. In use with, for example, vectorette units prepared from total human

genomic DNA, it may be preferable for the "initiating primer" to be longer than 15-17 nucleotides so as to avoid hybridisation and priming, at random, to sequences present in the human genome which happen by chance to match that of the initiating priming region.

The term "vectorette primer" is used herein to refer to a primer capable of hybridisation to the vectorette priming region of the target nucleic acid fragment/vectorette unit. The "vectorette primer" will have a nucleotide sequence such that it is capable of hybridising to an initiating primer extension product, after separation from its complement, whereby the initiating primer extension product serves as a template for synthesis of an extension product of the vectorette primer, thereby facilitating amplification. Since, in general, the method of the present invention will be effected such that only one of the target nucleic acid fragment/vectorette units has an initiating priming region, only that unit will be subjected to amplification. Those target nucleic acid fragment/vectorette units which do not have an initiating priming region will be incapable of PCR amplification because whilst the formation of a vectorette primer extension product may be possible, no initiating primer will be able to hybridise to the vectorette primer extension product since no initiating priming region will be present and thus no PCR amplification will be possible.

In use it is preferred that the synthesis of vectorette primer amplification products is dependent upon the inital synthesis of an extension product of the initiating primer. This avoids formation of large numbers of unamplifiable vectorette primer extension products which might be expected to deplete the available nucleoside triphosphates or other co-factors in the reaction mixture in a detrimental fashion.

The term "nested primer" as used herein means a primer displaced by one or more base pairs in the 3′ direction away from the 5′ terminus of the initiating primer or in the 3′ direction away from the 5′ terminus of the vectorette primer or in the 3′ direction away from the 5′ terminus of both such initiating primer and vectorette primer. It will be appreciated that the sequence of the nested primer or primer(s) will necessarily be selected from sequence complementary to the known initiating or vectorette priming regions or from both such regions.

The term "target nucleic acid fragment/vectorette unit" (also referred to herein as a "vectorette unit") is used herein to refer to a nucleotide sequence, for example a DNA sequence, comprising a target nucleic acid fragment and a portion of known nucleotide sequence, for example a DNA sequence, which portion in single stranded form is capable of hybridisation to a vectorette primer as hereinbefore defined. In this regard it will be appreciated that the "target nucleic acid fragment/vectorette unit" may be capable of hybridisation to a vectorette primer either by virtue of the presence in the aforesaid unit of a portion of known nucleotide sequence which sequence is substantially complementary to the sequence of the vectorette primer or by virtue of the ability of an initiating primer extension product, based on one strand of the aforesaid unit as template, to comprise a nucleotide sequence substantially complementary to the sequence of the vectorette primer. It will be appreciated in this regard that the "target nucleic acid fragment/vectorette unit" is such that in one strand it will have substantially the same sequence at least in part as the vectorette primer. This strand will also contain the initiating priming region.

The portion of known nucleotide sequence, for example, DNA sequence, may be derived from any convenient source, provided that it fulfils the above-stated requirement that in single stranded form it is capable of hybridisation to the vectorette primer. Thus for example the vectorette may be prepared separately using a DNA synthesiser and the vectorette obtained ligated to the target nucleic acid fragment-(s) to obtain the target nucleic acid fragment/vectorette unit(s). In this regard the vectorette will conveniently be adapted for ligation to the nucleic acid fragment(s), for example a cohesive end on the target nucleic acid fragment(s) hybridising with a cohesive end on the vectorette to form a vectorette unit as hereinbefore defined or a blunt ended target nucleic acid fragment may be ligated to a blunt ended vectorette to form a vectorette unit as hereinbefore defined. It is not necessary however that the vectorette be pre-formed prior to ligation with the target nucleic acid fragment although this may be preferable. Thus for example the aforementioned unit may be prepared, in an appropriate case, by ligation of a single stranded DNA to the target nucleic acid fragment, for example by utilising the overhang of the cohesive end to secure a first single stranded DNA thereto and then to permit a second single stranded DNA to be ligated so as to form the desired unit.

In one embodiment of the present invention the target nucleic acid fragment/vectorette unit contains a blocking vectorette portion. The term "blocking vectorette" as used herein refers to a vectorette or to the vectorette portion of a target nucleic acid fragment/vectorette unit in which one or both free terminal bases are in modified form to prevent ligation of nucleotides thereto or to prevent primer extension for example in the presence of appropriate nucleoside triphosphates and an agent for the polymerisation of the nucleoside triphosphates under hybridising conditions. Such modifications are known per se and may for example consist of the presence of a dideoxynucleoside. Thus for example the double stranded blocking vectorette may have a 3′ terminal dideoxynucleoside such as dideoxyadenosine (ddA). Such modification may also

include ribonucleosides in which the diol of the ribose is cleaved, for example with periodate. Alternatively a 3′-deoxynucleoside for example a 3′-deoxyadenosine residue may be added at the 3′-terminus using for example cordycepin triphosphate and terminal transferase. As a further alternative a 3′-amino or 3′-thio functionality may be introduced chemically at the 3′ end by methods known per se.

In a further embodiment of the present invention a vectorette or vectorette portion comprises two partially hybridised single stranded sequences which possess a degree of non-complementarity such that vectorette primer extension cannot be effected using such a vectorette.

The term "nucleoside triphosphate" is used herein to refer to the triphosphates of nucleosides present in either DNA or RNA and thus includes nucleosides which incorporate adenine, cytosine, guanine, thymine and uracil as base, the sugar moiety being deoxyribose or ribose. In general deoxyribonucleosides will be employed in combination with a DNA polymerase. It will be appreciated however that other modified bases capable of base pairing with one of the conventional bases adenine, cytosine, guanine, thymine and uracil may be employed. Such modified bases include for example 7-deazaguanine and hypoxanthine.

The term "nucleotide" as used herein can refer to nucleotides present in either DNA or RNA and thus includes nucleotides which incorporates adenine, cytosine, guanine, thymine and uracil as base, the sugar moiety being deoxyribose or ribose. It will be appreciated however that other modified bases capable of base pairing with one of the conventional bases, adenine, cytosine, guanine, thymine and uracil, may be used in the initiating primer and vectorette primer employed in the present invention. Such modified bases include for example 7-deazaguanine and hypoxanthine.

The agent for polymerization of the nucleoside triphosphates may be any compound or system which will function to accomplish the synthesis of primer extension products, including enzymes. Suitable enzymes for this purpose include, for example, E.coli DNA Polymerase I (Richardson C.C.et al, J Biol. Chem. 239,222 (1964)), Klenow fragment of E.coli DNA polymerase I (Jacobsen H. et al, Eur. J. Biochem. 45, 623-627 (1974)), T4 DNA polymerase (Panet A. et al, Biochemistry 12, 5045-5050 (1973)), T7 DNA polymerase (Tabor S. and Richardson C.C., Proc. Natl. Acad. Sci. USA 84, 4767-4771 (1987)) other available DNA polymerases, reverse transcriptase, and other enzymes, including thermostable enzymes. The term "thermostable enzyme" as used herein refers to an enzyme which is relatively stable to heat and is heat resistant and catalyzes (facilitates) combination of the nucleotides in the proper manner to form the primer extension products which are complementary to each nucleic acid strand. Generally, the synthesis will be initated at the 3′ end of each primer and will proceed in the 5′ direction along the template strand, until synthesis terminates, generally producing molecules of different lengths. In the context of the current invention synthesis will generally terminate at a position determined by the target nucleic acid cleavage site resulting in molecules of the same length. There may be enzymes, including thermostable enzymes, however, which initiate synthesis at the 5′ end and proceed in the other direction, using a similar process to that described above. A preferred thermostable enzyme which may be employed in the process of the present invention is extracted and purified from Thermus aquaticus and has a molecular weight of about 86,000 - 90,000 daltons as described in European Patent Publication No. 237,362 (see also European Patent Publication No. 258,017). Thermus aquaticus strain YT1 is available without restriction from the American Type Culture Collection, 12301 Parklawn Drive, Rockville, Maryland, USA as ATCC 25,104.

It will be obvious that DNA polymerases having superior or more advantageous properties may be obtained by random mutation of clones expressing these proteins. By way of example, it would obviously be preferable to obtain a mutated version of double stranded DNA encoding Taq DNA polymerase which resulted in the expression of a protein with superior properties such as lack of a 5′-exonuclease activity. Techniques for obtaining such desired mutated versions are well known and are well within the skill of the average molecular biologist.

The term "complementary to" is used herein in relation to nucleotides to mean a nucleotide which will base pair with another specific nucleotide when incorporated into DNA or RNA. Thus deoxyadenosine triphosphate is complementary to thymidine triphosphate and deoxyguanosine triphosphate is complementary to deoxycytidine triphosphate, whilst deoxyguanosine triphosphate is not complementary to thymidine triphosphate. It is appreciated in this regard that whilst thymidine triphosphate and deoxyguanosine triphosphate may base pair under certain circumstances they are not regarded as complementary for the purposes of this specification.

The primers herein are selected to be "substantially" complementary to the different strands of each specific sequence to be extended or amplified. This means that the primers must be sufficiently complementary to hybridise with their respective strands. Therefore, the primer sequences need not reflect the exact sequence of their templates although this would normally be preferable.

The term "vectorette library" is used herein to refer to the plurality of target nucleic acid fragment/vectorette units which are obtained after cleaving a target nucleic acid at all the potential cleavage

sites in respect of a given restriction endonuclease which the target nucleic acid contains and preparing target nucleic acid fragment/vectorette units from the total mixture of target nucleic acid fragments by cycles of ligation to a suitably adapted vectorette portion (and repeat cleavage if required). In general only a single vectorette unit in a given vectorette library will contain the initiating priming region of interest. In the case of human genomic DNA cleaved with a restriction endonuclease recognising a specific 6 bp sequence (a 6 bp cutter) the average size of the resulting target nucleic acid fragments will be 4096 bp and the target nucleic acid generates approximately $10^6$ such fragments. Hence a vectorette library containing approximately $10^6$ target nucleic acid fragment/vectorette units is obtained from human genomic DNA cleaved with a 6 bp cutter restriction endonuclease and only one such vectorette unit in that total human vectorette library will contain a given initiating priming region capable of initiating amplification in the presence of an initiating primer, a vectorette primer if desired, appropriate nucleoside triphosphates and an agent for polymerisation of the nucleoside triphosphates under hybridising conditions.

Different vectorette libraries may be prepared from the same target nucleic acid by cleavage with different restriction endonucleases and ligation of suitably adapted vectorette portions to generate target nucleic acid fragment/vectorette units. All available restriction endonucleases can be used in this process if desired and in the limit a vectorette portion can be ligated to target nucleic acid fragment at every restriction enzyme recognition site in the target nucleic acid. This feature is not always desirable as ideally the initiating priming region of interest in any given vectorette library will be separated by 100 bp or more from the attachment point of the vectorette portion. This is because initiating primer extension products or initiating primer/vectorette primer amplification products smaller than this generate so little sequence information in the practice of the invention as to be of little value for the efficient sequencing of long nucleotide sequences. Furthermore the nucleotide sequence of such small products will be contained within the products obtained using a vectorette library in which the initiating primer is further from the vectorette portion attachment site. The use of a plurality of different vectorette libraries with a particular initiating primer allows identification of those libraries wherein the extension or amplification products are of a convenient size for sequencing. For example it may be particularly convenient to select initiating primer extension or amplification products of approximately 200 bp, 400 bp, 600 bp, 800 bp, 1000 bp and so on obtained from particular vectorette libraries with a given initiating primer. Sequencing of such products, from the vectorette libraries in which they happen to occur for a given initiating primer, using a vectorette or nested vectorette sequencing primer and methods known per se is likely to generate overlapping sequence data for a large region to the 3′-side of the initiating primer. The amount of sequence data generated in one round of analysis of a plurality of vectorette libraries with a given initiating primer is only limited by the size of initiating primer extension or amplification products which can be obtained in practice and/or by the distance (from the initating primer region) to the most remote restriction endonuclease site represented in the plurality of vectorette libraries.

In an advantageous embodiment of the method of the present invention, the target nucleic acid fragment/vectorette units are prepared from target nucleic acid fragments by ligation such that the vectorette cannot be cleaved from the target nucleic acid fragment/vectorette unit formed by the same agent used to cleave the target nucleic acid to yield target nucleic acid fragments. It is especially preferred that the target nucleic acid is cleaved with a restriction endonuclease to yield a target nucleic acid fragment for ligation to a vectorette, the sequence of the vectorette being selected such that the restriction endonuclease recognition sequence of the said restriction endonuclease is absent in the target nucleic acid fragment/vectorette unit, for example in the case of EcoRI described

```
—GAATTC          —G          + AATTX—
—CTTAAG  ——>     —CTTAA            Y—   — Vectorette

            Target nucleic acid
                 fragment
```

above where X is any nucleoside other than C and Y as its complementary nucleoside.

The present invention may be effected such that the synthesis of primer extension products or preferably vectorette primer amplification products is dependent upon the initial synthesis of an extension product of the initiating primer.

This may, for example, be achieved by linear amplification or preferably by the use of a vectorette

primer which is only capable of hybridising to the extension product of the initiating primer. Thus advantageously the vectorette portion of the target nucleic acid fragment/vectorette unit comprises a double stranded portion having first and second strands, the first strand having a terminal polymerisation blocking moiety and the second strand, which is ligated to that strand of the target nucleic acid fragment containing the initiating priming region, carrying a single stranded portion, the terminal polymerisation blocking moiety being effective to prevent extension of the first strand to form a complement to the said single stranded portion of the second strand in the presence of appropriate nucleoside triphosphates and an agent for the polymerisation of the nucleoside triphosphates under hybridising conditions. The vectorette primer is thus incapable of hybridising to the target nucleic acid fragment/vectorette unit, but is capable of hybridising to the extension product of the initiating primer. Thus no vectorette primer extension products are obtained until generation of an extension product of the initiating primer, following which the extension products obtained normally include a portion complementary to the known nucleotide sequence(s) of the single stranded portion of the second strand. This advantegous embodiment is illustrated hereinafter in Figure 6.

The polymerisation blocking moiety is effective to prevent polymerisation of a nucleotide sequence from a primer to form the complement of a template nucleotide sequence in the presence of an agent for polymerisation of nucleoside triphosphates such as a DNA polymerase, for example Klenow fragment of E. coli DNA polymerase I, T7 DNA polymerase or Taq DNA polymerase. The polymerisation blocking moiety may be any convenient group known for this purpose such as an appropriate modified nucleoside for example a dideoxynucleoside or a 3′-deoxynucleoside such as cordycepin or a 3′ amino or a 3′-thio functionality.

In a further especially preferred embodiment of the present invention the vectorette portion of the target nucleic acid fragment/vectorette unit comprises a double stranded portion having first and second strands, the second strand of the vectorette portion being ligated to that strand of the target nucleic acid fragment which contains the initiating priming region, and the nucleotide sequence of the first strand, second strand and vectorette primer being selected such that the vectorette primer is capable of hybridising to the complement of the second strand but not to the first strand under the same hybridisation conditions. It will be appreciated that the presence of a polymerisation blocking moiety in this embodiment is not necessary. It will further be appreciated, in this case, that the sequence of the vectorette primer will be substantially the same as the sequence of at least a portion of the second strand of the vectorette. This especially preferred embodiment of the invention is discussed further hereinafter in relation to Figure 6.

A further preferred embodiment of the present invention comprises the preparation of a plurality of different vectorette libraries for use with the same single initiating primer, each vectorette library being prepared by cleaving target nucleic acidat different cleavage sites and preparing target nucleic acid fragment/vectorette units from the target nucleic acid fragments by ligation whereby to form the said vectorette library; treating each vectorette library either separately or together with appropriate nucleoside triphosphates and an agent for polymerisation of the nucleoside triphosphates under hybridising conditions whereby to obtain a plurality of initiating primer extension products based on use of the same single initiating primer. The size of such extension products will be determined by the distance from the initiating primer to the closest 3′ site for the particular cleavage means, for example restriction enzyme used to construct that particular library.

If desired one or more of said initiating primer extension products may be isolated and/or sequenced or at least a portion of the extension product may be sequenced. Thus for example this embodiment may be conveniently used to identify a desired, normally the longest, target nucleic acid fragment containing an initiating priming region, so that the 3′ terminal end may be sequenced conveniently with a nested vectorette primer as hereinbefore described in order to provide a new start point for further use of the method of the present invention such as this preferred embodiment. The sequence of the 3′ terminal end of the aforementioned longest target nucleic acid fragment may thus become the initiating priming region of a new target nucleic acid fragment for a further round of vectorette library multiple initiating primer extension product formation, identification of the longest target nucleic acid fragment and sequencing.

In selecting a new initiating priming region on the basis of novel sequence data generated using the method of the invention at the 3′ terminal end of a target nucleic acid fragment such sequence data may routinely be compared with the publicly available database compilations of known nucleic acid sequence (for example Genbank, EMBL) so as to ensure that a proposed new initiating priming region does not by chance closely match a known nucleic acid sequence elsewhere in for example the genomic DNA of interest. This is obviously most likely to occur in those cases where the 3′-terminal end of a particular target nucleic acid fragment happens to comprise repetitive elements such as for example Alu sequences. In such cases it is advantageous to perform the method of the invention of a plurality of vectorette libraries with a given initiating primer so as to guarantee that at least one of the resulting extension products has a non-

repetitive/unique 3′-terminal end for the selection of a further initiating priming region.

Stepwise progression from one previously unknown initiating priming region to another along a target nucleic acid, for example human genomic DNA, may conveniently be monitored using samples of the said target nucleic acid separately cleaved to completion with the same restriction endonucleases as used in the preparation of target nucleic acid fragment/vectorette units ("vectorette libraries" as hereinbefore defined) and subjected to agarose gel electrophoresis and Southern Blotting. Probing of the filters so obtained with a first initiating primer will reveal a pattern of bands consistent with the various restriction enzyme recognition sites surrounding this first initiating priming region in the target nucleic acid. Use of the method of the present invention with a plurality of vectorette libraries and this first initiating primer will generate a series of extension products each of whose 3′terminal ends are defined by the position relative to the initiating priming region of the closest recognition site for the restriction enzyme used to generate the vectorette library in question. Thus a map of the restriction sites to the 3′ side of a first initiating primer is effectively obtained. Having subsequently selected a second novel initiating priming region of previously unknown sequence, linkage to the first initiating priming region is established by reprobing the above Southern Blot filter with the second novel initiating primer. The pattern of bands obtained will be identical to that obtained with the first initiating primer in those cases where no recognition site for the restriction enzyme in question lies between the first and second initiating priming regions. In those cases where a recognition site for the restriction enzyme in question does occur between the initiating priming regions as judged by the appearance of smaller extension products in the corresponding vectorette library,then a fragment of different size will normally be observed on reprobing the Southern Blot filter with the second initiating primer. By repetition of this method consistency, accuracy and reliability of stepwise progression from one initiating priming region to another along a target nucleic acid is maintained and assured.

It will be appreciated that the seqeunce of the 3′-terminal ends of all the plurality of initiating primer extension products may be easily obtained using the same vectorette primer or nested vectorette primers for sequencing by methods known per se. In this way the entire sequence of an unknown segment of target DNA nucleic acid may be determined in a facile and systematic manner and with much greater convenience than for example using M13 "Shotgun" cloning. This is because the initiating primer extension products can be ordered by size and therefore the order of their sequences in the original target nucleic acid becomes apparent. Each initiating primer extension product shares a 5′-extremity determined by the initiating primer and a 3′-extremity determined by the closest 3′-site for the particular cleavage means, for example restriction enzyme, used in the synthesis of that particular vectorette library.

In a preferred embodiment according to the present invention any or all of the initiating primer extension products obtained is (are) sequenced (as hereinafter defined) at least at the end(s) distal to a given initiating primer so as to determine the sequence of a further initiating primer whereby to obtain further initiating primer extension products based on primer extension of the further initiating primer.

In a further preferred embodiment according to the present invention an initiating primer extension product or portion thereof is sequenced (as hereinafter defined) whereby to characterise the said extension product or portion thereof.

As described above one potentially important application of the present invention is the identification of a previously unidentified genotype, for example a genetic defect(s) responsible for a phenotype, for example a genetic disease or disorder or the identification of a previously unidentified genotype, for example a genetic defect(s) which is (are) responsible for or a contributory factor in predisposition to a phenotype, for example a disease

Thus for example in relation to a genotype such as a genetic disease or disorder the method of the present invention may be applied to nucleic acid which does not contain the genotype (e.g. genetic defect(s)) and to nucleic acid which does contain the genotype e.g. genetic defect(s) to be investigated, identification of the genotype e.g. genetic defect(s) being effected by comparison of the information generated by sequencing of the two nucleic acid samples. Such comparison might simply be effected, for example, by comparison of the sequencing gels conveniently by automatic scanning. In this regard it will be appreciated that the specific sequences need not be determined per se provided that sufficient data is generated to enable a difference or differences between the target nucleic acid samples to be detected and identified, and the terms "sequencing" and "sequenced" are accordingly used herein to include not only specific nucleotide sequence determination, but also the detection and identification of sequence differences without specific nucleotide sequence determination. It is convenient to apply the method of the invention to the target nucleic acid of an obligate heterozygote for example for the genetic disease or disorder to be investigated. Of necessity both a normal and a mutant allele for the locus in question will be present in such an individual and those sites identified using the method of the invention where more than a single nucleotide is present on sequencing are candidates to be the phenotype, e.g. disease or disorder

causing mutation.

In addition to the above it is suspected that certain genotypes e.g. genetic defects may predispose individuals to phenotypes for example diseases such as premature atherosclerosis, hypertension, diabetes and cancer. For example, if such genetic defects could be identified then such "high risk" patients could be monitored and any onset of the disease treated at an early stage. The method of the present invention may be applied to the identification of such predisposing genotypes. Thus for example the method of the present invention may be applied to the nucleic acid of a plurality of individuals affected by a phenotype to be investigated on the one hand and to the nucleic acid of a plurality of individuals presenting no evidence of the said phenotype on the other hand, identification of a genotype being effected by comparison of the sequences of the nucleic acid samples. Conveniently nucleic acid from the plurality of individuals affected by the phenotype to be investigated will be pooled and subjected to the method of the present invention and similarly nucleic acid from the individuals presenting no such evidence of the said phenotype will be pooled and subjected to the method of the present invention. Comparision of the sequence differences between the two pools will identify the presence of any predisposing genotype if any is(are) present. The advantage of this technique is that it enables individual predisposing genotype to be identified irrespective of their frequency of occurence and irrespective of the overall complexity and number of different contributory genetic factors to the overall phenotype. Thus if the presence of a combination of apparently unrelated genetic defects are responsible for or represent a contributory factor in the predisposition to a disease to be investigated, the method of the present invention will be able to identify this.

One embodiment of the present invention comprises circularising a target nucleic acid fragment having termini capable of ligation to each other, the target nucleic acid fragment containing a portion of known nucleotide sequence, such known nucleotide sequence or portion thereof being capable of serving as an initiating priming region for hybridisation with an initiating primer, hybridising an initiating primer to the said initiating priming region and subjecting the hybrid thus formed to primer extension in the presence of appropriate nucleoside triphosphates and an agent for polymerisation of the nucleoside triphosphates under hybridising conditions. For example 2 initiating primers oriented in the opposite sense to each other than that normal for PCR may then be used to effect amplification.

In this regard circularisation of the target nucleic acid fragment may be regarded as the preparation of the target nucleic acid fragment/vectorette unit by ligation and thus for example primer extension may be performed based on the circularised target nucleic acid fragment as template. If desired however, the circularised target nucleic acid fragment may be cleaved and primer extension performed based on the cleavage product as template as described in Nucleic Acids Research Vol 16 pp 8186 1988 which was published after our UK Patent Application No. 8818020.3 from which convention priority is claimed. Further, if desired the cleavage product may be used to prepare a target nucleic acid fragment/vectorette unit by ligation and initiating primer extension performed based on the target nucleic acid fragment/vectorette unit thus obtained as template. All such embodiments are within the scope of the present invention.

Thus for example the circularised target nucleic acid fragment formed may be cleaved to yield a cleavage product which contains at least a portion of known nucleotide sequence such portion being capable of serving as an initiating priming region for hybridisation with an initiating primer in which case for example the circularised target nucleic acid fragment may be cleaved within the region of known nucleotide sequence to form a linear molecule having known sequence at its termini flanking unknown sequence; it may be cleaved at a terminus of the known nucleotide sequence to form a linear molecule having a known nucleotide sequence at one terminus and a known cleavage site pattern at the other terminus which renders it capable of ligation to a vectorette; or more preferably it may be cleaved outside the known nucleotide sequence to form a linear molecule containing the known nucleotide sequence and having a known cleavage site pattern at at least one terminus, normally both termini, which enables the terminus or termini to be ligated to form a target nucleic acid fragment/vectorette unit. Thus a preferred embodiment of the present invention comprises circularising a target nucleic acid fragment having termini capable of ligation to each other, the target nucleic acid fragment containing a portion thereof being capable of serving as an initiating priming region for hybridisation. with an initiating primer; cleaving the circularised target nucleic acid fragment outside the known nucleotide sequence to form a linear molecule containing the known nucleotide sequence and having a known cleavage site pattern at at least one terminus for ligation to form a target nucleic acid fragment/vectorette unit; forming said target nucleic acid fragment/vectorette unit by ligation and treating the target nucleic acid fragment/vectorette unit, together or sequentially, with appropriate nucleoside triphosphates and an agent for polymerisation of the nucleoside triphosphates under hybridising conditions.

This preferred embodiment of the invention is of interest in relation to circularised target nucleic acid fragments of for example at least 1 kb up to about 20 kb but is of particular interest in relation to larger

circularised target nucleic acid fragments for example of from 20 kb up to about 120 kb such as circularised target nucleic acid fragments of about 100 kb. The upper size limit is dependent on practical considerations and is thus based on the maximum size of target nucleic acid fragments which can be circularised. Such circularisation may be effected by known techniques [F.S. Collins (1988) Genome Analysis - A practical approach (Editor K. Davies) p73-94, IRL Publishers, Oxford], for example by effecting ligation at low concentration. This preferred embodiment may thus be effected by for example digesting genomic DNA with a restriction enzyme, preferably a restriction enzyme which cuts relatively infrequently (for example average fragment size 10-20 kb) such as XbaI, KpnI or BamHI. The fragments may then be self ligated to form circles. The circles may then be cut with a restriction enzyme, preferably a restriction enzyme which cuts frequently (for example HinfI) and the fragments ligated to a corresponding (for example HinfI) vectorette. Thus for example if sequence adjacent to a known XbaI site is available, sequence adjacent to the next, possible distant, XbaI site can be obtained by analysis of the vectorette product. In this way, a series of jumps may be made providing additional start points for the method. It will be appreciated that the ability to make such jumps represents a considerable advantage over PCR techniques as previously described in so far as it may be possible to obtain amplified vectorette products at a considerable distance from the site of the initiating primer. Thus the method is not limited by the difficulty encountered in routine use of obtaining standard PCR products of more than about 5 kb.

In order that the present invention may be more fully understood it is described hereinafter, by way of example, with reference to the accompanying drawings, in which:-

Figure 1(a) illustrates a double stranded target nucleic acid, Figure 1(b) illustrates the restricted target nucleic acid, Figure 1(c) illustrates the target nucleic acid fragment/vectorette units obtained by ligation of the target nucleic acid restriction fragments and the vectorette, and Figure 1(d) illustrates hybridisation of (i) the initiating primer and (ii) vectorette primer with the target nucleic acid fragment/vectorette units.

Figure 2 illustrates one embodiment of the invention; Figure 2(a) depicting visualisation of restriction fragments obtained by partial digestion of genomic DNA and subjected to gel electrophoresis. Figure 2(b) depicts the mixture of different types of high molecular weight fragments size selected for further processing. Figure 2(c) depicts the mixture of products obtained following ligation to form target nucleic acid fragment/vectorette units.

Figure 3 illustrates one embodiment of the invention which seeks to increase the relative concentration of restriction fragments capable of hybridising to the initiating primer.

Figure 4 illustrates a further embodiment of the invention which avoids the use of S1 nuclease and which seeks to increase the relative concentration of restriction fragments capable of hybridising to both the initiating primer and vectorette primer, Figure 4(a) depicting the mixture of products which might be obtained following treatment of the restriction fragments produced by complete digestion of the target DNA sequence of interest with blocking vectorette in the presence of DNA ligase. Figure 4(b) depicts a blocking vectorette for use in the present invention after digestion with the restriction enzyme Eco R1 and Figure 4(c) depicts the products obtained by complete digestion of the mixture of products depicted in Figure 4(a).

Figure 5 shows two different vectorette portions for use in the method of the present invention, vectorette portion (i) is a blocking double stranded vectorette having a short top (5′) strand having a 3′ terminal group capable of blocking polymerisation and a bottom (3′) strand forming a tail, the 3′ terminal group of the top (5′) strand being capable of blocking polymerisation in the presence of nucleoside triphosphates, and an agent for polymerisation of the nucleoside triphosphates, under hybridising conditions; vectorette portion (ii) is double stranded but having a degree of non-complementarity between the strands.

Figure 6 shows a schematic representation of the application of the present invention to the vectorette portions of Figure 5.

Figure 7 shows a schematic representation of the use of vectorette libraries.

Figure 8 shows the schematic application of the present invention to amplification of circularised target nucleic acid fragments.

Figure 9 depicts amplification of part of the $\alpha$-1 antitrypsin gene using oligonucleotides 58 and 61 (Step 6 in Method I) and an XbaI vectorette unit/library.

Figure 10 shows the restriction map of the plasmid B3(pUC8 with a 440 bp insert in the EcoRI site).

Figure 11 (i) and (ii) are photographs of an agarose gel showing the amplified fragments obtained in Examples 1, 5 and 7 hereinafter.

Figure 12 shows a sequence from the phenylalanine hydroxylase gene flanking exon 9 and the relative positions of binding for the oligonucleotides 58, 63, 66, and 59.

Figure 13 shows a portion of a photograph of an agarose gel revealing the amplified product obtained according to Example 8.

Figure 14 shows a photograph of an agarose gel revealing the amplified products obtained according to Example 9.

Figure 15 shows a photograph of an agarose gel revealing the amplified products obtained according to Example 10.

Figure 16 shows a photograph of an agarose gel revealing the amplified products obtained according to Example 11.

Figure 17 shows a photograph of an agarose gel revealing the amplified products obtained according to Example 12.

Figure 18 shows a photograph of an agarose gel revealing the amplified products obtained according to Example 13.

Figure 19 shows a photograph of an agarose gel revealing the amplified products obtained according to Example 14.

Figure 20 shows a photograph of an agarose gel revealing the amplified products obtained according to Example 15.

Figure 21 shows an autoradiograph of a sequencing gel showing the sequence of the products generated in Example 16.

Figure 22 shows the nucleotide sequence data generated from the products described in Example 16.

Figure 23 shows a section of the phenylalanine hydroxylase gene flanking exon 1 and indicates the relative positions of the oligonucleotides 58, 62 and 67 as well as the EcoRI restriction site used in vectorette unit construction.

Figure 24

(a) is a photograph of an agarose gel showing the primary amplification product obtained according to Example 17;

(b) is a photograph of an agarose gel showing the secondary amplification products obtained according to Example 17.

Figure 25 shows the nucleotide sequence data generated from the products described in Example 17.

25(a) shows sequence read with oligonucleotide 62 (as hereinafter defined), Figure 25 (b) shows sequence read with oligonucleotide 67 (as hereinafter defined) and Figure 25 (c) shows the overall sequence read with oligonucleotides 62 and 67.

Figure 26 shows a section of the $\alpha$-1 antitrypsin gene flanking exon V and showing the relative positions of the oligonucleotides 58, 60, 68, 69 and 70 as well as the EcoRI restriction site for annealing and ligation of the vectorette.

Figure 27

(a) is a photograph of an agarose gel showing the primary amplification product obtained according to Example 18;

(b) is a photograph of an agarose gel showing the secondary amplification product obtained according to Example 18.

(c) is an autoradiograph of a nylon filter, blotted from the gel shown in Figure 27(b) and probed with an $\alpha$-1 antitrypsin probe.

Figure 28 shows the nucleotide sequence of D5, an anonymous fragment on chromosome 7 of the human genome, indicating the relative positions of oligonucleotides 71 and 72.

Figure 29 shows a restriction map of the region containing D5. The relative positions of oligonucleotides 71 and 72 are indicated. The positions of the products synthesised accordingly to Example 19 are also shown.

Figure 30 is a photograph of an agarose gel showing the amplified products obtained according to Example 19.

Figure 31 shows the nucleotide sequence generated from the Bcl 1 product obtained as described in Example 19. The position of the Hind III and Hae III sites predicted from restriction enzyme digests is shown. The position of oligonucleotide 73 which would be used as an initiating primer in subsequent cycles of vectorette unit amplification is also indicated.

Figure 32 is a photograph of an agarose gel showing the amplified products obtained according to Example 20.

Figure 33 shows the nucleotide sequence generated from the EcoR1 product obtained as described in Example 20.

Figure 34 is a photograph of an agarose gel showing the fragments obtained after digestion with restriction enzymes of the EcoR1 product obtained as described in Example 20.

Figure 35 is a photograph of an agarose gel showing the amplified products obtained according to Example 21.

Figure 36

(a) shows a restriction map of a section of Hind III/cosmid vectorette library, indicating the positions of oligonucleotides 58, 76 and 77.

(b) shows a photograph of an agarose gel showing the amplified products obtained according to Example 22.

Figure 37

(a) shows a restriction map of a segment of target genomic DNA ligated to an EcoR1 vectorette. The positions of oligonucleotides 27, 76, 78 and 79 are indicated.

(b) shows a restriction map of a segment of target genomic DNA ligated to an EcoR1 vectorette. The positions of oligonucleotides 78,79 and 84 are indicated.

(c) shows a segment of the Phenylalanine Hydroxylase gene exon 9 ligated to an EcoR1 vectorette. The positions of oligonucleotides 63 and 79 are indicated.

(d) shows a restriction map of the segment of target genomic DNA (described in 37(c) above) ligated to a modified EcoR1 vectorette. The positions of oligonucleotides 76, 79, 80 and 81 are indicated.

(e) shows a restriction map of a segment of target genomic DNA (described in 37(b) above) ligated to a modified EcoR1 vectorette. The positions of oligonucleotides 79, 80, 81 and 84 are indicated.

(f) shows a segment of the Phenylalanine Hydroxylase gene exon 9 ligated to a modified EcoR1 vectorette. The positions of oligonucleotides 63, 79, 80 and 81 are indicated.

(g) shows a restriction map of a segment of target genomic DNA ligated to a modified Hind III vectorette. The positions of oligonucleotides 76, 78 and 79 are indicated.

(h) shows a restriction map of a segment of target genomic DNA ligated to a modified Hind III vectorette. The positions of oligonucleotides 76, 79, 81 and 82 are indicated.

(i) shows a restriction map of a segment of target genomic DNA ligated to a modified Hind III vectorette. The positions of oligonucleotides 76, 79, 81 and 83 are indicated.

Figure 38 shows a photograph of an agarose gel revealing the amplified products obtained according to Examples 23 (a), (b), (c), (d), (e), (f), (g), (h) and (i).

Figure 39

(a) shows a restriction map of the target genomic DNA sequence containing the polymorphic Pst 1 site detected by the probe KM19. The polymorphic Pst 1 site is indicated by an asterisk.

(b) shows the formation of circular products by ligation of Pst 1 cleaved target genomic DNA fragments.

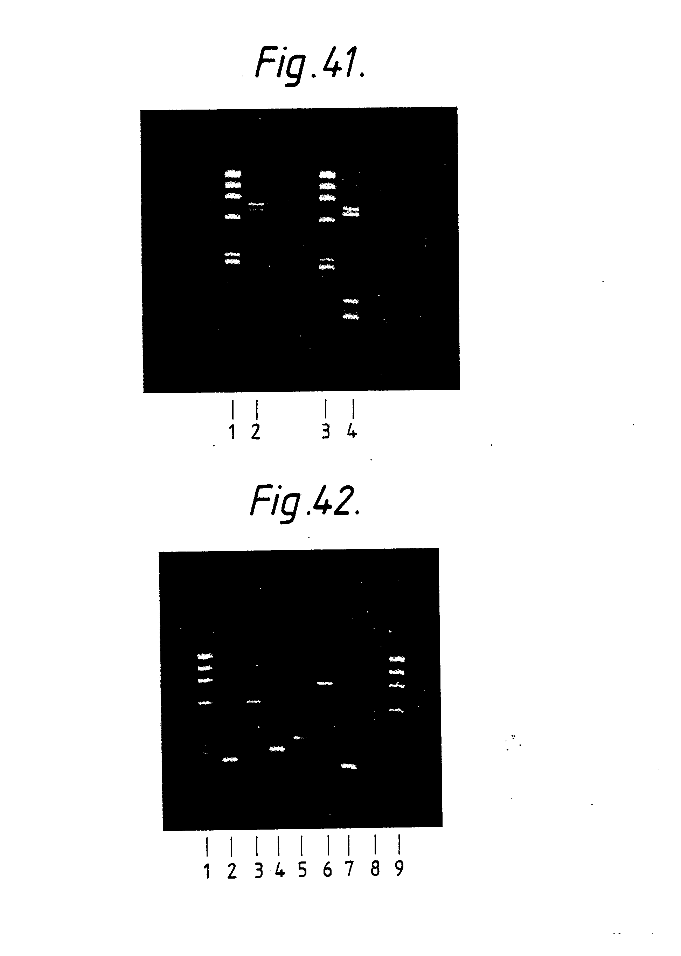

(c) shows the construction of Hind III vectorette unit libraries by ligation of oligonucleotides to Pst 1 circles cleaved by Hind III Figure 40 shows a photograph of an agarose gel revealing the amplified products obtained according to Example 24.

Figure 41 shows a photograph of an agarose gel obtained by taking a sample from lane 3 of Figure 40, precipitation and loading onto a fresh agarose gel.

Figure 42 shows a photograph of an agarose gel revealing the amplified products obtained according to Example 25.

Figure 1(a) shows schematically a double stranded target nucleic acid of for example 100 kb or more, the sequence of which is unknown except for the initial $5'$ nucleotides as shown hatched, for example about 20 nucleotides. A locus of interest, X, of unknown sequence is shown at an unknown distance from the $5'$-end. The target nucleic acid is subjected to restriction (see Figure 1(b)) using a 6 bp cutter such as EcoRI which is expected to cleave a nucleic acid, on average, every 4096 bp. The recognition sequence for EcoRI is GAATTC, the cleavage point being indicated by the arrow. EcoRI therefore cleaves the target nucleic acid to yield cohesive ends. The cohesive end of the target nucleic acid restriction fragments are then ligated to an appropriate vectorette as shown in Figure 1(c) the vectorette being hatched. The vectorette may be of any convenient nucleotide sequence as described above but will have at its $5'$ end a nucleotide sequence pattern consistent with ensuring ligation with the cohesive ends of the restriction fragments referred to above. Thus if EcoRI is used as the restriction endonuclease the $3'$-end of the restriction fragments and the $5'$ end of the vectorette will have the sequences

| | | | |
|---|---|---|---|
| Restriction | 5' – G | AATTC – 3' | Vectorette |
| fragments | 3' – CTTAA | G – 5' | |

Preferably that portion of the restriction endonuclease recognition pattern which does not take part in the ligation is altered in the vectorette to destroy the recognition pattern. Thus any restriction endonuclease present for the purpose of cleaving the target nucleic acid is unable to cleave the restriction fragment/vectorette units once formed. Where the restriction endonuclease used is EcoRI therefore the 5′terminal end of the vectorette is preferably

```
5'  AATTA 3'  or 5'AATTT 3'  or 5' AATTG 3'
        T              A              C
```

the CG portion of the recognition pattern being changed to AT or the CG being transposed to that in the EcoRI recognition pattern. The restriction fragment/vectorette units are then treated together or preferably sequentially (with the intiating primer added first) with initiating primer and vectorette primer under hybridising conditions (see Figure 1 (d)). The initiating primer will only be capable of hybridising to that portion of the one target restriction fragment containing a known sequence (see Figure 1 (d) (i)). The vectorette primer is designed to hybridise to the nucleic acid strand which is formed by extension of the initiating primer (and as shown in Figure 1(d) the nucleic acid strand which is complementary to the nucleic acid strand to which the initiating primer is capable of hybridising). The vectorette primer will hybridise to the vectorette priming region of each restriction fragment/vectorette unit present (see Figure 1 (d) (ii)), but in the presence of appropriate nucleoside triphosphates and an agent for the polymerisation of the nucleoside triphosphates, amplification will only take place in respect of the restriction fragment/vectorette unit to which the initiating primer was hybridised then extended. It will be appreciated that preferably the hybridisation (ii) will only occur in use after the hybridisation (i), extension in the presence of nucleoside triphosphates and an agent for polymerisation of the nucleoside triphosphates and subsequent denaturation. This prevents one situation apparent from Figure 1(d) where the vectorette primer (ii) is seen to hybridise to and therefore be capable of forming an extension product with every target nucleic acid fragment/vectorette unit present in the complex mixture. It is thus preferably to treat the vectorette unit first with the initiating prime and then, after ther steps detailed above to add the vectorette primer.

It will therefore be possible to identify the amplified target fragment and if desired sequence the whole fragment, for example, by techniques described above. If desired, however, only the 3′end of the amplified target restriction fragment need be sequenced so that a new initiating primer may be designed. This new initiating primer may then be used to obtain amplification of a new target restriction fragment obtained by digesting the target nucleic acid with a different restriction endonuclease, preferably also a 6 bp cutter, and the method of the present invention repeated. In this way a target nucleic acid may be sequenced from the 5′ end without the need for cloning and with the direction of sequencing constantly and consistently proceeding in the 5′ to 3′ direction. By repeating the method of the present invention therefore it is possible to rapidly reach and sequence the aforementioned locus, X, of interest.

Figure 2 illustrates one embodiment of the present invention. The DNA sequence of interest is subjected to partial digestion for example with EcoRI and the restriction fragments obtained subjected to gel electrophoresis as depicted in Figure 2(a). Electrophoresis proceeds in the direction indicated by the arrow and thus high molecular weight restriction fragments appear towards the top of the gel whilst the lower molecular weight restriction fragments appear towards the bottom of the gel. The X axis represents an increasing concentration of restriction enzyme or prolonged incubation. Restriction fragments of less than a predetermined size, for example 10 kb, are discarded and the higher molecular weight restriction fragments are taken through to the next stage. These higher molecular weight restriction fragments will constitute a mixture of overlapping partial digestion fragments as depicted in Figure 2(b), restriction sites being at a restriction fragment terminus marked (⊥ ) or within the fragment, marked (v). All the restriction fragments will have been cut using the same single restriction endonuclease, for example EcoRI, and thus each fragment will have cohesive ends characteristic of the restriction endonuclease employed to effect the digestion. Only the extreme 5′ and 3′ fragments will have a single such cohesive end whilst the remaining fragments will each have two such cohesive ends. A vectorette as hereinbefore defined in which the restriction endonuclease recognition sequence has been destroyed may then be mixed with the above-mentioned target restriction fragments in the presence of a ligase to yield a mixture of products as exemplified in Figure 2(c), the vectorette being shown as a terminal box. It will be appreciated that the presence of ligase enables not only ligation of the target restriction fragments with the vectorette to be effected, but also enables ligation of the target restriction fragments themselves. It will be appreciated therefore that under the conditions of ligation in Figure 2 it is not unlikely that segments A and B in the

initial target nucleic acid fragment/vectorette units will not be adjacent in the original target genomic nucleic acid but will have come together anomalously during ligation. The products of ligation are then subjected to complete digestion with the restriction endonuclease employed to effect the partial digestion, for example EcoRI. The products of complete digestion will in general be double stranded target restriction fragments having a vectorette ligated at each end of the fragment and target restriction fragments having a vectorette at only one end of the fragment. There will be a background population of target restriction fragments, for example fragments A and B in Figure 2(c) which will not have a vectorette on either end. The former products will constitute a minor proportion of the total mixture of products. On denaturation only a trace of product vectorette units will be able to hybridise with the initiating primer, but a very substantial proportion of the product mixture may be capable of hybridising with the vectorette primer unless substantial modification of the vectorette is undertaken as described below. Amplification of the desired restriction fragment will therefore be possible but the substantial proportion of the vectorette unit library capable of hybridising with the vectorette primer may result in the need to use substantial quantities of the vectorette primer, of the agent for polymerisation of the nucleoside triphosphates (e.g. Taq DNA polymerase) and of the nucleoside triphosphates themselves. This difficulty may be obviated to some extent by the use of small quantities of starting material, but it would be advantageous to selectively increase the relative proportion of the target restriction fragment/vectorette units capable of hybridising to the initiating primer in the product mixture.

The rationale for the approach to construction of a vectorette unit library illustrated in Figure 2 is as follows. Total digestion with a 6 bp cutting enzyme will generate a population of fragments with a mean size of 4096 bp. Ligation of vectorette units to this population (approximately $10^6$ fragments from total human genomic DNA) would be expected to generate many fragments with a vectorette unit at each end. Many of these will be considerably smaller than 4096 bp given the Gaussian distribution of restriction sites. In use a vectorette primer in these circumstances might act as both primers in a standard PCR. Large amounts of spurious amplification products would be generated as well as any product arising from an initiating primer. In the discussion hereinbefore reference has been made to methods in which the synthesis of vectorette primer amplification products is dependent upon the initial synthesis of an extension product of the initiating primer. In the Figure 2 embodiment the vectorette unit itself may hybridise with and initiate copying from the vectorette primer. The above disadvantage of PCR product formation from target nucleic acid fragments with vectorette portions at each end is overcome by a size fractionation step such that any such vectorette unit constructs have the 2 identical vectorettes separated by such a distance (eg greater than 10 kb) that standard PCR is so inefficient that no significant product is generated.

Figure 3 illustrates one embodiment which seeks to increase the relative concentration of restriction fragments capable of hybridising to the initiating primer in the vectorette unit product mixture obtained as described above in relation to Figure 2. In Figure 3 (a), (i) is a restriction fragment containing an initiating priming region (1) and has a vectorette portion (hatched) (2), (ii) is a restriction fragment having a vectorette portion (hatched) (2) ligated at each end, but no initiating priming region and (iii) is a restriction fragment having no initiating priming region, but having a single vectorette portion (hatched) (2). In this regard the product mixture is treated, after denaturation (see Figure 3b), with intiating primer only (3), in the presence of an agent for polymerising nucleoside triphosphates, such as Taq DNA polymerase, and nucleoside triphosphates. No vectorette primer is employed at this stage. The target restriction fragment/vectorette units capable of hybridising to the initiating primer are thus selectively replicated (see Figure 3 (c)); no replication of fragments without the necessary initiating priming region being possible. The product mixture thus obtained is then subjected to treatment with a single-strand specific endonuclease such as S1 nuclease (see Figure 3 (d)). This endonuclease breaks the internal phosphodiester bonds in a single stranded DNA and thus results in the production of blunt-ended double stranded DNA. This process increases the relative concentration of the target restriction fragment/vectorette units capable of hybridising to the initiating primer over the remaining background fragments. Moreover if desired this process step of denaturation, treatment with initiating primer only in the presence of an agent for polymerising nucleoside triphosphates and nucleoside triphosphates followed by treatment of the product mixture obtained with a single-stranded specific endonuclease such as S1 nuclease may be repeated as many times as thought desirable or necessary to increase the relative concentration of the target restriction fragment/vectorette units capable of hybridising to the initiating primer. When the concentration of such vectorette/units is considered to be sufficiently high in relation to the remaining background fragments the product mixture may be additionally treated with vectorette primer to effect amplification as described in US Patents Nos 4,683,195 and 4,683,202.

A disadvantage of the above-proposed technique is that S1 nuclease tends not to be as single-strand specific as might be desirable and thus even some double stranded DNA may be degraded.

Furthermore linear amplification would not be appropriate in relation to the technique of Figure 3 since only a single round of initiating primer extension would yield S1-nuclease resistant double stranded DNA.

Figure 4 illustrates a further embodiment of the present invention which avoids the use of S1 nuclease and which seeks to increase the relative concentration of restriction fragments capable of hybridising to the initiating primer in the product mixture obtained by complete restriction endonuclease digestion of the target DNA sequence of interest. The advantage of this embodiment is that no fractionation of the fragments obtained is necessary nor is gel electrophoresis required as an initial step. Following complete digestion of the DNA sequence of interest with a restriction endonuclease such as EcoRI the fragments obtained are treated with a blocking vectorette (as hereinbefore defined) in the presence of a DNA ligase to yield a complete mixture of products as depicted in Figure 4(a). It will be appreciated that the fragments A and B will not necessarily be adjacent in the original DNA sequence but may have been joined anomalously in the DNA ligase reaction. The blocking vectorette consists of a double stranded oligonucleotide sequence as depicted in Figure 4(b) having a first cohesive end at one end thereof adapted for ligation to the corresponding cohesive ends of the restriction fragments, but in which that part of the restriction endonuclease recognition site which does not take part in the ligation is altered in the vectorette to destroy the recognition site such that the restriction fragment/vectorette unit once formed cannot be cleaved by the restriction endonuclease which gave rise to the restriction fragments. The other end of the blocking vectorette comprises a terminus having a blocking residue which might preferably be a 3′-dideoxynucleoside such as dideoxyadenosine (ddA), or any other 3′ residue such that primer extension cannot proceed there, for example a 3′-deoxynucleoside or other chemical modification known per se. The lower strand overlaps the upper strand as shown in Figure 4(b). This strand preferably terminates in a non-phosphorylated residue to avoid problems of self ligation. The region (4) possess the same sequence as the vectorette primer such that the vectorette primer is able to hybridise with the extension product of an initiating primer but not with the unextendable upper strand of the 3′-modified blocking vectorette. The blocking vectorette is conveniently prepared as two separate strands which are subsequently hybridised. The first and second strands are each prepared separately, for example manually, or conveniently using a DNA synthesiser, the first strand being 5′ phosphorylated by chemical means, conveniently enzymatically for example by the use of polynucleotide kinase, and a terminal transferase being conveniently used to introduce a 3′-terminal dideoxynucleotide or other modified nucleotides. Alternatively other blocking moieties (for example amino) may be introduced chemically at the 3′ end by methods known per se. The first and second strands thus prepared are then allowed to hybridise to form the double-stranded blocking vectorette.

The mixture of products depicted in Figure 4(a) and obtained following treatment of the restriction fragments with blocking vectorette in the presence of a DNA ligase is subjected to complete digestion with the same restriction endonuclease used to create the restriction fragments prior to formation of the restriction fragment/vectorette units whereby the types of products (i), (ii) and (iii) depicted in Figure 4(c) are obtained. In order to reduce the relative concentration of restriction fragments (iii) present in the mixture of restriction fragment/vectorette units (i) and (ii) the above mentioned cycle may be repeated as many times as thought necessary or desirable to form a pool of restriction fragment/vectorette units for example based on the use of the restriction endonuclease EcoR1.

Figure 5 shows in (i) a blocking vectorette comprising a 14 base top oligonucleotide and a 42 base bottom oligonucleotide annealed together with the four base overhang (a 5′ overhang) complementary to the ends produced in the cleaved target DNA. $N^1$, $N^2$, $N^3$, $N^4$, $N^5$ and $N^{5'}$ are as hereinafter defined in Table 1. The 3′end of the 14 base oligonucleotide is modified (X) such that 5′ → 3′ extension from that end cannot be carried out using any of the known DNA polymerases eg Klenow fragment, T7 DNA polymerase (sequenase) or Taw DNA polymerase. This modified base, may be a dideoxynucleoside or a 3′-deoxynucleoside (eg cordycepin). The 3′ sugar residue may, if desired, be modified by any chemical methods known per se.

The 3′ terminal base of the bottom 42 mer and the complementary base at position 5 (from the 5′ end) in the 14 base top oligonucleotide are chosen such that following ligation of the blocking vectorette to the cleaved target DNA the resulting hexanucleotide (at the ligation junction) is not recognised by the restriction endonuclease used to cleave the genomic DNA originally. This is important when ensuring that every cleaved genomic DNA fragment has a blocking vectorette ligated to its ends. Obviously, the ligation products will contain cleaved genomic DNA fragments re-ligated to each other and these are digested again in order to generate receiving ends for the blocking vectorette in a second ligation reaction. This restriction and ligation cycle may need to be repeated more than once, for example three to four times to ensure every cleaved genomic DNA fragment has a blocking vectorette at either end. It is preferred that each cleaved genomic DNA fragment has a blocking vectorette at either end so as to prevent random printing

from any unblocked 3′-ends of genomic DNA fragments in the reaction mixture after denaturation and treatment with an initiating primer.

The blocking vectorette shown in Figure 5 (i) is suitable for target genomic DNA cleaved with restriction endonucleases that produce 5′ overhangs. For restriction endonucleases that produce a 3′ overhang the blocking vectorette would have to be modified slightly. Thus for example the 14 base top oligonucleotide might be replaced by a 10 base top strand (missing the 4 base overhang at the 5′ end) and the 42 base bottom oligonucleotide strand might be replaced by a 46 base bottom strand, with the extra 4 bases being at the 3′ end and being complementary to the ends produced by the restriction endonuclease used to cleave the genomic target DNA.

Figure 5 (ii) shows a non-complementary vectorette portion consisting of two annealed single stranded sequences (a 57 base and a 53 base oligonucleotide) which possess a degree of non-complementarity such that vectorette primer extension cannot take place immediately. The discussion above in relation to the blocking vectorette of Figure 5 (i) applies exactly to the non-complementary vectorette portion with the exception that the non-complementary vectorette portion need not possess any modified nucleosides.

Figure 6 shows a schematic representation of the application of the present invention to the vectorette portions of Figure 5 (i). Genomic target DNA is digested to completion with a single restriction endonuclease to produce fragments as shown in (i). The blocking vectorette portions [5 in Figure 5(i)] are ligated onto the ends of every cleaved genomic target DNA fragment (ii) in the presence of DNA ligase. Amplification is then carried out after denaturation using the primers of known nucleotide sequence x and y in the presence of for example Taq polymerase.

Thus in Figure 6 (iii), x represents the initiating primer and possesses the same or at least substantially the same sequence as the region marked IP (initiating primer) in strand 1. x is capable of hybridising to the initiating priming region (IPR) in strand 2. Strand 2 which contains the initiating priming region (IPR) also contains a portion of nucleotide sequence which is the same or at least substantially the same as the nucleotide sequence of the vectorette primer (VP). In use, primer extension of x yields a strand which contains a vectorette priming region (VPR) suitable for hybridisation with the vectorette primer (VP) represented by the primer y. Since strand 1 does not contain a vectorette priming region itself and cannot be subject to primer extension because of the presence of the polymerisation blocking moiety and strand 2 also has no vectorette priming region, no primer extension of a vectorette primer can take place until a vectorette priming region is created by primer extension of the initiating primer.

It will therefore be appreciated that in the first amplification cycle only the primer x can produce an extension product extending up to the end of the 42 base oligonucleotide of the blocking vectorette (iii). Primer y is redundant in the first cycle since there is no complementary sequence for it to hybridise to and produce extension products. The sequence of primer y is exactly the same as bases 2 and 31 from the 5′ end of the 42 base bottom oligonucleotide in Figure 5 (i) and bases 13 and 42 inclusive of the 53 base bottom oligonucleotide in Figure 5 (ii). In the second cycle and thereafter (v and iv) the primer y can hybridise to the extension product of primer x and complementary synthesis of this extended product (iv) may proceed. Thus primer y cannot hybridise to any of the ligation products shown in (iii). It can only hybridise to an essentially completely extended product of primer x which is the known nucleotide sequence in the locus of interest. Thus only an amplification product containing primer x, unknown nucleotide sequence z and primer y is produced. The nucleotide sequence of z can be determined from both ends by using primer x and y as sequencing primers. Alternatively "nested" sequencing primers x′ and y′ may be prepared. These will contain sequence 3′ of primer x or 3′ of primer y for example bases 25 to 42 from the 5′ end of the 42 mer oligonucleotide in Figure 5(i).

Figure 6 illustrates the method of the present invention by reference to a target nucleic acid fragment/vectorette unit in which the vectorette portion contains a polymerisation blocking moiety. Similar considerations apply where the vectorette portion comprises at least a region of non-complementarity as hereinbefore described and as illustrated by way of example in Figure 5 (ii). Thus strand 1 will possess the same or at least substantially the same sequence as the region marked IP. An initiating primer (IP) will be capable of hybridising to the initiating priming region (IPR) in strand 2. Strand 2 which contains the IPR also contains a portion of nucleotide sequence which is the same or at least substantially the same as the nucleotide sequence of the vectorette primer (VP), but this portion of strand 2 does not hybridise to the corresponding portion of strand 1 because of the degree of non-complementarity deliberately introduced into strand 1. Thus the vectorette primer (VP) hybridise to strand 1 since strand 1 contains no vectorette priming region (VPR), nor can it hybridise to strand 2 because strand 2 contains a portion of sequence which is the same as VP, but no complementary sequence or VPR. Thus no vectorette primer extension products can be formed until after a vectorette priming region (VPR) has been created by primer extension of the initiating primer (IP)

The above described process is then preferably repeated using a different restriction endonuclease and different correspondingly designed blocking vectorettes to form a different pool of restriction fragment/vectorette units constituting a different vectorette library.

This process may be repeated separately for any desired number of different restriction endonucleases (for example 10 to 30, conveniently 15 to 25, advantageously about 20) and correspondingly designed blocking vectorettes to form as many different libraries of restriction fragment/vectorette units. The different libraries of restriction fragment/vectorette units may then be mixed to form a plurality of libraries of such hybrids. The plurality of libraries may then be treated with an initiating primer under hybridising conditions in the presence of nucleoside triphosphates and an agent for the polymerisation of the nucleoside triphosphates such as Taw DNA polymerase. The addition of vectorette primer under the conditions appropriate for polymerase chain reaction (PCR) as described in US Patents Nos 4,683,195 and 4,683,202 results in amplification of the loci of interest only and such amplified fragments may be subjected to sequencing, thus enabling a new initiating primer to be designed and the above-mentioned process to be repeated using the new initiating primer as a starting-point for amplification and, if desired sequencing of a further region of interest, the procedure being repeated as desired.

Figure 7 illustrates the above by showing a schematic representation of the use of vectorette libraries. Genomic DNA is digested to completion with the restriction endonuclease EcoR1. Vectorette portions with 4 base overhangs complementary to the 4 base overhangs produced by EcoR1 cleavage, are ligated onto the ends of every cleaved genomic DNA fragment. This is called an EcoR1 vectorette library. Similar libraries are constructed using for example about 15 to 20 restriction endonucleases recognising hexanucleotide sequences. When these libraries are pooled and subjected to amplification a range of PCR products may be obtained, or they can more preferably be used individually and separately to produce a single PCR product from each library.

Figure 7 (a) shows a hypothetical restriction map to the 3′ side of a locus of interest of known nucleotide sequence (w). Upon amplification of pooled vectorette libraries using primers x and y the PCR products Z, Z1, Z2, Z3 and Z4 shown in (b) are produced. Electrophoresis of these PCR products on an agarose gel would produce the band pattern shown in (c) where the PCR reactions are run separately on each vectorette library rather than on a pooled plurality of libraries. The largest PCR product visualised on the gel (Z4 in this instance) may be gel purified and its 3′ end nucleotide sequence determined using oligomer y or a "nested" oligomer y′ as the sequencing primer. This nucleotide sequence, at the 3′ end of Z4, could then serve as or be used for the synthesis of a new initiating primer x in further walking steps using this method.

Figure 8 shows the schematic application of the present invention to inverse PCR, inverse PCR having been latterly described in Triglia T. et al. Nucleic Acids Research Vol 16 1988 page 8186 and by Ochman H. et al, Genetics 120; 621-623 (November 1988).

Genomic DNA is digested with a restriction enzyme which preferably cuts relatively infrequently (eg. average fragment size 10-20 kb) such as XbaI, KpnI or BamHI. The fragments at low concentration are self ligated to form circles. The circles are then cut with a restriction enzyme which preferably cuts frequently (such as HinfI) and the fragments are ligated to a corresponding vectorette, for example a HinfI vectorette vectorette. Thus for example if sequence adjacent to a known Xba site is available, sequence adjacent to the next Xba site can be obtained by analysis of the vectorette unit product. In this way, a series of jumps could be made providing additional start points for the method of the present invention. Whilst an average fragment size of 10-20 kb is referred to above by way of example it would be preferable to cleave the target nucleic acid to give even larger fragments for example of about 100 kb. Such fragments may for example be obtained by the use of restriction enzymes which cut genomic DNA very infrequently such as NotI, BssHII, and Sal I.

Figure 11(i) is a photograph of an agarose gel showing in lanes 1 and 11 the marker ϕx174 cleaved with HaeIII, in lane 12 the marker λ/Hind III, in lane 2 a PCR control and in lane 9 the amplified fragment obtained using oligonucleotides 58 and 61 in Example 1 and as depicted in Figure 9.

Figure 11(ii) is the upper portion of a photograph of an agarose gel showing the marker λ/Hind III in lanes 1 and 13, the marker ϕx174 cleaved with Hae III in lanes 2 and 12, a PCR control in lane 3 and the amplified fragment obtained using oligonucleotides 58 and 61 in Example 1 (see Figure 9) in lane 10.

Figure 11(iii) is the lower portion of a photograph of an agarose gel showing the marker λ/Hind III in lanes 1 and 11, the marker ϕx174 cleaved with Hae III in lane 6, the amplified fragment obtained according to Example 5 in lanes 2, 3, 4 and 5 and the amplified fragment obtained according to Example 7 in lanes 7, 8, 9 and 10.

The remaining Figures are discussed in detail in the Examples hereinafter.

The present invention will now be illustrated, but not limited, by reference to the following methods and

examples in which the following oligodeoxynucleotides detailed below were used, each nucleotide sequence stated herein being read in the conventional 5′→3′ sense:-

Oligonucleotide Type A (a set of 14 base long oligonucleotides with 4 or 5 end bases at the 5′ end varying in sequence and thus suitable for use with 5′ overhangs. The 4 end bases represent the 5′ overhang whilst the fifth end base is present to destroy the restriction enzyme recognition site).

Specifically used were:

Oligonucleotide 1

CTAGGAAGGAGAGG
(for use with DNA digested with the restriction endonucleases Xba I or NheI or SpeI)

Oligonucleotide 2

AATTGAAGGAGAGG
(for use with DNA digested with the restriction endonuclease EcoR1)

Oligonucleotide 3

GATCGAAGGAGAGG
(for use with DNA digested with the restriction endonucleases BamHI or Bgl II or Xho II or BclI).

Oligonucleotide 4

AGCTGAAGGAGAGG
(for use with DNA cleaved with the restriction endonuclease Hind III)

Oligonucleotide 5

TCGAGAAGGAGAGG
(for use with DNA cleaved with the restriction endonuclease Sal I)

Oligonucleotide 6

CGCGGAAGGAGAGG
(for use with DNA cleaved with the restriction endonuclease MluI or Bss HII)

Oligonucleotide 7

GTACGAAGGAGAGG
(for use with restriction endonuclease Asp718 cleaved DNA)

Oligonucleotide 8

TCGACAAGGAGAGG
(for use with DNA cleaved with the restriction endonuclease XhoI)

Oligonucleotide 9

CATGCAAGGAGAGG
(for use with DNA cleaved with the restriction endonuclease NcoI)

Oligonucleotide 10

GGCCCAAGGAGAGG
(for use with DNA cleaved with the restriction endonuclease NotI or Eag I)

Oligonucleotide 11

CCGGTAAGGAGAGG
(for use with DNA cleaved with the restriction endonucleases Bspm II or Xma I or Acc III)

Oligonucleotide 12

CATGTAAGGAGAGG
(for use with DNA digested with the restriction endonuclease Bsp HI or Nco I)

Oligonucleotide 13

CTAGTAAGGAGAGG
(for use with DNA digested with the restriction endonucleases Avr II, NheI or Xba I)

Oligonucleotide Type B (a set of 42 base long oligonucleotides with the 3′ end base varying in sequence, the oligonucleotides being suitable for use with 5′ overhangs).

Specifically used were:-

Oligonucleotide 14

CGAATCGTAACCGTTCGTACGAGAATCGCTGTCCTCTCCTTC
This oligonucleotide is to be used with oligonucleotides 1, 2, 3, 4, 5, 6 and 7.

Oligonucleotide 15

CGAATCGTAACCGTTCGTACGAGAATCGCTGTCCTCTCCTTG
(The nucleotide sequence is the same as 14 except the 3′ terminal residue C is replaced by G). This oligonucleotide is to be used with oligonucleotides 8, 9 and 10.

Oligonucleotide 16

CGAATCGTAACCGTTCGTACGAGAATCGCTGTCCTCTCCTTT
(The nucleotide sequence is the same as 14 except the 3′ terminal residue C is replaced by T).

Oligonucleotide 17

CGAATCGTAACCGTTCGTACGAGAATCGCTGTCCTCTCCTTA
(The nucleotide sequence is the same as 14 except the 3′ terminal residue C is replaced by A). This oligonucleotide is to be used with oligonucleotides 11, 12, 13 and 18 (defined hereinafter).

Oligonucleotide 18 (10 mer)

TAAGGAGAGG
Oligonucleotide 18 is annealed to oligonucleotide 17. They are designed for use as a vectorette unit with DNA digested with a restriction endonuclease capable of creating blunt ends)
Oligonucleotides 19 and Type C series oligonucleotides are suitable for use with 3′ overhangs:

Oligonucleotide 19 (10 mer)

5′- AAA GGA GAG G-3′
This oligonucleotide is designed for use with oligonucleotides 20 to 25 as hereinafter defined.

Oligonucleotide Type C (a set of 46 base long oligonucleotides)

Oligonucleotide 20

5'-CGA ATC GTA ACC GTT CGT ACG AGA ATC GCT GTC CTC TCC TTT TGC A-3'
(for use with DNA digested with the restriction endonucleases NsiI or PstI)

Oligonucleotide Oligonucleotide 21

5'-CGA ATC GTA ACC GTT CGT ACG AGA ATC GCT GTC CTC TCC TTT AGC T-3'
(for use with DNA digested with the restriction enonucleases SstI or SacI)

Oligonucleotide 22

5'-CGA ATC GTA ACC GTT CGT AGC AGA ATC GCT GTC CTC TCC TTT CAT G-3'
(for use with DNA digested with the restriction endonuclease SphI)

Oligonucleotide 23

5'-CGA ATC GTA ACC GTT CGT ACG AGA ATC GCT GTC CTC TCC TTT GTA C-3'
(for use with DNA digested with the restriction endonuclease Kpn I)

Oligonucleotide 24

5'-CGA ATC GTA ACC GTT CGT ACG AGA ATC GCT GTC CTC TCC TTT ACG T-3'
(for use with DNA digested with the restriction endonuclease Aat II)

Oligonucleotide 25

5'-CGA ATC GTA ACC GTT CGT ACG AGA ATC GCT GTC CTC TCC TTT GGC C-3'
(for use with DNA digested with the restriction endonuclease Apa I)

Oligonucleotide Type D

This is a set of 57 base long oligonucleotides with 4 to 5 bases at the 5' end only varying in sequence.

Oligonucleotide 26

CTAGGAAGGAGAGGACGCTGTCTGTCGAAGGTAAGGAACGGAGGAGAGAAGGGAGAG
To be used with oligonucleotide 40 (defined hereinafter) and DNA digested with XbaI, Nhe I or Spe I.

Oligonucleotide 27

5'- AAT TGA AGG AGA GGA CGC TGT CTG TCG AAG GTA AGG AAC GGA GGA GAG AAG GGA GAG-3'
To be used with oligonucleotide 40 and DNA cleaved with EcoRI.

Oligonucleotide 28

5'-TCGAGA AGG AGA GGA CGC TGT CTG TCG AAG GTA AGG AAC GGA GGA GAG AAG GGA GAG-3'
To be used with oligonucleotide 40 and DNA cleaved with SalI.

Oligonucleotide 29

5'-CGC GGA AGG AGA GGA CGC TGT CTG TCG AAG GTA AGG AAC GGA GGA GAG AAG GGA GAG-3'
To be used with oligonucleotide 40 and DNA cleaved with Bss HII or MluI.

Oligonucleotide 30

5'-AGC TGA AGG AGA GGA CGC TGT CTG TCG AAG GTA AGG AAC GGA GGA GAG AAG GGA GAG-3'
To be used with oligonucleotide 40 and DNA cleaved with Hind III.

Oligonucleotide 31

5'-GAT CGA AGG AGA GGA CGC TGT CTG TCG AAG GTA AGG AAC GGA GGA GAG AAG GGA GAG-3'
To be used with oligonucleotide 40 and DNA cleaved with Bam HI, BclI or Bgl II.

Oligonucleotide 32

5'-CCG GGA AGG AGA GGA CGC TGT CTG TCG AAG GTA AGG AAC GGA GGA GAG AAG GGA GAG
To be used with oligonucleotide 40 and DNA cleaved with Acc III or BspM II.

Oligonucleotide 33

5'-TGC AGA AGG AGA GGA CGC TGT CTG TCG AAG GTA AGG AAC GGA GGA GAG AAG GGA GAG-3'
To be used oligonucleotide 40 and DNA cleaved with Apa LI.

Oligonucleotide 34

5'-TCG ATA AGG AGA GGA CGC TGT CTG TCG AAG GTA AGG AAC GGA GGA GAG AAG GGA GAG-3'
To be used with oligonucleotide 41 and DNa cleaved with Xho I.

Oligonucleotide 35

5'-GGC CTA AGG AGA GGA CGC TGT CTG TCG AAG GTA AGG AAC GGA GGA GAG AAG GGA GAG-3'
To be used with oligonucleotide 41 and DNA cleaved with Eag I or Not I or Xma III.

Oligonucleotide 36

5'-CCG GTA AGG AGA GGA CGC TGT CTG TCG AAG GTA AGG AAC GGA GGA GAG AAG GGA GAG-3'
To be used oligonucleotide 41 and DNA cleaved with Bspm II or Xma I or Acc III.

Oligonucleotide 37

5'-CAT GTA AGG AGA GGA CGC TGT CTG TCG AAG GTA AGG AAC GGA GGA GAG AAG GGA GAG-3'
To be used with oligonucleotide 41 and DNA cleaved with Bsp HI or Nco I.

Oligonucleotide 38

5'-CTA GTA AGG AGA GGA CGC TGT CTG TCG AAG GTA AGG AAC GGA GGA GAG AAG GGA GAG-3'
To be used with oligonucleotide 41 and DNA cleaved with Avr II, NheI or XbaI.

Oligonucleotide 39

5'-TA AGG AGA GGA CGC TGT CTG TCG AAG GTA AGG AAC GGA GGA GAG AAG GGA GAG-3'
To be used with oligonucleotide 41 and DNA cleaved with a restriction endonuclease capable of creating blunt ends.

Oligonucleotide Type E

This a set of 52 base long oligonucleotides with a varying 3' terminal residue.

Oligonucleotide 40

5'-CTC TCC CTT CTC GAA TCG TAA CCG TTC GTA CGA GAA TCG CTG TCC TCT CCT TC-3'
To be used with oligonucleotides 26 to 33.

Oligonucleotide 41

5'-CTC TCC CTT CTC GAA TCG TAA CCG TTC GTA CGA GAA TCG CTG TCC TCT CCT TA-3'

To be used with oligonucleotides 34 to 39.

Oligonucleotide Type F

This is a 53 base long oligonucleotide for use with the set of oligonucleotide Type G (43 to 48) as hereinafter defined and is for use with 3′ overhangs.

Oligonucleotide 42

5′-AAAGG AGA GGA CGC TGT CTG TCG AAG GTA AGG AAC GGA GGA GAG AAG GGA GAG-3′

Oligonucleotide Type G

This is a set of 57 base long oligonucleotides with 4 bases at the 3′ end varying in sequence.

Oligonucleotide 43

5′-CTC TCC CTT CTC GAA TCG TAA CCG TTC GTA CGA GAA TCG CTG TCC TCT CCT TTT GCA-3′
To be used with oligonucleotide 42 and DNA cleaved with PstI or NsiI.

Oligonucleotide 44

5′-CTC TCC CTT CTC GAA TCG TAA CCG TTC GTA CGA GAA TCG CTG TCC TCT CCT TTA GCT-3′
To be used with oligonucleotide 42 and DNa cleaved with SstI or SacI.

Oligonucleotide 45

5′-CTC TCC CTT CTC GAA TCG TAA CCG TTC GTA CGA GAA TCG CTG TCC TCT CCT TTC ATG-3′
To be used with oligonucleotide 42 and DNA cleaved with Sph I.

Oligonucleotide 46

5′-CTC TCC CTT CTC GAA TCG TAA CCG TTC GTA CGA GAA TCG CTG TCC TCT CCT TTG TAC-3′
To be used with oligonucleotide 42 and DNA cleaved with Kpn I.

Oligonucleotide 47

5′-CTC TCC CTT CTC GAA TCG TAA CCG TTC GTA CGA GAA TCG CTG TCC TCT CCT TTA CGT-3′
To be used with oligonucleotide 42 and DNA cleaved with Aat II.

Oligonucleotide 48

5′-CTC TCC CTT CTC GAA TCG TAA CCG TTC GTA CGA GAA TCG CTG TCC TCT CCT TTG GCC-3′
To be used with oligonucleotide 42 and DNA cleaved with Apa I.

The restriction endonucleases indicated are to be used for cleaving the DNA for use with that particular oligonucleotide or pair of oligonucleotides. Thus oligonucleotide 1 is to be used with for example genomic DNA cleaved with Nhe I or Spe I or Xba I.

All the oligonucleotides for use with 5′ overhangs (that is Type A, D and F (42) and oligonucleotides 18, 19 and 39 are preferably phosphorylated.

Oligonucleotides 49-57 are used in the construction of alternative vectorette units.

Oligonucleotide 49

5′-AAT TGA AGG AGA GGA CGC TGT CTG TCG AAG GTA AGG AAC GGA GGA G-3′

Oligonucleotide 50

5′-CGA ATC GTA ACC GTT CGT ACG AGA ATC GCT GTC CTC TCC TTC-3′

Oligonucleotide 51

5′-AAT TGA AGG AGA GGA CGC TGT CAG AGG ACG GTT ACG AAC GTA GGA CAG AAG GGA GAG-3′

Oligonucleotide 52

AAT TGA AGG AGA GGA CGC TGA CTG TCG AAC GTA CGG ATA GGA GTC GAG AAG GGA GTC GAG AAG GGA GAG-3′

Oligonucleotide 53

5′-AAT TGA AGG AGA GGA GAG AAG GGA CGC TGT CTG TCG AAG GTA AGG AAC GGA GGA-3′

Oligonucleotide 54

5′-CGA ATC GTA ACC GTT CGT ACG AGA ATC GCT TCC CTT CTC TCC TCT CCI TC-3′

Oligonucleotide 55

5′-AAT TGA AGG AGA GGA CGC TGT CTG TCG AAG GTA AGG AAC GGA GGA GAG AAG GGA GAG AAA GAG GAA GGG AAG-3′

Oligonucleotide 56

5′-CTT CCC TTC CTC TTT CTC TCC CTT CTC GAA TCG TAA CCG TTC GTA CGA GAA TCG CTG TCC TCT CCT TC-3′

Oligonucleotide 57

5′-AAT TGA AGG AGA GGC AGA AGG GAG AG-3′

Alternative vectorette units

Oligonucleotide 49 should be annealed to oligonucleotide 50.
Oligonucleotide 51, 52 and 57 should be annealed to oligonucleotide 40.
Oligonucleotide 53 should be annealed to oligonucleotide 54.
Oligonucleotide 55 should be annealed to oligonucleotide 56. These oligonucleotides would be used with genomic DNA cleaved with EcoRI.

**TABLE 1**

**VECTORETTE UNITS**

```
1.  5'  N1N2N3N4N5 AAGGAGAGGACGCTGTCTGTCGAAGGTAAGGAACGGAGGAG
               |   |||||||||||   |  | ||| | | | ||    |    |
    3'          N5'TTCCTCTCCTGTCGCTAAGAGCATGCTTGCCAATGCTAAGC
                          12/30  complementary

2.  5'  N1N2N3N4N5 AAGGAGAGGACGCTGTCAGAGGACGGTTACGAACGTAGGACAGAAGGGAGAG
               |   |||||||||||   |      |   |    |     |    |||||||||||
                N5'TTCCTCTCCTGTCGCTAAGAGCATGCTTGCCAATGCTAAGCTCTTCCCTCTC
                          5/30 complementary

3.  5'  N1N2N3N4N5 AAGGAGAGGACGCTGACTGTCGAACGTACGGATAGGAGTCGAGAAGGGAGAG
               |   |||||||||||   || | ||| ||| |||| || || ||||||||||||||
                N5'TTCCTCTCCTGTCGCTAAGAGCATGCTTGCCAATGCTAAGCTCTTCCCTCTC
                          20/30 complementary

4.  5'  N1N2N3N4N5 AAGGAGAGGAGAGAAGGGACGCTGTCTGTCGAAGGTAAGGAACGGAGGA
               |   ||||||||||||||||||| ||  |      | |    |  |||
                N5'TTCCTCTCCTCTCTTCCCTTCGCTAAGAGCATGCTTGCCAATGCTAAGC
                                 9/30 complementary

5.  5'  N1N2N3N4N5 AAGGAGAGGACGCTGTCTGTCGAAGGTAAGGAACGGAGGAGAGAAGGGAGAGAAAGAGGAAGG
               |   |||||||||||   |  | ||| | | | ||    ||   |||||||||||||||||||||||
                N5'TTCCTCTCCTGTCGCTAAGAGCATGCTTGCCAATGCTAAGCTCTTCCCTCTCTTTCTCCTTCC
                          13/30 complementary
```

**Table 1 continued.**

```
6.   5'  N1N2N3N4N5 AAGGAGAGGACGCTGTCTGTCGAAGGTAAGGAACGGAGGAGAGAAGGGAGAG
                    | |||||||||||   |  | ||| | | | | ||    ||   ||||||||||||
                 N5'TTCCTCTCCTGTCGCTAAGAGCATGCTTGCCAATGCTAAGCTCTTCCCTCTC
                                     13/30 complementary

7.   5'  N1N2N3N4N5 AAGGAGAGGCAGAAGGGAGAG
                    | ||||||||| |||||||||||
                 N5'TTCCTCTCC TCTTCCCTCTC
                             |  |
                             T  C

8.   5'  N1N2N3N4N5 AAGGAGAGG
                    | ||||||||
                 N5'TTCCTCTCCTGTCGCTAAGAGCATGCTTGCCAATGCTAAGC

9.   5'  N1N2N3N4N5 AAGGAGAGGACGCTGTCTGTCGAAGGTAAGGAACGGAGGAGAGAAGGGAGAG
                    | |||||||||||         |  |    |     |   ||||||||||||
                 N5'TTCCTCTCCTGGCGGCCTGCGTAGCACCGGCCGTAGTGGCCTCTTCCCTCTC  5'
                              GGCGGCCTGCGTAGCACCGGCCGTAGTGGCC  5'  79
```

```
                   Not 1
10.  5'  N1N2N3N4N5 GCGGCCGCCATCCTAATTCTGTCGAAGGTAAGGAACGGAGGAGAGAACT
                    | |||||||||||||        |     |    ||| |     | |||||||
            ________N5'CGCCGGCGGTAGGCGGCCTGCGTAGCACCGGCCGTAGTGGCCCTCTTGA
     Fok 1 cleavage site         GCGGCCTGCGTAGCACCGGCCGTAGTGGCC    5'   79
                                 Fok 1 recognition site

                   Not 1
11.  5'  N1N2N3N4N5 GCGGCCGCCATCCTAATTCTGTCGAAGGTAAGGAACGGAGGAGAGAACT
                    | |||||||||||||        |     |    ||| |     | |||||||
            ________N5'CGCCGGCGGTAGGCGGCCTGCGTAGCACCGGCCGTAGTGGCCCTCTTGA
     Fok 1 cleavage site         GCGGCCTGCGTAGCACCGGCCGTAGTGGCC    5'   79
                                 Fok 1 recognition site

12.  5'  N1N2N3N4N5 GCGGCCGCCATCC-NH2
                    | |||||||||||||
            ________N5'CGCCGGCGGTAGGCGGCCTGCGTAGCACCGGCCGTAGTGGCCCTCTTGA
     Fok 1 cleavage site         GCGGCCTGCGTAGCACCGGCCGTAGTGGCC    5'      79
                                 Fok 1 recognition site
```

In Table 1, $N^1$, $N^2$, $N^3$, and $N^4$ represent four nucleotides present to provide cohesive ends capable of ligation to a corresponding restriction site on a target nucleic acid fragment. The nucleotide $N^5$ and $N^{5'}$ are deliberately selected so as to destroy the restriction site recognition pattern. Thus for example:-

$N^1N^2N^3N^4N^5$ may represent AATTG

$N^{5'}$ C

in respect of the EcoRI restriction site, and

$N^1N^2N^3N^4N^5$ may represent AGCTG
N5' C

in respect of the Hind III restriction site.

Where $N^1$,$N^2$,$N^3$,$N^4$,$N^5$ and $N^{5'}$ represent the above mentioned EcoR1 restriction site recognition pattern, the specific vectorette units 1-10 are detailed below in Table 2, each oligonucleotide being labelled by its appropriate identication number. In vectorette units 11 and 12 of Table 2 $N^1$,$N^2$,$N^3$,$N^4$,$N^5$ and $N^{5'}$ represent the above-mentioned Hind III restriction site recognition pattern and each oligonucleotide is labelled by its appropriate identification number.

## TABLE 2

## SPECIFIC VECTORETTE UNITS

| | | | |
|---|---|---|---|
| 1. | 49 | 5' | AATTGAAGGAGAGGACGCTGTCTGTCGAAGGTAAGGAACGGAGGAG |
| | 50 | 3' | CTTCCTCTCCTGTCGCTAAGAGCATGCTTGCCAATGCTAAGC |
| 2. | 51 | 5' | AATTGAAGGAGAGGACGCTGTCAGAGGACGGTTACGAACGTAGGACAGAAGGGAGAG |
| | 40 | | CTTCCTCTCCTGTCGCTAAGAGCATGCTTGCCAATGCTAAGCTCTTCCCTCTC |
| 3. | 52 | 5' | AATTGAAGGAGAGGACGCTGACTGTCGAACGTACGGATAGGAGTCGAGAAGGGAGAG |
| | 40 | | CTTCCTCTCCTGTCGCTAAGAGCATGCTTGCCAATGCTAAGCTCTTCCCTCTC |
| 4. | 53 | 5' | AATTGAAGGAGAGGAGAGAAGGGACGCTGTCTGTCGAAGGTAAGGAACGGAGGA |
| | 54 | | CTTCCTCTCCTCTCTTCCCTTCGCTAAGAGCATGCTTGCCAATGCTAAGC |
| 5. | 55 | 5' | AATTGAAGGAGAGGACGCTGTCTGTCGAAGGTAAGGAACGGAGGAGAGAAGGGAGAGAAAGAGGAAGG |
| | 56 | | CTTCCTCTCCTGTCGCTAAGAGCATGCTTGCCAATGCTAAGCTCTTCCCTCTCTTTCTCCTTCC |

**Table 2 continued.**

```
6.   27   5'   AATTGAAGGAGAGGACGCTGTCTGTCGAAGGTAAGGAACGGAGGAGAGAAGGGAGAG
                  ||||||||||||| |  |  || | | | ||    ||   ||||||||||||
          40      CTTCCTCTCCTGTCGCTAAGAGCATGCTTGCCAATGCTAAGCTCTTCCCTCTC

7.   57   5'   AATTGAAGGAGAGGCAGAAGGGAGAG
                  ||||||||||  ||||||||||
          40      CTTCCTCTCC  TCTTCCCTCTC
                           |  |
                           T  C

8.    2   5'   AATTGAAGGAGAGG
                  ||||||||||
          14      CTTCCTCTCCTGTCGCTAAGAGCATGCTTGCCAATGCTAAGC

9.   27   5'   AATTGAAGGAGAGGACGCTGTCTGTCGAAGGTAAGGAACGGAGGAGAGAAGGGAGAG
                  ||||||||||||      |  |   |   |  ||||||||||||
          78      CTTCCTCTCCTGGCGGCCTGCGTAGCACCGGCCGTAGTGGCCTCTTCCCTCTC   5'
                           GCGGCCTGCGTAGCACCGGCCGTAGTGGCC   5'   79
```

```
                          Not 1
10.  80   5'      AATTGGCGGCCGCCATCCTAATTCTGTCGAAGGTAAGGAACGGAGGAGAGAACT
                    ||||||||||||||         |     |  ||  |     | |||||||
     81           ____CCGCCGGCGGTAGGCGGCCTGCGTAGCACCGGCCGTAGTGGCCCTCTTGA   5'
          Fok 1 cleavage site        GCGGCCTGCGTAGCACCGGCCGTAGTGGCC   5'   79
                                Fok 1 recognition site

                          Not 1
11.  82   5'      AATTGGCGGCCGCCATCCTAATTCTGTCGAAGGTAAGGAACGGAGGAGAGAACT
                    ||||||||||||||         |     |  ||  |     | |||||||
     81           ____CCGCCGGCGGTAGGCGGCCTGCGTAGCACCGGCCGTAGTGGCCCTCTTGA   5'
          Fok 1 cleavage site        GCGGCCTGCGTAGCACCGGCCGTAGTGGCC   5'      79
                                Fok 1 recognition site

12.  83   5'      AGCTGGCGGCCGCCATCC-NH2
                    ||||||||||||||
     81           ____CCGCCGGCGGTAGGCGGCCTGCGTAGCACCGGCCGTAGTGGCCCTCTTGA
          Fok 1 cleavage site        GCGGCCTGCGTAGCACCGGCCGTAGTGGCC           79
                                Fok 1 recognition site
```

Oligonucleotides 58, 59 and 60 may be used with all constructs. Oligonucleotide 58 is a universal vectorette primer.

eg.

```
(40)    CTTCCTCTCCTGTCGCTAAGAGCATGCTTGCCAATGCTAAGCTCTTCCCTCTC
     3'              ||||||||||||||||||||||||||||||           5'
                     TCGCTAAGAGCATGCTTGCCAATGCTAAGC
                  3'             58              5'
```

Oligonucleotide 59 is a universal sequencing primer and a nested vectorette primer. eg.

```
        (40) ---------CTTCCTCTCCTGTCGCTA-----------
                        ||||||||||||||
                   3'   TTCCTCTCCTGTCGC   5'
                             59
```

Oligonucleotide 60 is a nested universal vectorette primer. eg.

```
    (40)  3'  CTTCCTCTCCTGTCGCTAAGAGCATGCTTGCCAA-----
                CCTCTCCTGTCGCTAAGAGCATGCTTGCCA
            3'              60              5'
```

Short oligonucleotides are the 14mer + 42mer annealed.

Long oligonucleotides are the 57mer + 53mer annealed.

Type A and Type D are equivalent short and long oligonucleotides respectively. Therefore 1 and 26 have the same overhang and are both to be used with DNA cleaved with Nhe I, Spe I or Xba I. Similarly 2 and 27 are both to be used with DNA cleaved with EcoRI.

Exactly the same applies to Type B and Type E, 19 and Type F (42) and Type C and Type G.

Each oligonucleotide is to be ligated to DNA cleaved with the restriction endonuclease indicated next to that oligonucleotide. Types A and B (and Type D and Type E) are for use with 5′ overhangs.

Oligonucleotides 19 + Type C (and Type F + Type G) are for use with 3′ overhangs.

Oligonucleotides 1 to 8 are paired with oligonucleotide 14.

9 to 13 and 18 are paired with oligonucleotide 17.

19 is paired with oligonucleotides 20 to 25.

26 to 33 are paired with oligonucleotide 40.

34 to 39 are paired with oligonucleotide 41.

Type F (42) paired with Type G oligonucleotides 43 to 48.

Oligonucleotide 7 (and 32) may be replaced by oligonucleotide 11 (and 36) for use with Bspm II restricted DNA.

It will be appreciated that oligonucleotides 1 to 7 are for annealing to oligonucleotide 14. Oligonucleotides 8 to 10 are for annealing to oligonucleotide 15. Oligonucleotides 11 to 13 are for annealing to oligonucleotide 17. It may not be necessary to use oligonucleotide 16.

Oligonucleotide 58

CGAATCGTAACCGTTCGTACGAGAATCGCT

(a 30 base long oligonucleotide intended as the "universal vectorette primer" in the polymerase chain reaction (PCR) outlined in step 6 of Method I hereinafter).

Additional Oligonucleotides used in the Examples hereinafter

These oligonucleotides may be used with any vectorette library irrespective of the restriction endonuclease employed except for oligonucleotide 65 as detailed below:-

Oligonucleotide 61

GAGACTTGGTATTTTGTTCAATCATTAAG
(a 3′ oligonucleotide of exon V of the α-1 antitrypsin gene.

Oligonucleotide 62

AAAAGCCAGAGACCTCACTCCCGGGGAGCC
(a 5′ oligonucleotide of exon 1 of the human Phenylalanine Hydroxylase gene, mutations in which cause phenylketonuria (PKU)).

Oligonucleotide 63

AGGGACTTACTGTGGCGAGCTTTTCAATGT
(a 3′ oligonucleotide of exon 9 of the phenylalanine hydroxylase (PAH) gene)

Oligonucleotide 64

GGGCCTCAGTCCCAACATGGCTAAGAGGTG
(a 3′ oligonucleotide of exon III of the α1 antitrypsin gene)

Oligonucleotide 65

AATTTCACACAGGAAACAGCTATGACCATG
(an extended M13 reverse primer).
This is to be used as one of the PCR primers for amplification of inserts in either M13 DNA or pUC plasmid DNA.

Oligonucleotide 66

5′ATC AGG TGC ACC CAG AGA GGC AAG GCC-3′
(a 3′ oligonucleotide of exon 9 of the phenylalanine hydroxylase (PAH)gene).

Method I

Method I comprises the following six steps:-

Step I - Phosphorylation of oligonucleotide Type A, followed by gel purification of the phosphorylated species.

Step 2 - Addition of a ddA residue to the 3′ end of the phosphorylated oligonucleotide Type A of step 1 followed by gel purification.

Step 3 - Annealing of the modified oligonucleotide Type A to the corresponding oligonucleotide Type B.

Step 4 - DNA preparation and restriction digestion.

Step 5 - Ligation of the annealed oligonucleotides Type A and Type B to the cleaved dephosphorylated DNA of step 4.

Step 6 - Amplification of the ligation products of step 5 and detection and analysis of the PCR products.

Such steps may for example be effected as follows:-

Step 1

The following buffers and other standard solutions were used:-

I.1 10X kinase buffer

0.5M Tris.HCl (pH 7.6)
0.1M $MgCl_2$
50 mM dithiothreitol
1 mM Spermidine
1 mM EDTA
stored at -20°C.

I.2 Adenosine 5′-triphosphate

Sodium salt as a 10 mM solution (Pharmacia)
Diluted to 20 pMol/µl with water.
stored at -20°C.

I.3 Radioisotopes

a) Adenosine 5′-[$\gamma^{32}$P]triphosphate (Amersham) Specific activity approx 6000 Ci/mmol.
b) Dideoxyadenosine 5′-[$\alpha^{32}$P]triphosphate (Amersham) Specific activity approx 3000 Ci/mmol.

I.4 Formamide Sample Buffer

80% (v/v) deionised formamide
50 mM Tris.borate pH 8.3
1 mM EDTA
0.1% (w/v) Bromophenol Blue
0.1% (w/v) xylene cyanol
The formamide was deionised by stirring for 30 minutes with 2 g Amberlite MB-1 resin (Bio-Rad) and filtering off using a Whatman number 1 filter. The buffer was stored in the dark.

I.5 40% Stock Acrylamide per 500 ml;

Acrylamide (electrophoretic grade 190 g)
Bis-acrylamide (electrophoretic grade 10 g)
The acrylamide was stirred for 30 minutes with 5 g of Amberlite MB-1 resin (Bio-Rad) and filtered using two sheets of Whatman Number 1.
The stock solution was stored in the dark at 4°C.

I.6 10% Ammonium Persulphate

1 g of ammonium persulphate (Sigma, electrophoresis grade) was dissolved and made up to 10 ml in sterile deionised water. It was stored in the dark.

| I.7 0.5X Gel mix | |
|---|---|
| (20% acrylamide, 7M urea) per 50 ml: | |
| 21 g<br>25 ml<br>2.5 ml | Urea<br>40% stock acrylamide (1.5)<br>10 x TBE (1.8) |
| Just before use add polymerising agents: | |
| 300 µl<br>40 µl | 10% ammonium persulphate (1.6)<br>TEMED |

| I.8 10X TBE per litre:- | |
|---|---|
| Tris | 109 g |
| EDTA | 9.5 g |
| Boric Acid | 55 g |
| 10x TBE buffer was diluted in water to 1 x or 0.5x strength as required. | |

| I.9 Buffer A | |
|---|---|
| 0.1M | Tris.HCl pH 7.7 |
| 10 mM | Triethylamine |
| 1 mM | EDTA |
| The buffer was stored at 4°C and made up fresh once a month. | |

| I.10 Reagent B per 100 ml:- | |
|---|---|
| 50 ml | ethanol |
| 50 ml | sterile deionised water. |

<u>Phosphorylation of the 14 mer oligonucleotide Type A</u>

The following were mixed in a 1.5 ml Starstedt tube:

| | |
|---|---|
| Oligonucleotide Type A | 100 pmoles |
| 10X kinase buffer (1.1) | 5 µl |
| Adenosine 5′-triphosphate (1.2) | 9.5 µl (190 pmoles) |
| Adenosine 5′-[$\gamma^{32}$P]triphosphate (1.3) | 5 µl (10 pmoles) |

T4 Polynucleotide kinase E.coli B (Amersham) 40 units
$H_2O$ to 50 µl.

The ratio of oligonucleotides to ATP molecules was kept at 1:2.

Phosphorylation of the oligonucleotides was carried out by incubation at 37°C for 60 minutes.

20 µl of the formamide sample buffer (1.4) was added and the enzyme sample was denatured in a 90°C water bath for 10 minutes prior to loading on a 20% acrylamide 7M urea denaturing gel.

Gel purification of the kinased oligonucleotides was performed Phosphorylated oligonucleotides were purified by electrophoresis on denaturing polyacrylamide gels as described by Wu R. et al "Purification and sequence analysis of synthetic oligonucleotides" in Oligonucleotide Synthesis - a practical approach, Edited by M J Gait, IRL Press.

<u>c) Final purification through a column</u>

NeNsorbTM (DuPont) nucleic acid purification cartridges were employed for this final purification step.

1) pre-equilibration of the column.

The column was clamped to a clamp stand. The inside of the cartridge was rinsed with 2 ml of HPLC grade methanol. This washed any loose packing back onto the column bed.

The adaptor was securely pressed into the top of the cartridge forming an air tight seal. A disposable plastic syringe was filled with air and attached to the adaptor. With constant gentle pressure all the methanol was pushed through the cartridge (flow rate 1 drop per 2 seconds). In the same way the column was 'primed' by flowing 2 ml of buffer A (1.9) through it.

2) Sample loading.

2.8 µl of triethylamine were added to the 2 ml of water containing the oligonucleotide. This was then added directly to the top of the column bed using a syringe and needle. The sample was pushed through the column using a syringe filled with air as in C(1).

3) Sample wash.

3 ml of buffer A (1.9) was added to the column using a syringe and needle. This was gently forced through the column as in C(1).

4) 1 ml of reagent B (1.10) was applied to the top of the column. The DNA was eluted from the column bed by gentle force using a syringe filled with air as in C(1). The effluent was collected in a 1.5 ml Sarstedt tube and evaporated to dryness in a centrifuge to which a vacuum is applied (UNIVAP, Uniscience).

The dried, purified oligonucleotide was resuspended as required.

<u>STEP 2</u>

The following buffers and other standard solutions were used:-

<u>2.1</u>

1.4M Sodium Cacodylate
pH 7.5 with HCl
Stored at -20°C.

<u>2.2</u>

5 mM cobaltous chloride
Stored at -20°C.

<u>2.3</u>

1 mM dithiothreitol
Stored at -20°C

<u>2.4</u>

Dideoxyadenosine 5′-triphosphate Lyophilised sodium salt.
(Boehringer Mannheim)
Diluted to 10 mM stock solution in $H_2O$
and 1 mM working solution in $H_2O$

2.5

0.5 M EDTA pH 8.0

Addition of a dideoxyadenosine residue to the 3′-end of the oligonucleotide Type A.

Oligonucleotide Type A was phosphorylated and the phosphorylated species purified as described in step 1. With 80% recovery, there are 80 pmoles of oligonucleotide kinased and available for the terminal transferase reaction.

The 80 pmoles of kinased and purified oligonucleotide I were resuspended in 48 µl of sterile deionised water.

The following were added to the kinased oligonucleotide.

| | |
|---|---|
| (48 µl | oligonucleotide (80 pmol) |
| 1 µl | 1 mM ddATP (1 nmol) (2.4) |
| 10 µl | 1.4M Cacodylic acid (2.1) |
| 10 µl | 1 mM DTT (2.3) |
| 20 µl | 5 mM Cobaltous chloride (2.2) |
| 5 µl | ddATP ($\alpha^{32}P$) (15 pmol) (1.3b) |
| 6 µl | Terminal Transferase (150 units of Boehringer TdT) |

The reaction mixture was incubated at 31°C. for 1 hr and the reaction was then terminated by the addition of 4 µl of 0.5M EDTA.

40 µl of the formamide sample buffer was added (1.4) and the sample denatured by incubating in a 90°C water bath for 10 minutes prior to loading on a 20% acrylamide, 7M urea denaturing gel.

Gel electrophoresis was exactly as described in step 1.

The now 15 mer oligonucleotide Type A band was cut out and purified exactly as described in step 1. The final recovery is estimated to be 50 pmoles. This is resuspended in 250 µl of sterile deionised water to give a concentration of 0.2 pmol/µl.

Step 3

Annealing of the modified oligonucleotide Type A to the corresponding oligonucleotide Type B

The following solution was required.

| 3.1 10 x Annealing buffer. | |
|---|---|
| 100 mM | Tris HCl (pH 8.0) |
| 100 mM | $MgCl_2$ |

The following were mixed in a 1.5 ml screw cap Sarstedt tube:-

25 pmoles of modified Type A oligonucleotide (obtained via steps 1 and 2)
25 pmoles of Type B oligonucleotide
5 µl 10 x annealing buffer (3.1)
and $H_2O$ to 50 µl.

If the Type A oligonucleotide is selected from oligonucleotide 1, 2, 3 or 4 then a convenient Type B oligonucleotide for these (1-4) will be oligonucleotide 14.

The Eppendorf tube was placed in a boiling water bath (conveniently a small beaker). After 5 minutes, the water in the beaker was allowed to cool slowly to room temperature over a period of 2 hours. Finally the contents of the tube were collected at the bottom by centrifugation for 10 seconds. The annealed oligonucleotide, at a concentration of 0.5 pmol/µl is ready for use in ligations.

Step 4

DNA preparation and restriction

The following DNA Samples were used:-

1. Human Genomic DNA I

2. Human genomic DNA II (Genomic DNA prepared from lymphoblastoid cells) to which 0.1 mg/ml of 5-azacytidine had been added to the culture medium to prevent methylation at the cytidine residues during replication of DNA and cell growth.

The following buffers were used:

| 4.1 10x Low salt buffer | |
|---|---|
| 100 mM | Tris-HCl pH 7.5 |
| 100 mM | $MgCl_2$ |
| 10 mM | Dithioerythritol |

| 4.2 10x Medium Salt buffer | |
|---|---|
| 100 mM | Tris-HCl pH 7.5 |
| 100 mM | $MgCl_2$ |
| 500 mM | NaCl |
| 10 mM | Dithioerythritol |

| 4.3 10x High Salt buffer | |
|---|---|
| 500 mM | Tris-HCl pH 7.5 |
| 100 mM | $MgCl_2$ |
| 1000 mM | NaCl |
| 10 mM | Dithioerythritol |

| 4.4 10X BamH1 buffer | |
|---|---|
| 100 mM | Tris-HCl pH 8.0 |
| 50 mM | $MgCl_2$ |
| 1000 mM | NaCl |
| 10 mM | 2-mercaptoethanol. |

| 4.5 10x EcoR1 buffer | |
|---|---|
| 100 mM | Tris-HCl pH 7.5 |
| 100 mM | $MgCl_2$ |
| 500 mM | NaCl |

| 4.6 TE buffer | |
|---|---|
| 10 mM | Tris-HCl pH 7.5 |
| 1 mM | EDTA |

| 4.7 Agarose Sample Buffer | |
|---|---|
| 15% (w/v) | Ficoll 400 |
| 0.05% (w/v) | Bromophenol Blue |
| 0.05% (w/v) | Xylene Cyanol |

The sample buffer was made up in 1X TBE buffer (1.8).

| 4.8 10X CIP Buffer | |
|---|---|
| 500 mM | Tris-HCl pH 8.0 |
| 1 mM | EDTA |

(i) The following conditions were employed for cleaving the genomic DNA with specific restriction endonucleases:-

50 µg of DNA

20 µl of the appropriate 10X restriction buffer.

50 units of the desired restriction endonuclease.

The final volume of the reaction mixture was adjusted to 200µl.

Digestion was effected at 37°C for 2 hrs. The reaction was terminated by the addition of 4 µl of 0.5M EDTA (2.5) and heating the sample to 70°C for 10 minutes.

(ii) 1 µg of the sample was checked by agarose gel electrophoresis to ensure complete digestion:-

Agarose gel electrophoresis

The gels were made 0.7% (w/v) agarose (Pharmacia) in 1X TBE (1.8) buffer and submerged in the same strength TBE buffer for electrophoresis. A fifth-volume of agarose sample buffer (4.7) was added to the sample prior to loading into the wells of the gel. Following electrophoresis the gel was stained for 10 minutes by submerging in deionised water containing 1 µg/ml ethidium bromide. The DNA was visualised at 302 nm using a transilluminator (Macrovue-LKB).

(iii)(a) The digested DNA was purified by phenol extraction:

An equal volume of buffered redistilled phenol (BRL, nucleic acid grade phenol buffered with TE(4.6)) was added to the restricted DNA and the mixture vortexed for 30 seconds. The white emulsion formed was separated into two phases by centrifugation for 5 minutes. The aqueous phase was transferred to a fresh tube. Any residual phenol was removed after first extracting the aqueous layer with phenol: chloroform: isoamylalcohol (25: 24: 1) - an equal volume-, with an equal volume of chloroform: isoamylalcohol (24:1).

(b) The DNA was finally purified by ethanol precipitation:

A tenth-volume of 3M sodium acetate was added to the DNA solution to give a final concentration of 300 mM sodium acetate. The DNA was precipitated by the addition of 2.5 volumes of 95% ethanol and then standing at -80°C for 30 minutes followed by centrifugation (microcentrifuge for 5 minutes, 12,000 rpm). The pelleted DNA was washed with 80% (v/v) cold ethanol and dried under vacuum. The DNA was dissolved in buffer as required.

(iv) Dephosphorylation of the cleaved DNA

The cleaved DNA was dephosphorylated to prevent self ligation.

The restricted and purified DNA was dissolved in 175 µl sterile deionised water.
To this DNA were added:

| | |
|---|---|
| 5 µl | of Calf intestine phosphatase (CIP) (Boehringer Mannheim ) (1 unit/µl) |
| 20 µl | of 10X CIP buffer (4.8) |

The sample was incubated at 37°C for 60 minutes. 4 µl of 0.5M EDTA was added and the dephosphorylated DNA was purified by phenol extraction and ethanol precipitation as described in (iii)(a) and (iii)(b) above. The final DNA pellet was dissolved in 50 µl of TE buffer (4.6) to give a concentration of 1 µg/µl.

Step 5

Ligation of the annealed oligonucleotides I & II to the cleaved dephosphorylated DNA

The following buffer was used

| 5.1 10X ligation buffer | |
|---|---|
| 200 mM | Tris-HCl pH 7.4 |
| 100 mM | $MgCl_2$ |
| 100 mM | DTT |
| 10 mM | ATP |

The following were mixed in a 1.5 ml Sarstedt tube

| | |
|---|---|
| Cleaved genomic DNA (7.4 pmole) | 10µg |
| Annealed oligonucleotides from step 3 | 11 pmole |
| 10X ligation buffer | 10 µl |
| T4 DNA ligase (Boehringer Mannheim) | 2 units |
| Sterlised deionised water to bring volume to 100 µl | |

The ligation mix was left at 4°C overnight. Efficiency of ligation was estimated by gel electrophoresis of 5% of the ligation products - see Step 4 (ii) for procedure details.

The ligation products were then purified by phenol extraction and ethanol precipitation as described in Step 4(iii). The final DNA pellet was dissolved in 9.5 µl of TE buffer (4.6) to give a concentration of 1 µg/µl.

Step 6

Amplification of the ligation products of Step 5

The following buffers and other standard solutions were used

| 6.1 10X Amplification buffer | |
|---|---|
| 500 mM | KCl |
| 100 mM | Tris-HCl pH8.3 |
| 15 mM | $MgCl_2$ |
| 0.1% | gelatin |
| Stored at -20°C. | |

| 6.2 Nucleotides | |
|---|---|
| 10X stock | |
| 2 mM | dATP |
| 2 mM | dTTP |
| 2 mM | dGTP |
| 2 mM | dCTP |
| Stored at -20°C. | |

6.3 Size markers

Bacteriophage φX174 cleaved with HaeIII;
Size of bands in base pairs is:-
1353, 1078, 872, 603, 310, 281, 271, 234, 194, 118 and 72.

The following were mixed in a 1.5 ml screw cap Sarstedt tube:-
1 µg of ligation products from step 5.
100 pmoles of oligonucleotide 58
100 pmoles of oligonucleotide 61 or 62 or 63
10 µl 10X Amplification buffer (6.1)
10 µl 10X Stock nucleotides.

The volume was made up to 100 µl with distilled deionised water.

The tube was placed in a boiling water bath for 5 minutes. Taq polymerase (Anglian) was diluted to 1 unit/µl in 1X Amplification buffer (6.1).

2 µl of the diluted enzyme were added to the above boiled reaction mix, and mixed. The contents were spun to the bottom in a microcentrifuge for 5 seconds. 50 µl of light mineral oil (Sigma) was then placed on the reaction mixture (to prevent evaporation during the following reactions).

The tube was placed in a water bath at:

1) 60°C for 2 minutes

2) 72°C for 3 minutes

3) 91°C for 2 minutes Steps 1), 2) and 3) were then repeated 39 times. The tube was then placed in the water bath at 60°C for 2 minutes followed by incubation for 8 minutes at 72°C. At the end of the amplification the contents were spun to the bottom and the mineral oil pipetted off.

15 µl of the amplified products were analysed by agarose gel electrophoresis as described in Step 4(ii). To estimate the size of amplified products(s) φX174 cleaved with Hae III (6.3) was electrophoresed adjacent to the amplified sample.

METHOD II

Method II comprises the following steps:-

1. Adding a ddA residue to the 3′ end of oligonucleotide Type A followed by gel purification of the 3′ end labelled species.

2. Phosphorylating the 3′ end labelled oligonucleotide Type A followed by separation from unincorporated nucleotides.

3. Annealing of the modified oligonucleotide Type A to the corresponding oligonucleotide Type B.

4. Cleavage of the genomic DNA.

5. Ligating the annealed oligonucleotides A + B to the cleaved DNA.

6. Amplification of the ligation products. Detection and analysis of the PCR products.

The above steps may for example be effected as follows:-

Step 1

Experimental details are exactly the same as step 2 of Method I.

Step 2

The oligonucleotide is phosphorylated as described in step I of Method I. The phosphorylated oligonucleotide is purified directly through a column as detailed in Step I (ii)C of Method I.

Prior to loading the sample on the column, 200 µl of buffer A(1.9) is added to the reaction mix in place of triethylamine.

Steps 3-6 are exactly the same as in Method I.

METHOD III

The following steps are involved:-

1. Phosphorylating the 14 mer oligonucleotide Type A followed by gel purification of the phosphorylated species.

2. Adding a ddA residue to the 3′ end of the kinased oligonucleotide I followed by gel purification.

3. Annealing of the oligonucleotide Type A to the corresponding oligonucleotide Type B.

4. Restriction of the DNA.

5. Ligating the annealed oligonucleotides A + B to the restricted DNA.

6. Addition of a dideoxynucleotide to free 3′ ends.

7. Amplification of the ligation products. Detection and analysis of the amplification products.

Such steps may for example be effected as follows:-

Steps 1 to 5 are exactly as detailed in METHOD I.

Step 6

The purified ligation products from Step 5 are dissolved in 30 µl of distilled deionised water. To the DNA are added the following:-

5 µl of 10x amplification buffer (6.1)

15 pmoles of the appropriate dideoxynucleotide

1 unit of Taq polymerase (Anglian)

water added to bring volume to 50 µl.

The reaction mix is incubated at 37 °C for 30 minutes.

The DNA is purified by phenol extraction and ethanol precipitation as described in Method I Step 4 (iii).

The DNA pellet is dissolved in 50 µl of TE buffer (4.6) to give a concentration of 1 µg/µl.

Step 7 is the same as Step 6 of METHOD I.

METHOD IV

Exactly the same as METHOD II except that Step 6 of METHOD III is inserted between steps 5 and 6 of METHOD II.

METHOD V

Method V comprises the following steps:-

1. Phosphorylating the 57-mer Type D oligonucleotide followed by its separation from unincorporated nucleotide.

2. Annealing of the phosphorylated, for example kinased Type D oligonucleotide to the appropriate Type E oligonucleotide. Followed by Step 4 onwards of Methods I-IV.

Such steps may for example be effected as follows:-

1. The oligonucleotide is phosphorylated as described in Method I step I. Purification is through a NeNsorbTM column as described in Method I Step I(ii)C.

2. Annealing is as detailed in Method I. Step 4 onwards are as detailed previously (METHODS I and III).

Method VI

Method VI comprises the following steps:-

1. Phosphorylating either the Type A 14 mer oligonucleotide (or oligonucleotides 18 or 19 or 39 or Type F (42) or Type D followed by gel purification of the phosphorylated species.

2. Annealing of the phosphorylated oligonucleotide for 18, 19 or Type D to the corresponding appropriate oligonucleotide Type B (or Type C) or Type E, or Type G.

3. Restriction enzyme digestion of the genomic DNA.

4. Ligation of the annealed oligonucleotides to the restricted DNA.

5. Restriction of ligation products from above step.

6. Ligation of more annealed oligonucleotides to products from step (5).

7. Repeat of steps (5) and (6).

8. Final restriction of products from step (7).

9. Addition of a dideoxynucleotide triphosphate to the 3′ end of the products from step (8).

10. Amplification of the products from step (9). Detection and analysis of the amplified products.

Such steps may for example be effected as follows:-

Step 1

As described in step 1 of method I.

Step 2

This step is the same as described in step 3 of method I.

For example:-

Oligonucleotides 1 to 7 are annealed to 14
Oligonucleotides 11 to 13 and 18 are annealed to 17
Oligonucleotides 20 to 25 are annealed to 19
Oligonucleotides 26 to 32 are annealed to 40
Oligonucleotides 24 to 39 are annealed to 41
Oligonucleotides 43 to 48 are annealed to 42

Step 3

This step is the same as step 4(i), 4(ii) and 4(iii) of method I but restricted DNA is not dephosphorylated.

Step 4

Ligating the annealed oligonucleotides to the cleaved DNA Buffers and other standard solutions:

| 4.1 10x ligation buffer | |
|---|---|
| 200 mM | Tris-HCl pH 7.4 |
| 100 mM | $MgCl_2$ |
| 100 mM | DTT |
| 10 mM | ATP |

(i) The calculation of pmoles of sticky ends in 1µg of cleaved DNA is as set out in step 5 of method I.

(ii) Ligations

The following were mixed in a 1.5ml Sarstedt tube:

| | |
|---|---|
| Cleaved genomic DNA 7.4pmoles ( 10µg) | |
| Annealed oligonucleotides from step 2 | 11 pmoles |
| 10x ligation buffer (4.1) | 10µl |
| T4 DNA ligase (Boehringer Mannheim) | 2 units |
| Sterilised deionised water to bring volume to | 100µl |

The ligation mix was left at 4°C overnight.

Efficiency of ligation was estimated by gel electrophoresis of 5% of the ligation products - see step 4(ii) of method I for the procedure details.

The ligation products were purified by phenol extraction and ethanol precipitation as described in section 4(iii) or method I.

Step 5

This step is the same as step 3 above.

Step 6

This step is the same as step 4 above except equimolar concentrations of DNA to annealed oligonucleotides are used.

For example

7.4 pmoles of DNA

7.4 pmoles of annealed oligonucleotides

Step 7

Repeat of steps 5 and 6 above.

Step 8

As step 5 above.

Step 9

Buffers and other standard solutions

| 9.1 T7 DNA polymerase buffer (5x) | |
|---|---|
| 200 mM | Tris. HCl pH7.5 |
| 100 mM | $MgCl_2$ |
| 250 mM | NaCl |

| 9.2 dNTP-dATP | |
|---|---|
| 1 mM | dTTP |
| 1 mM | dGTP |
| 1 mM | dCTP |
| made up in sterilised deionised water. | |

| 9.3 ddATP | |
|---|---|
| 1 mM | ddATP |
| made up in sterilised deionised water. | |

(i) Addition of a ddA residue to 3′ ends of products from step 8:-

The following were mixed in a 1.5ml Sarstedt tube:-

4µg of DNA from step 8 above
20µl of 5x T7 DNA polymerase buffer (9.1)
20µl of dNTP-dATP (9.2)
20µl of ddATP (9.3)
5 units of T7 DNA polymerase (Sequenase)

Volume made up to 100µl with distilled deionised water.

The tube was incubated at 37°C for 30 minutes. The enzyme was then denatured by incubation at 70°C for 10 minutes.

The DNA was then purified by phenol extraction and ethanol precipitation as described previously.

Step 10

This step is the same as step 6 of method I or the amplification may be carried out on a temperature cycling machine (for example the Techne programmable Dri-Block PHC-1.)

It will be appreciated that in this method VI dideoxyadenosine-5′-triphosphate is reacted with the 3′ ends of the ligated oligonucleotides in the presence of T7 DNA polymerase (Sequenase) in order to replace any loss of 3′ terminal ddA residues as a consequence of an unexpected exonuclease activity of the T4 DNA ligase specific for dideoxynucleotides.

Method VII

Method VII is the same as Method VI with the exception that step 1 of method VI is omitted. Further, in this method no gel purification is effected, but the efficiency of ligation may be checked by agarose gel electrophoresis and ethidium bromide staining.

Method VIII

Method VIII is the same as Method VI with the exception that step 9 is omitted. The oligonucleotides used in this method are for example as follows:-
26 to 32 annealed to 40
36 to 39 annealed to 41
43 to 48 annealed to 42

Method IX

Method IX comprises the following steps:-

1. Phosphorylation
2. Annealing
3. Restriction of DNA
4. Ligation (for example at 25°C) of annealed oligonucleotides to cleaved DNA
5. Restriction/ligation
6. Amplification of the products from step 5 and detection and analysis of the amplification products.

The above steps may for example be effected as described in relation to the corresponding steps in Method VI.

Method X

Steps 1, 2 and 3 of Method X are the same as steps 1, 2 and 3 of method VIII. Step 4(i) of method X is also the same as step 4(i) of method VIII but step 4(ii) is effected as follows:-

4(ii) Ligations

The following were mixed in a 0.5ml Sarstedt tube:-

| | |
|---|---|
| Restricted 7387 DNA | 0.74 pmoles (1µg) |
| Annealed oligonucleotides | 1.65 pmoles |
| 10x ligation buffer (4.1) | 1µl |
| 10x ATP (4.2) | 1µl |
| T4 DNA ligase (Boehringer Mannheim) | 1 unit |
| $H_2O$ to | 10µl |

The ligation mix was left at 25°C (in the Techne Dri-Block) for 120 minutes.

The ligation mix was then diluted in water to a DNA concentration of 10ng/µl and subjected to the amplification reaction as described in Step 10 of Method VIII hereinbefore.

Method XI

Method XI is the same as method X except as follows:-

Following the ligation reaction at 25°C for 120 minutes the ligation products are digested with the restriction endonuclease by the addition of:- 2µl of buffer for the appropriate restriction endonuclease (see

3.1)
2 units of restriction endonuclease
$H_2O$ to 20µl
and incubation at 37°C for 60 minutes.

The sample was then diluted in $H_2O$ to a DNA concentration of 10ng/µl.

Method XII

Method XII is the same as Method X except that in step 4(ii), the ligation mix is left at 4°C overnight rather than at 25°C for 2 hours.

Finally the ligation mix is diluted in water to a DNA concentration of 10ng/µl.

Method XIII

Method XIII is the same as method XII except that following the overnight ligation reaction at 4°C, the ligation products are digested with the appropriate restriction endonuclease.
This is done by:
adding to the 10µl ligation mix
2µl buffer for the appropriate endonuclease (see 3.1)
2 units of restriction endonuclease (Boehringer Mannheim)
$H_2O$ to 20µl
and incubation at 37°C for 60 minutes.

The sample is then diluted in $H_2O$ to a DNA concentration of 10ng/µl.

Example 1

Oligonucleotides 1 and 14 were prepared as described in Steps 1 and 2 of Method I above. These oligonucleotides for use in Method I were annealed as described in Step 3 above. Human genomic DNA II was prepared and cleaved as described in Step 4 of Method I. Human genomic DNA II cleaved with restriction endonuclease Xba 1 and dephosphorylated (as detailed in Step 4 above) was ligated with the annealed oligonucleotides 1 and 14, exactly as described in Step (5) above.

Amplification was then performed on the ligation products.

Figure 11(i) as hereinbefore described shows the marker ϕX174 cleaved with Hae III in lanes 1 and 11, the marker λ Hind III in lane 12, an amplification control in lane 2 and the amplified fragment obtained using oligonucleotides 58 and 61. This amplified fragment is 800 bp in length as expected.

Figure 11(ii) also shows the market λ Hind III in lanes 1 and 13, the marker ϕX174 cleaved with Hae III in lanes 2 and 12, an amplification control in lane 3 and the amplified fragment obtained using oligonucleotides 58 and 61 in lane 10.

Example 2

Using oligonucleotides 1 and 14 with DNA cleaved with the restriction endonuclease Xba I.

a) Oligonucleotide 1 (100 pmoles) was phosphorylated and the kinased species purified as described in Step 1 Method I.

b) The kinased oligonucleotide 1 was terminal transferased to add a dideoxyadenosine residue to the 3′ end of the oligonucleotide. The resulting 15-mer oligonucleotide 1 (+ddA at 3′ end) was purified as detailed in Step 2 of Method I.

c) Oligonucleotide 1 was hybridised to oligonucleotide 14.

d) Human genomic DNA II was cleaved with the restriction endonuclease XbaI and dephosphorylated as detailed in Method I.

e) The annealed oligos 1 and 14 were ligated to human genomic DNA II restricted with XbaI - see Method I for details.

f) Amplification was carried out on the ligation products using oligonucleotides 58 and 61. The expected amplified product in this case is approximately 800 bp.

Example 3

Using oligonucleotide 2 and 14 with DNA cleaved with the restriction endonuclease EcoRI.

a) The oligonucleotide 2 was phosphorylated and a ddA residue added at the 3′ end as described above in example (2). This modified oligonucleotide was then annealed to oligonucleotide 14.

b) Genomic DNA 1 was cleaved with the restriction endonuclease EcoR1 and dephosphorylated as described in Method I.

c) The annealed oligonucleotides 2 + 14 were then ligated to genomic DNA I cleaved with EcoRI.

d) Amplification was carried out on the ligation products using:

(i) Oligonucleotides 58 and 62. The expected product is about 220 bp.

(ii) Oligonucleotides 58 and 63 The expected product is 560 bp.

Example 4

Using oligonucleotide 3 and 14 with DNA cleaved with the restriction endonuclease BamHI.

a) The oligonucleotide 3 was phosphorylated and a ddA nucleotide added to the 3′ end as described above in Example 1.

b) This prepared oligo was then annealed to oligonucleotide 14.

c) Genomic DNA I was cleaved with the restriction endonuclease BamHI and dephosphorylated as described in Method I.

d) The annealed oligonucleotides 3 and 14 were then ligated to genomic DNA I cleaved with BamHI (see Method I for details).

e) Any free 3′ends in the ligation products were then blocked with a ddG residue using the procedure described in step 6 of Method 3.

f) Finally amplification was carried out on these products using oligonucleotides 58 and 63. The product expected is about 200 bp.

Example 5

Using oligonucleotide 3 and 14 with DNA cleaved with the restriction endonuclease BamHI.

a) Oligonucleotide 3 was prepared and annealed to oligonucleotide 14 as described above in example (4).

b) pUC8 containing a 440bp insert (see B3, Figure 10) plasmid DNA was digested to completion with the restriction endonuclease BamHI and dephosphorylated as detailed in Method I.

c) Oligonucleotides 3 and 14 were annealed to B3 plasmid DNA cleaved with BamHI.

d) A ddG residue was attached to 3′free ends as detailed in example 4. Amplification was carried out on the ligation products using oligonucleotides 58 and 65 Figure 11(iii) hereinbefore described shows the marker λ Hind III in lanes land 11, the marker OX174 cleaved with Hae III in lane 6 and the amplified fragment obtained according to Example 5 in lanes 2, 3, 4 and 5. As expected the product is about 440 bp in length.

Example 6

Using oligonucleotides 26 and 40 with DNA cleaved with the restriction endonuclease XbaI.

a) Oligonucleotide 26 was phosphorylated and the kinased species purified as described in Method V.

b) The kinased oligo 26 was annealed to oligonucleotide 40 under conditions described in Step 3 of Method I.

c) Human genomic DNAII was restricted with Xba I and dephosphorylated as detailed in Step 4 of Method I.

d) The annealed oligos 26 & 40 were then ligated to genomic DNA II cleaved with the restriction endonuclease XbaI (see step 5 of Method I for details).

e) Any free 3′ ends in the ligation products were blocked with a ddA residue using the procedure described in step 6 of Method III.

f) Amplification was carried out on the ligation products with:

(i) Oligonucleotides 58 and 61 The expected product is about 800 bp.
(ii) oligonucleotides 58 and 64 The expected product is about 760 bp.

Example 7

Using oligonucleotides 27 and 40 with DNA cleaved with the restriction endonuclease EcoRI.

a) Oligonucleotide 27 was phosphorylated, purified and annealed to oligo 40 as described above in example 5.

b) Genomic DNA I was cleaved with the restriction endonuclease EcoRI and dephosphorylated as detailed in step 4 of Method I.

c) The annealed oligos 27 and 40 were then ligated to genomic DNA I cleaved with EcoRI (see step 5 of Method 1 for details).

d) Any free 3′ ends in the ligation products were blocked with ddA residue as described in step 6 of Method III.

e) Amplification was carried out on the ligation products using:
(i) Oligonucleotides 58 and 62 The expected product is about 220 bp
(ii) Oligonucleotides 58 and 63 The expected product is 560 bp.

Figure 11(iii) as hereinbefore described shows the marker λ Hind III in lanes 1 and 11, the marker $\phi$X174 cleaved with Hae III in lane 6 and the amplified fragments obtained according to Example 7 in lanes 7, 8, 9 and 10.

Example 8

An EcoRI vectorette library was constructed according to Method VI and vectorette unit 6 (oligonucleotide 27,40) was used together with 7387 cell line DNA.

The amplification reaction was carried out on 10 ng of the constructed library in order to amplify the sequence depicted in Figure 12.

Amplification conditions

The following were mixed in a 0.5 ml Sarstedt tube.
10 ng of constructed EcoRI vectorette library
100 pmole 58
100 pmole 63
100μm (final concentration) dNTPs (see M1.7 as defined hereinafter)

The volume was adjusted to 100μm with sterile double distilled $H_2O$. 50μl of light mineral oil (Sigma) was gently layered over the reaction mix in order to prevent evaporation. The tube was then placed in Techne Dri-Block set at 96°C. The DNA was allowed to denature for 10 minutes at 96°C. The block was then allowed to cool to 90°C. 2 units of Taq polymerase (Cetus) were added to the reaction mix. The following temperatures and times were employed to amplify the sequence between oligonucleotides 58 and 63.
91° 2 minutes
62° 2 minutes
72° 2 minutes

40 cycles were performed. On the final cycle the temperature of the block was maintained at 72°C for 9.9 minutes then allowed to cool to room temperature over a period of 1 hour.
15 μl of the amplification reaction mixture was analysed by agarose gel electrophoresis.
The result is shown in Figure 13 which shows the amplification product obtained according to this Example in lane 1 and the marker $\phi$X174 cleaved with Hae III in lane 2. The amplification product is of the expected size (ca 600 bp).

Example 9

This example was effected according to method IX as follows:-

Step 1

Phosphorylation of the 'top' oligonucleotide

Oligonucleotide 49, 51, 52, 53, 55, 27, 57 and 2.

Buffers and other standard solutions

| 1.1 10x kinase buffer | |
|---|---|
| 0.5M | Tris.HCl (pH 7.6) |
| 0.1M | $MgCl_2$ |
| 50mM | Dithiothreitol |
| 1mM | Spermidine |
| 1mM | EDTA |
| Stored at -20°C | |

1.2 Adenosine 5′-triphosphate

Sodium salt bought from Pharmacia as a 10mM solution.
Diluted to 20pmole/µl in water
Stored at -20°C

Procedure

The following were mixed in a 1.5ml Sarstedt tube:

| | |
|---|---|
| Oligonucleotide | 10 pmoles |
| 10x kinase buffer (I.1) | 1µl |
| ATP (I.2) | 1µl (20 pmoles) |
| T4 polynucleotide kinase (Amersham) | 5 units |
| $H_2O$ | to 10µl |

The molar ratio of oligonucleotides to ATP was maintained at 1:2.

Phosphorylation of the oligonucleotides was carried out by incubation at 37°C for 60 minutes.

At the end of this period the T4 polynucleotide kinase was inactivated by incubation at 90°C for 10 minutes. The tubes were then spun in a microcentrifuge for 10 seconds to collect all the condensate.

The phosphorylated species are not gel purified in this method.

Step 2

Annealing of the phosphorylated 'top' oligonucleotide to the corresponding 'bottom' oligonucleotide

Solution required:

| 2.1 10x Annealing buffer | |
|---|---|
| 100mM<br>100mM | Tris HCl (pH 8.0)<br>$MgCl_2$ |

The following were mixed in a 1.5ml screwcap Starstedt tube:-
10 pmoles (in 10µl) of phosphorylated 'top' oligonucleotide from step 1
10 pmoles of the corresponding 'bottom' oligonucleotide
2µl of 10x annealing buffer (2.1)
$H_2O$ to 20µl

The tube was placed in a boiling water bath. After 5 minutes heating was discontinued and the water bath allowed to cool slowly to room temperature over a period of about 2 hours. Finally the contents of the tube were collected at the bottom by centrifugation in a microfuge for 10 seconds. The annealed oligonucleotides, at a concentration of 0.5pmole/µl, are then ready for use in ligations.

Vectorette units 1-8 were as defined in Table 1 hereinbefore.

Step 3

Preparation of DNA

Cell line 7387 DNA was used.

Buffers required:

| 3.1 10x EcoR1 buffer | |
|---|---|
| 100mM<br>100mM<br>500mM | Tris-HCl pH 7.5<br>$MgCl_2$<br>NaCl |

| 3.2 TE Buffer | |
|---|---|
| 10mM<br>1mM | Tris-HCl pH 7.5<br>EDTA |

| 3.3 Agarose sample buffer | |
|---|---|
| 15% (w/v)<br>0.05% (w/v)<br>0.05% (w/v) | Ficoll 400<br>Bromophenol Blue<br>Xylene Cyanol |
| The sample buffer was made up in 1xTBE buffer (3.4 below). | |

| 3.4 10x TBE buffer: per litre:- | |
|---|---|
| Tris | 109g |
| EDTA | 9.5g |
| Boric Acid | 55g |

10xTBE buffer was diluted in water to 1x or 0.5x strength as required.

(i) The following conditions were employed for cleaving the 7387 cell line DNA with the restriction endonuclease EcoR1:-

40µg of 7387 genomic DNA
40µl of 10X EcoR1 buffer (3.1)
80 units of the restriction endonuclease
EcoR1 (from Boehringer Mannheim)
$H_2O$ to 400µl

Digestion was carried out at 37°C for 2 hours. The reaction was terminated by heating the sample at 70°C for 10 minutes.

(ii) 5 µl of the sample was checked by agarose gel electrophoresis to ensure complete digestion:-

Agarose gel electrophoresis

The gels were made 0.7% (w/v) agarose (Pharmacia) in 1XTBE (3.4) buffer and submerged in the same strength TBE buffer for electrophoresis. To the sample (5 µl), a fifth-volume of agarose sample buffer (3.3) was added prior to loading the samples in the wells of the gel. Following electrophoresis the gel was stained for 10 minutes by submerging in deionised water containing 1µg/ml ethidium bromide. The DNA was visualised at 302nm using a transilluminator (Chromato-Vue C-63 transilluminator).

(iii)(a) The digested DNA was purified by phenol extraction:-

An equal volume of buffered redistilled phenol (BRL DNA grade phenol buffered with TE-3.2) was added to the restricted DNA and the mixture vortexed for 30 seconds. The white emulsion formed was separated into two phases by microcentrifugation for 5 minutes. The aqueous phase was transferred to a fresh tube. Any residual phenol was removed by first extracting the aqueous layer with an equal volume of phenol: chloroform: isoamylalcohol (25:24:1) - an equal volume-, and then with an equal volume of chloroform: isoamylalcohol (24:1).

(b) The DNA was finally purified by ethanol precipitation:

To the DNA solution, of 3M sodium acetate was added to give a final concentration of 300 mM sodium acetate. The DNA was precipitated by the addition of 2.5 volumes of 95% ethanol and cooling at -80°C for 30 minutes followed by centrifugation (microcentrifuge for 5 minutes, 12,000rpm). The pelleted DNA was washed with 80% (v/v) cold ethanol and dried under vacuum. The DNA was dissolved in the TE buffer (3.2) to give a concentration of 0.5µg/l.

Step 4

Ligating the annealed oligonucleotides to the cleaved DNA

Buffers and other standard solutions

| 4.1 10x ligation buffer | |
|---|---|
| 200mM | Tris-HCl pH 7.4 |
| 100mM | $MgCl_2$ |
| 100mM | DTT |

| 4.2 10x ATP | |
|---|---|
| 10mM | ATP |

| 4.3 | |
|---|---|
| 0.5M | NaCl |

The calculation of the pmoles of sticky ends in 1µg of cleaved DNA was as set out in 4.1(i) of method I.

Ligations

The following were mixed in a 0.5ml Sarstedt tube:

| | |
|---|---|
| Cleaved 7387 DNA | 1.48 pmoles (2µg) |
| Annealed oligonucleotides (from step 2) | 3 pmoles |
| 10x ligation buffer (4.1) | 2µl |
| 10x ATP (4.2) | 2µl |
| T4 DNA ligase (Boehringer Mannheim) | 2 units |
| $H_2O$ final volume to | 20µl |

The ligation mix was left at 25°C for 120 minutes. NaCl (4.3) was then added to the ligation mix to a final concentration of 50mM. Any genomic DNA fragments ligated to other genomic DNA fragments were recut by the addition of 2 units of EcoR1 (Boehringer Mannheim) and incubation of the sample at 37°C for 30 minutes. Vectorette units were then ligated to these recut genomic DNA fragments by adding:
ATP to 1mM concentration (4.2)
1.5 pmoles of annealed oligonucleotides
1 unit of T4 DNA ligase
(Boehringer Mannheim)
and incubation at 25°C for 1 hour. After recutting the DNA at 37°C for 30 minutes with EcoR1, the restriction endonuclease is not inactivated.

Therefore during the second and final ligation at 25°C for 60 minutes, any genomic DNA fragments ligating to other genomic DNA fragments are presumably being recleaved in the same reaction, such that at the end of this restriction/ligation reaction virtually 100% of the genomic DNA fragments have the vectorette units at both ends. Ligation of the vectorette unit to cut genomic DNA inactivates that particular restriction site.

The DNA (reaction mix) was then diluted to 10ng/µl. EcoR1 vectorette libraries were constructed using this method and vectorette units 1 to 8 together with 7387 cell line DNA.

A control EcoR1 library was also constructed where the vectorette units were omitted from the ligations described above

Step 5

Amplification applied to the vectorette libraries.

10ng of each of the libraries constructed (for vectorette units 1-8 and the DNA minus vectorettes control library) were subjected to amplification. The sequence shown in Figure 12 was amplified using Oligonucleotides 58 and 63 as amplimers and the amplification conditions used were as described in Example 8.

15µl of the amplification product was analysed by agarose gel electrophoresis.

The result is as shown in Figure 14. The expected size (ca 600bp) product has been produced in all reactions except where the substrate DNA is from the library constructed without vectorettes in the ligation mix. This is as expected since the sequence of oligonucleotide (one of the amplimers above) is within the vectorette units.

These results indicate that the design of the vectorette units has no significant affect on the amplification of the nucleotide sequence between the vectorette units and any desired genomic sequence (ie, oligonucleotide 63 above)

The results are shown in Figure 14 which shows a photograph of and ethidium bromide stained gel depicting the products obtained which are as set out below:-

1. Library constructed with 7387 DNA + vectorette unit 1
2. Library constructed with 7387 DNA + vectorette unit 2
3. Library constructed with 7387 DNA + vectorette unit 3
4. Library constructed with 7387 DNA + vectorette unit 4
5. Library constructed with 7387 DNA + vectorette unit 5
6. Library constructed with 7387 DNA + vectorette unit 6
7. Library constructed with 7387 DNA + vectorette unit 7
8. Library constructed with 7387 DNA minus vectorettes
9. ϕX174 HaeIII marker
10. ϕX174 HaeIII marker
11. Library constructed with 7387 DNA + vectorette unit 8 The expected size product is obtained in each case.

Example 10

This example was effected according to method X as hereinbefore described.

EcoR1 vectorette libraries were constructed using this method and vectorette units 1 to 7 as herein defined and 7387 cell line DNA.

A control EcoR1 library was also constructed where the vectorette units were omitted from the ligations described above.

Amplification applied to the vectorette libraries

10ng of each of the libraries constructed (for vectorette units 1-7, and the DNA minus vectorettes control library) were subjected to amplification. The sequence shown in Example 8 was amplified using oligonucleotides 58 and 63 as amplimers. The amplification conditions described in Example 8 were used.

15µl of the product was analysed by agarose gel electrophoresis.

The result is as shown in Figure 15. The expected size (ca 600bp) product has been produced in all reactions except where the substrate DNA is from the library constructed without vectorettes in the ligation mix. This is as expected since the sequence of oligonucleotide 58 (one of the amplimers above) is within the vectorette units.

The lanes of the photograph shown in Figure 15 are as follows:-

1. ϕX174 HaeIII marker
2. Amplification product from library 7387 DNA + vectorette unit 1
3. Amplification product from library 7387 DNA + vectorette unit 2
4. Amplification product from library 7387 DNA + vectorette unit 3
5. Amplification product from library 7387 DNA + vectorette unit 4

6. Amplification product from library 7387 DNA + vectorette unit 5
7. Amplification product from library 7387 DNA + vectorette unit 6
8. Amplification product from library 7387 DNA + vectorette unit 7
9. Amplification product from library 7387 DNA minus vectorettes.
10. $\phi$X174 Hae III marker.

The expected result is obtained from each library.

These results indicate that the design of the vectorette units has no significant affect on the amplification of the nucleotide sequence between the vectorette units and any desired genomic sequence (ie oligonucleotide 63 above).

Example 11

This example was effected according to method XI as hereinbefore described.

EcoR1 vectorette libraries were constructed using this method and vectorette units 1 to 7 and 7387 cell line DNA.

A control EcoR1 library was also constructed where the vectorette units were omitted from the ligations described above.

Amplification applied to the vectorette libraries

10ng of each of the libraries constructed (for vectorette units 1-7, and the DNA minus vectorettes control library) were subjected to polymerase chain reaction. The sequence shown in example 1 was amplified using oligonucleotides 58 and 63 as amplimers. The amplification conditions described in Example 8 were employed.

15$\mu$l of the product was analysed by agarose gel electrophoresis.

The result is as shown in Figure 16. The expected size (ca 600bp) product has been produced in all reactions except where the substrate DNA is from the library constructed without vectorettes in the ligation mix. This is as expected since the sequence of oligonucleotide 58 (one of the amplimers above) is within the vectorette units.

The lanes of the photograph shown in Figure 16 are as follows:-

1. $\phi$X174 Hae III marker
2. Amplification product from library 7387 DNA + vectorette unit 1
3. Amplification product from library 7387 DNA + vectorette unit 2
4. Amplification product from library 7387 DNA + vectorette unit 3
5. Amplification product from library 7387 DNA + vectorette unit 4
6. Amplification product from library 7387 DNA + vectorette unit 5
7. Amplification product from library 7387 DNA + vectorette unit 6
8. Amplification product from library 7387 DNA + vectorette unit 7
9. Amplification product from library 7387 DNA minus vectorettes
10. $\phi$X174 HaeIII marker.

These results indicate that the design of the vectorette units have no significant adverse affect on the amplification of the nucleotide sequence between the vectorette units and any desired genomic sequence (ie oligonucleotide 63 above).

Example 12

This example was effected according to method XII as hereinbefore described.

EcoR1 vectorette libraries were constructed using this method and vectorette units 1 to 7 and 7387 cell line DNA.

A control EcoR1 library was also constructed where the vectorette units were omitted from the ligations described above.

Amplification applied to the vectorette libraries

10ng of each of the libraries constructed (for vectorette units 1-7, and the DNA minus vectorettes control library) were subjected to amplification. The sequence shown in Example 1 was amplified using oligonucleotides 58 and 63 as amplimers. The amplification conditions described in Example 8 were employed.

15µl of the amplification product was analysed by agarose gel electrophoresis.

The result is as shown in Figure 17. The expected size (ca 600bp) product has been produced in all reactions except where the substrate DNA is from the control library constructed without vectorettes in the ligation mix. This is as expected since the sequence of oligonucleotide 58 (one of the amplimers above) is within the vectorette units.

The lanes of the photograph shown in Figure 17 are as follows:-

1. ϕX174 HaeIII marker.
2. Amplification product from library 7387 DNA + vectorette unit 1
3. Amplification product from library 7387 DNA + vectorette unit 2
4. Amplification product from library 7387 DNA + vectorette unit 3
5. Amplification product from library 7387 DNA + vectorette unit 4
6. Amplification product from library 7387 DNA + vectorette unit 5
7. Amplification product from library 7387 DNA + vectorette unit 6
8. Amplification product from library 7387 DNA + vectorette unit 7
9. Amplification product from library 7387 DNA minus vectorettes
10. ϕX174 HaeIII marker.

These results indicate that the design of the vectorette units have no significant affect on the amplification of the nucleotide sequence between the vectorette units and any desired genomic sequence (ie oligonucleotide 63 above).

Example 13

This example was effected according to method XIII as hereinbefore described.

EcoR1 vectorette libraries were constructed using this method and vectorette units 1 to 7 and 7387 cell line DNA.

A control EcoR1 library was also constructed where the vectorette units were omitted from the ligation reaction.

Amplification applied to the vectorette libraries

10ng of each of the libraries constructed (for vectorette units 1-7, and the DNA minus vectorettes control library) were subjected to the amplification. The sequence shown in example 1 was amplified using oligonucleotides 58 and 63 as amplimers. The PCR conditions described in Example 8 were employed.

15µl of the amplification product was analysed by agarose gel electrophoresis.

The result is as shown in Figure 18. The expected size ( 600bp) product has been produced in all reactions except where the substrate DNA is from the library constructed without vectorettes in the ligation mix. This is as expected since the sequence of oligonucleotides 58 (one of the amplimers above) is within the vectorette units.

The lanes of the photograph shown in Figure 18 are as follows:-

1. ϕX174 HaeIII marker.
2. Amplification product from library 7387 DNA + vectorette unit 1
3. Amplification product from library 7387 DNA + vectorette unit 2
4. Amplification product from library 7387 DNA + vectorette unit 3
5. Amplification product from library 7387 DNA + vectorette unit 4
6. Amplification product from library 7387 DNA + vectorette unit 5
7. Amplification product from library 7387 DNA + vectorette unit 6
8. Amplification product from library 7387 DNA + vectorette unit 7
9. Amplification product from library 7387 DNA minus vectorettes.
10. ϕX174 HaeIII marker.

These results indicate that the design of the vectorette units have no significant affect on the amplification of the nucleotide sequence between the vectorette units and any desired genomic sequence (see oligonucleotide 63 above).

Example 14

Five different EcoR1 vectorette libraries were constructed using 7387 cell line DNA and vectorette unit 8 in the following way.

(1)

(a) One library was constructed using Method IX

(b) One library was constructed using Method X

(c) One library was constructed using Method XI

(d) One library was constructed using Method XII

(e) One library was constructed using Method XIII

(2) Prior to amplification, the libraries constructed in (1) above were subjected to a 3′-end blocking reaction:-

Buffers and other standard solutions

| 7.2.1 T7 DNA polymerase buffer 5x | |
|---|---|
| 200mM | Tris-HCl pH 7.5 |
| 100mM | $MgCl_2$ |
| 250mM | NaCl |

| 7.2.2 dNTPs-dATP | |
|---|---|
| 1mM | dTTP |
| 1mM | dGTP |
| 1mM | dCTP |
| made up in sterilised deionised water. | |

7.2.3 ddATP

1mM ddATP made up in sterilised deionised water.

Addition of a ddA residue to 3′-ends of products from (1) above.

The following were mixed in a 0.5ml Sarstedt tube:

0.5µl of DNA from step (1) above

4µl of 5x T7 DNA polymerase buffer (7.2.1)

2µl dNTPs-dATP solution (7.2.2)

2µl ddATP solution (7.2.3)

1 unit of T7 DNA polymerase (Sequenase)

$H_2O$ to 20µl

The tube was maintained at 37°C for 30 minutes. The enzyme was then denatured by incubation at 70°C for 10 minutes.

The concentration of DNA in all the samples was then diluted in water to 10ng/µl.

Amplification applied to the vectorette libraries

10ng of each of the five libraries constructed were subjected to polymerase chain reaction. The sequence shown in Example 8 was amplified using oliognucleotides 58 and 63 as amplimers. The amplification conditions in Example 8 were employed.

15µl of the amplification product was analysed by agarose gel electrophoresis.

The result is as shown in Figure 19.

The lanes of the autoradiograph shown in Figure 19 are as follows:-

1. φX174 HaeIII marker.
2. Amplification product from 7387 DNA + vectorette unit 8 method IX + 3′end blocking
3. Amplification product from 7387 DNA + vectorette unit 8 method X + 3′end blocking
4. Amplification product from 7387 DNA + vectorette unit 8 method XI + 3′end blocking
5. Amplification product from 7387 DNA + vectorette unit 8 method XII + 3′end blocking
6. Amplification product from 7387 DNA + vectorette unit 8 method XIII + 3′end blocking All products are of the expected size.

<u>Example 15</u>

5 different EcoR1 vectorette libraries were constructed using 7387 cell line DNA and vectorette unit 8.

(a) one library was constructed using method IX
(b) one library was constructed using method X
(c) one library was constructed using method XI
(d) one library was constructed using method XII
(e) one library was constructed using method XIII except that oligonucleotide 2 was nonphosphorylated.

Libraries (b) to (e) were prepared as described for methods IX to XII Libraries (b), (c), (d,) (e) and an aliquot of library (a) were treated with ddATP and T7 DNA polymerase (Sequenase) to block free 3′ ends. Conditions were as described in Example 14.

The amplification was carried out on all of these libraries. The sequence shown in Figure 12 was amplified using oligonucleotides 63 and 58. The conditions described in Example 8 were employed.

15µl of the amplification product was analysed by agarose gel electrophoresis (1.4% w/v gel).

The result obtained was as shown in Figure 20.

The lanes of the photograph shown in Figure 20 are as follows:-

1. Amplification product from 7387 DNA + vectorette unit 8 method IX (oligonucleotide 2 non-phosphorylated)
2. Amplification product from 7387 DNA + vectorette unit 8 method IX + 3′end blocking (oligonucleotide 2 non-phosphorylated)
3. Amplification product from 7387 DNA + vectorette unit 8 method X + 3′end blocking (oligonucleotide 2 non-phosphorylated)
4. Amplification product from 7387 DNA + vectorette unit 8 method XI + 3′end blocking (oligonucleotide 2 non-phosphorylated)
5. Amplification product from 7387 DNA + vectorette unit 8 method XII + 3′end blocking (oligonucleotide 2 non-phosphorylated)
6. Amplification product from 7387 DNA + vectorette unit 8 method XIII + 3′end blocking (oligonucleotide 2 non-phosphorylated)
7. φX174 HaeIII marker. All products are of the expected size.

<u>Example 16</u>

This example was conducted to show that the <u>ca</u> 600bp product shown in Figures 13-20 is a product of oligonucleotides 58 and 63 (see example 8) and <u>not</u> a product of one of the amplimers.

10ng of each of the following vectorette libraries was used for each amplification:

A library prepared using 7387 DNA and vectorette units <u>1-8</u> in method VII.

A library prepared using 7387 DNA and vectorette <u>unit 8</u> using method VII + 3′-end blocking (see example 14 for details).

A library prepared using 7387 DNA and vectorette unit 8 (1b oligonucleotide <u>non</u> phosphorylated) using method VII + 3′-end blocking (see example 15 for details).

A library prepared using 7387 DNA minus vectorette using method VII.

To show that products produced in example 8 from libraries constructed with vectorette units 1-8 were all identical and of the correct sequence amplification products shown in Figure 16 (products 2-8) and the product shown in lane 11 of Figure 14 were sequenced using oligonucleotides 5 and 66.

Figure 21 shows a photocopy of the autoradiograph produced from the sequencing of products 2-8 from Figure 16 with oligonucleotide 66.

The autoradiograph also shows the sequencing of parts of intron 8 and exon 9 of the phenylalanine hydroxylase gene (phenylketonuria -PKU) as a control. The amplification products 2-8 appear from left to right (as shown in Figure 16) and are all identical. The lane order is CGTA.

Figure 22 shows the nucleotide sequence data generated from the products described in Example 16. Bases 1 to 16 are at the 3′ end of the published PKU intron 8 sequence. Bases 17 to 452 constitute new PKU intron 8 sequence data determined according to the method of the present invention.

## Example 17

The ca 220bp product from example 7(i), track(c) lanes 7, 8, 9, 10, 11 was sequenced using oligonucleotides 62 and 67.

Thus the 220bp product from Example 7(i) was

1) amplified in 3 step cycles a) 60°C 2 minutes, b) 72°C 3 minutes and c) 91°C 2 minutes, 40 cycles being performed (0.5µg DNA used) /100µl loaded/track (see Figure 24a)

2) the product from (1) above was then gel purified and reamplified in 2 step cycles a) 60°C 4 minutes and b) 91°C 2 minutes, 40 cycles being performed (see Figure 24b).

The positions of the oligonucleotides 58, 62 and 67 are shown in Figure 23. In Figure 25, the sequence set out in (a) is the sequence read with oligonucleotide 62, the sequence set out in (b) being the sequence read with oligonucleotide 67. In (a) and (b) the underlined sequence is the complementary /reverse sequence read with oligonucleotide 62 and 67. The overall 5′→3′ sequence is depicted in Figure 25(c) in which:-

Positions 2 - 31 reads the 5′-3′ sequence of oligonucleotide 62.

Positions 75 - 104 reads the reverse complementary sequence of oligonucleotide 67.

Positions 95 - 104 (marked by a continuous line above the sequence) is the first 10 bases of the published intron sequence (see Biochemistry 1985 25 p556-561 Kwok et al).

Positions 2 - 104 match the published sequence for PKU exon I and parts of the intron sequences.

Position 105 onwards is the new sequence data for PKU intron 1.

## Example 18

Exon V of the alpha antitrypsin gene was subjected to the method of the present invention (see Figure 26) using the following oligonucleotides:-

58 (universal vectorette primer), 60 (universal vectorette nested oligonucleotide), 68 (exon V 5′), 69 (exon V 5′ nested) and 70 (exon V 3′).

The vectorette library was made with EcoRI cut genomic DNA with annealed vectorette oligonucleotides (87/40 as herein defined) and ligated.

The amplification was effected as follows:-

100 ng of the vectorette library was used in a first round of amplification as follows:-

| | |
|---|---|
| 100ng Vectorette Library DNA<br>Oligonucleotides 58 + 68 (100 pmoles)<br><br>100µM dNTP's<br>2U Taq polymerase<br>Make up volume to 100µl with double distilled water | 10µl Buffer: 500mM KCl<br>100mM $MgCl_2$<br>14mM $MgCl_2$<br>0.1% gelatin] |

Conditions of reaction:

Initial denaturation step→ 93°C for 10 minutes

39 cycles of
93°C 2 minutes
65°C 2 minutes
72°C 8 minutes
Final cycle; 72°C for 10 minutes

The result is shown in Figure 27(a) in lane 4. Lane 3 represents a control amplification reaction using oligonucleotides 68 and 70 to produce a ca 200bp product.

The primary round of amplification gave a smear of the approximate size expected. The primary reaction mix was then diluted as follows:-
1µl of the product of lane 4 in Figure 27(a) was made up to 1ml with $ddH_2O$
(ie. $10^3$ dilution)
1µl of this was used in subsequent PCR's (total = 100µl)
(ie. Total dilution = $10^5$)
and reamplified using a range of primer pairs as follows:-

| 2nd PCR (with nested oligonucleotides) | | |
|---|---|---|
| 1µl the product of lane 4 in Figure 27(a) ($10^3$)DNA | | |
| 100pmoles oligonucleotides 60 + 69 | | |
| 10µl 10X Buffer.<br>100µm dNTP's<br>2 units Taq Polymerase | 10X buffers: | 500mM KCl<br>100mM Tris pH8.3<br>10mM $MgCl_2$<br>0.1% gelatin |
| Make up volume to 100µl with double distilled water | | |

Conditions of reaction:
Initial denaturation step →95°C for 10 minutes

The secondary amplification product is shown as a 3.0 kb product in lanes 1,2 and 3 of Figure 27(b).

Figure 27(b) is a photograph of an agarose gel showing the product of secondary amplification between oligonucleotides 60 and 69.

Lane 1 the 3.0 kb product of secondary amplification between oligonucleotides 60 and 69
2 the 3.0 kb product of secondary amplification betwen oligonucleotides 60 and 69
3 the 3.0 kb product of secondary amplification between oligonucleotides 60 and 69
4 the marker ϕx174 cleaved with Hae III and the marker λ cleaved with Hind III.

The gel was blotted onto a nylon membrane and probed with an α-1, anti trypsin probe. A single band of the correct size (3.0 kb) was observed and is shown in Figure 27(c).

Figure 27(c) is an autoradiograph of a nylon filter (blotted from the gel shown in Figure 27(b)) which had been probed with an α-1, antitrypsin probe. The position of DNA markers is shown.

Lane 1 the 3.0 kb product of secondary amplification between oligonucleotides 60 and 69
2 the 3.0 kb product of secondary amplification between oligonucleotides 60 and 69
3 the 3.0 kb product of secondary amplification between oligonucleotides 60 and 69

Example 19

D5 is an anonymous genomic sequence which has been cloned as a 474 bp Xbal -EcoR1 fragment. The sequence of D5 has been determined by standard methods (Newton et al, Nucleic Acids Research 16, 8233-8243 1988) and is shown in Figure 29.

Two oligonucleotide primers, 71, 72 were synthesised.
71 5′ AAGTTTGAGCATAGGAAAAGTTCTGTGCCC
72 5′ AGTTCTGTGCCCAAAATTGCATCCAAG
The position of oligonucleotides 71, 72 is indicated on Figure 29.

Genomic DNA was isolated from the lymphoblastoid cell line, GM 7387, (NIGMS Human Genetic

Mutant Cell Repository) and aliquots (20µg) were digested with the following restriction enzymes; Dra I, Bcl I, Hind III and Hae III. Vectorette libraries were then prepared according to Method X.

Primary amplifications were performed on aliquots of each library as described hereinunder.

The following were mixed in a 0.5 ml Sarstedt tube.

10 ng of constructed vectorette library
100 pmole oligonucleotide 58
100 pmole oligonucleotide 71

100 µm dNTPs
10 µl 10X Buffer

10X Buffer is:
500mM KCl
100mM Tris-HCl pH8.3
10mM MgCl
0.1% gelatin

The volume was adjusted to 100 µl with sterile, double distilled water. 50 µl of light mineral oil (Sigma) was gently pipetted over the reaction mix in order to prevent evaporation.

The tube was then heated to 96°C (in a Techne Programmable Dri-Block PHC-1 which had been equilibrated at 96°C). The DNA was allowed to denature for 10 minutes at 96°C. The block was allowed to cool to 90°C. Two units of Taq polymerase (Cetus) were added to the reaction mix. The following temperatures and times were employed to amplify the sequence between oligonucleotides 58 and 71.

92°C 2 minutes
65°C 2 minutes
72°C 5 minutes

40 cycles were performed. During the final cycle, the reaction mix was held at 72°C for 10 minutes.

Aliquots (15 µl) were withdrawn from the reaction mixtures. Dye loading mix (5 µl) was added and the samples were analysed on 1.4% agarose gels.

Dye Loading Mix
155 (w/v) Ficoll 400
0.05% (w/v) Bromophenol Blue
0.05% (w/v) Xylene Cyanol
Dissolved in 1X TBE A secondary amplification was performed on aliquots of the primary reaction mixtures. An aliquot of the primary reaction mixture (2 µl) was diluted to 400 µl with water. An aliquot of the diluted material (2 µl) was placed in a 0.5 ml Sarstedt tube.

The following were also added to the tube.

100 pmole oligonucleotide 60
100 pmole oligonucleotide 72
100 µM dNTPs
10 µl 10X BUFFER
10X Buffer is:
500 mM KCl
100 mM Tris HCl pH 8.3
10 mM MgCl
0.1% gelatin

The volume was made up to 100 µl with sterile, double distilled water. 50 µl of light mineral oil (Sigma) was gently pipetted over the reaction mix in order to prevent evaporation.

The tube was then heated to 90°C (in the Techne Programmable Dri-Block PHC-1 which had been equilibrated 96°C). The DNA was allowed to denature for 10 minutes at 96°C. The block was allowed to cool to 90°C. Two units of Taq polymerase (Cetus) were added to the reaction mix. The following temperatures and times were employed to amplify the sequence between oligonucleotides 60 and 72.

92°C 2 minutes
65°C 2 minutes

72°C 5 minutes

37 cycles were performed. During the final cycle, the reaction mixture was held at 72°C for 10 minutes.

Aliquots (15 µl) were withdrawn from the reaction mixtures. Dye loading mix (5 µl) was added an the samples were analysed on a 1.4% agarose gel.

Dye Loading Mix
15% (w/v) Ficoll 400
0.05% Bromophenol Blue
0.05% Xylene Cyanol
Dissolved in 1x TBE

The libraries (Hae III, Hind III, Bc1I, DraI) gave distinct products in the size range 0.4-1.5 kb (Figure 30). From the observed size of the products, a provisional restriction map could be prepared (Figure 31). The products were further analysed by restriction digests. Authentic products should be cleaved to give the predicted size fragments. Thus, the 0.82 kb Bc1I product could be cleaved by Hind III to give a product of 0.78 kb and could also be cleaved by Hae III to give a fragment of 0.75 kb. The analysis of the 0.82 kb Bc1I product by Hind III and Hae III is shown in Figure 30. The predicted products were observed and the Bc1I product was then eluted for sequence analysis.

Thus in Figure 30 the product resulting from amplification of a Hae III vectorette library is shown in Lane 2, the product resulting from amplification of a Hind III vectorette library is shown in Lane 3, the product resulting from amplification of a Bc1I vectorette library is shown in Lane 4 and the product resulting from amplification of a Dra I vectorette library is shown in Lane 5. Lane 7 shows the fragments obtained after digestion of the Bc1I product with the restriction enzyme Hae III. Lane 8 shows the fragments obtained after digestion of the Bc1I product with the restriction enzyme, Hind III. Lane 9 shows the Bc1I product, Lanes 1 and 6 contain 1 kb DNA markers (BRL).

Restriction Digest Analysis of Vectorette Products.

| | |
|---|---|
| 16 µl | Amplification Reaction Mixture |
| 2 µl | 10 X Restriction Buffer |
| 2 µl | Restriction Enzyme |

The sample was incubated at 37°C, for 1 hour. 5 µl of Dye Loading Mix was added to the sample and the products were analysed on 1.4 % agarose gel.

SEQUENCING

The Bc1I product was eluted from the agarose gel and sequenced using oligonucleotide 59 and standard methodology (Newton et al Nucleic Acids Research 16, 8233-8243 1988). The sequence is illustrated in Figure 31. A further primer (73) was designed from this sequence and used in subsequent walks.

73 5′ GGCCTTTGANNAAGAGAAGAGTCAAGGATG

Examination of the sequence confirms the presence of the Hae III and Hind III sites predicted by walks according to the method of the present invention.

## Example 20

### Examination of the Chlamydia genome using the method of the present invention.

Chlamydia trachomatis serotype L2 was grown in cycloheximide treated McCoy cells. Elementary bodies were purified on glycerol-tartrate gradients yielding $10^9$ elementary bodies. DNA was extracted by treatment with proteinase K/SDS, phenol/chloroform and recovered by ethanol precipitation. 90 mg of DNA was recovered of which 900ng was estimated to be Chlamydial DNA and the remainder was mouse DNA derived from the McCoy cells.

DNA (5 µg equivalent to 50 ng Chlamydial DNA) was digested with either EcoRI or BamHI. Vectorette libraries were prepared according to Method X using the appropriate oligonucleotides (27, 40, EcoR1, 31,

40, BamHI). The libraries were made up to a final volume of 100 µl.

Two oligonucleotide primers were synthesised based on the consensus sequence of MOMP (Major Outer Membrane Protein) of Chlamydia L2. (Stephens et al J Bacteriology 169, 3879-3885 1987).

74 5′ CTGCTCACGTAAATGCACAATTCCG

75 5′ AACCGAGAGCAAAAGCCATATTGGC

Amplification

The following were mixed in a 0.5 ml Sarstedt tube.

| | | |
|---|---|---|
| 10 µl | EcoRI vectorette library | |
| 20 µl | 5X Buffer<br>5X Buffer = | <br>335 mM Tris HCl pH 8.5<br>83 mM $NH_4SO_4$<br>10 mM MgCl<br>50 mM 2-mercaptoethanol<br>0.8 mg/ml Bovine Serum Albumin |
| 100 pmole<br>100 pmole<br>200 µM | Oligonucleotide 58<br>Oligonucleotide 74<br>dNTPs | |

The volume was adjusted to 100 µl with sterile, double distilled water, 50 µl of light mineral oil (Sigma) was gently pipetted over the reaction mix in order to prevent evaporation.

The tube was then heated to 96°C (in the Techne Programmable Dri-Block PHC-1 which had been equilibrated to 96°C). The DNA was allowed to denature for 10 minutes at 96°C. The block was then allowed to cool to 90°C. Two units of Taq polymerase (Cetus) were added to the reaction mix. The following temperatures and times were employed to amplify the sequence between oligonucleotides 60 and 72.

92°C 0.7 minutes

65°C 0.7 minutes

72°C 1 minute

40 cycles were performed. 10% of the reaction mix was removed for analysis on agarose gel. The results are shown in Figure 32 in which.

Lane 1 shows the marker ϕX174 cleaved with Hae III.

Lane 2 shows the product of amplification between oligonucleotides 58 and 74 using 10 µl of an EcoRI library.

Lane 3 shows the product of amplification between oligonucleotides 58 and 74 using 1 µl of an EcoRI library.

The product from the amplification of an EcoRI library with oligonucleotides 58 and 74 was purified. Asymmetric amplification (Proc. Natl. Acad. Sci. USA Vol 85 pages 7652-7656, October 1988 U.B. Gyllenstein and H. Ehrlich) of the product was performed with 50 pmole oligonucleotide 74 and oligonucleotide 58 for 35 cycles as described above. The amplified product was purified on a G50 spun column (as described in Molecular Cloning - A laboratory manual; Maniatis T, Fritsch E.F., Sambrook J. Cold Spring Harbor Laboratory 1982) and sequenced with oligonucleotide 59 as described in example 17. The sequence is shown in Figure 3A. Digestion of an aliquot of the PCR product with either Hinf I or Cla I confirms the presence of these restriction sites (Figure 34).

In Figure 34 the lanes of the photograph are as follows:-

Lane 1 ϕx174 Hae III DNA marker

2 EcoRI product digested with Cla I

3 EcoRI product

4 EcoRI product digested with HinfI

5 ϕX174 Hae III DNA marker

Example 21

Linear amplification applied to the Chlamydia genome

The following were placed in a 0.5 ml Sarstedt tube

| | |
|---|---|
| 10 µl | BamHI vectorette library |
| 20 µl | 5X Buffer (defined in Example 20) |
| 100 pmole | Oligonucleotide 75. |
| 200 µm | dNTPs |

The volume was adjusted to 100 µl with sterile, double distilled water, 50 µl of light mineral oil (Sigma) was gently pipetted over the reaction mix in order to prevent evaporation.

The tube was then heated to 96°C (in the Techne Programmable Dri-Block PHC-1 which had been equilibrated 96°C). The DNA was allowed to denature for 10 minutes at 96°C. The block was then allowed to cool to 90°C. Two units of Taq polymerase (Cetus) were added to the reaction mix. The following temperatures and times were employed to amplify the sequence between oligonucleotides 75 and the BamHI site.

93°C 0.7 minutes
65°C 0.7 minutes
72°C 1.5 minutes

40 cycles were performed. 100 pmole oligonucleotide 58 and two units of Taq polymerase were then added to the reaction mix and amplification was continued for a further 20 cycles as described above. An aliquot reaction mix was analysed by agarose gel electrophoresis (Figure 35).

An additional reaction was performed in which a BamHI library was amplified using oligonucleotide 75 and 58 under the reaction conditions described above. An aliquot of the reaction mix was analysed by agarose gel electrophorsis (Figure 35).

In Figure 35:-

Lane 1 shows the marker ϕX174 cleaved with Hae III.

Lane 2 shows the product of linear amplification of a Bam HI vectorette library using oligonucleotide 75.

Lane 3 shows the product resulting from 20 cycles of secondary amplification with oligonucleotides 58 and 75.

Lane 4 shows the product resulting from 35 cycles of secondary amplification with oligonucleotides 58 and 75.

Lane 5 shows the product resulting from 40 cycles of primary amplification with oligonucleotides 58 and 75.

Example 22

A human genomic library was constructed using the cosmid vector p Cos EMBL2 according to published procedures (PFR Little (1987) in DNA cloning - A practical approach Vol III p 19-42 Edited by DM Glover). The library was screened by hybridisation to the probe KM19 (X Estivill et al (1987) Nature 326, 840-845). Several positive clones were identified and one clone 4.17 was selected which contained the KM19 sequence in a 40 kb insert.

Cosmid 4.17 was digested with Hind III and a vectorette library was prepared according to Method X.

Two oligonucleotide primers 76, 77 were synthesised

76 5′ GGAAGGCCTTCAAAATTAACAGTGTAGCC
77 5′ GCTGCATCATATAAGTTGCC

The positions of these primers is shown in Figure 36(a).

PCR amplification was performed on aliquots of the library as described hereinunder.

The following were mixed in a 0.5 ml Sarstedt tube.

0.1 pg Cosmid vectorette library

| | | |
|---|---|---|
| 100 pmoles | Oligonucleotide 58 | |
| 100 pmoles | Oligonucleotide 76 | |
| 100 µM | dNTPs | |
| 10 µl | 10x buffer | 10x buffer is 500 mM KCl |
| | | 100 mM Tris HCl pH 8.3 |
| | | 10 mM $MgCl_2$ |
| | | 0.1 % gelatin |

A second reaction was set up containing the following in a 0.5 ml Sarstedt tube.

| | |
|---|---|
| 0.1 pg | Cosmid vectorette library |
| 100 pmoles | Oligonucleotide 58 |
| 100 pmoles | Oligonucleotide 77 |
| 100 µM | dNTPs |
| 10 µl | 10x buffer |

The volume of the reaction mixes was adjusted to 100 µl with sterile double distilled water. 50 µl of light mineral oil (Sigma) was gently pipetted over the reaction mix in order to prevent evaporation. the tubes were then placed in a Thermal cycler (Techne Programmable Dri-Block PHC-1) which had been equilibrated at 96°C. The block was allowed to cool to 90°C. Two units of Taq polymerase (Cetus) were added to the reaction mix. The following temperatures and times were employed to amplify the sequences between oligonucleotides 58 and 76 and between 58 and 77.

92°C 2 minutes
65°C 2 minutes
72°C 5 minutes

40 cycles were performed. During the final cycle, the reaction mix was held at 72°C for 10 minutes.

Aliquots (15 µl) were withdrawn from the reaction mixtures. Dye loading mix (5 µl) was added and the samples were analysed on 1.4% agarose gels.

Dye loading mix
15% (w/v) Ficoll 400
0.05% (w/v) Bromophenol Blue
0.05% (w/v) Xylene Cyanol
Dissolved in 1 x TBE

The results are shown in Figure 36(b).

Lane 1 φX174 Hae III DNA marker
2 230 bp product (oligonucleotide 58, oligonucleotide 78)
3 1200 bp product ( oligonucleotide 58, oligonucleotide 77)
4 φX174 Hae III DNA markers

Example 23

The following oligonucleotides were synthesised.

78 5′ CTCTCCCTTCTCCGGTGATGCCGGCCACGATGCGTCCGGCGGTCCTCTCCTTC

A modified type E oligonucleotide containing a sequence representing nucleotide residues 385-414 of the tetracycline resistance gene of plasmid pBR322 (T Maniatis, EF Fritsch and J Sambrook (1982) Molecular Cloning - A laboratory manual p 479-503. To be used in conjunction with type D oligonucleotides.

79 5′ CCGGTGATGCCGGCCACGATGCGTCCGGCG

A modified universal vectorette primer representing nucleotide residues 385-414 of the tetracycline resistance gene of plasmid pBR322 (Maniatis et al Molecular Cloning - A laboratory manual p 479-503). To be used with oligonucleotide 78 and oligonucleotides 80/81, 82/81 and 83/81 described hereinafter.

80 5′ AATTGGCGGCCGCCATCCTAATTCTGTCGAAGGTAAGGAACGGAGGAGAGAACT

A modified type D oligonucleotide containing an EcoR1 cohesive end, a Not 1 recognition site and a Fok1 recognition site. To be used with oligonucleotide 81.

81 5′ AGTTCTCCCGGTGATGCCGGCCACGATGCGTCCGGCGGATGGCGGCCGCC

Amodified type E oligonucleotide

82. 5′ AGCTGGCGGCCGCCATCCTAATTCTGTCGAAGGTAAGGAACGGAGGAGAGAACT

A modified type D oligonucleotide containing a Hind III cohesive end, a Not 1 recognition site and a Fok 1 recognition site. To be used with oligonucleotide 81.

83. 5′ AGCTGGCGGCCGCCATCC-$NH_2$

A modified type A oligonucleotide containing a Hind III cohesive end with an aminohexyl modification blocking the 3′ terminus. To be used with oligonucleotide 81.

84. 5′ GGCTCAACAGTCAGTTTGAACTAGC

A primer adjacent to an EcoR1 site in the KM19 region.

Restriction maps appropriate to each of the following amplifications are shown in Figure 37 in which

(a) shows a restriction map of a segment of genomic DNA ligated to an EcoRI vectorette. The positions of oligonucleotides 27, 76, 78 and 79 are indicated.

(b) shows a restriction map of a segment of genomic DNA ligated to an EcoRI vectorette. The positions of oligonucleotides 78, 79 and 84 are indicated.

(c) shows a segment of the Phenylalanine Hydroxylase gene exon 9 ligated to an EcoRI vectorette. The positions of oligonucleotides 63 and 79 are indicated.

(d) shows a restriction map of the segment of genomic DNA (described in 37(a) above) ligated to a modified EcoRI vectorette. The positions of oligonucleotides 76, 79, 80 and 81 are indicated.

(e) shows a restriction map of a segment of genomic DNA (described in 37(b) above) ligated to a modified EcoRI vectorette. The positions of oligonucleotides 79, 80, 81 and 84 are indicated.

(f) shows a segment of the Phenylalanine Hydroxylase gene exon 9 ligated to a modified EcoRI vectorette. The positions of oligonucleotides 63, 79, 80 and 81 are indicated.

(g) shows a retriction map of a segment of genomic DNA ligated to a modified Hind III vectorette library. The positions of oligonucleotides 76, 78 and 79 are indicated.

(h) shows a restriction map of a segment of genomic DNA ligated to a modified Hind III vectorette library. The positions of oligonucleotides 78, 79, 81 and 82 are indicated.

(i) shows a restriction map of a segment of genomic DNA ligated to a modified Hind III vectorette library. The positions of oligonucleotides 76, 79, 81 and 83 are indicated.

(a) An EcoR1 vectorette library was prepared from EcoR1 digested genomic DNA (cell line 7387) and oligonucleotides 27 and 79 according to Method X. An aliquot of the library (1 ng) was amplified with oligonucleotides 76 and 79. Amplification was performed as described in example 8. An aliquot of the reaction mix (15 µl) was analysed on a 1.4% agarose gel. The results are shown in Figure 38.

Lane 1 ϕx174 Hae III DNA markers

2 870 bp product resulting from amplification between oligonucleotides 76 and 79.

(b) An aliquot (1ng) of the EcoR1 vectorette library described in the previous example was amplified with oligonucleotides 79 and 84. Amplification was performed as described in Example 8. An aliquot of the reaction mix (15 µl) was analysed on a 1.4% agarose gel. The result is shown in Figure 38.

Lane 3 520 bp product resulting from amplification between oligonucleotides 79 and 84.

(c) An aliquot (1 ng) of the EcoR1 vectorette library described in the previous examples was amplified with oligonucleotides 63 and 79. Amplification was performed as described in Example 8. An aliquot of the reaction mix (15 µl) was analysed on a 1.4% agarose gel. The result is shown in Figure 38.

Lane 4 600 bp product resulting from amplification between oligonucleotides 63 and 79.

(d) An EcoR1 vectorette library was prepared from EcoR1 digested genomic DNA (cell line 7387) and oligonucleotides 80 and 81 according to Method X. An aliquot of the library (1 ng) was amplified with oligonucleotides 76 and 79. Amplification was performed as described in Example 8. An aliquot of the reaction mix (15 µl) was analysed on 1.4% agarose gel. The results are shown in Figure 38.

Lane 5 870 bp product resulting from amplification between oligonucleotides 76 and 79.

(e) An aliquot of the library described in (4) above was amplified with oligonucleotides 79, 84. Amplification was performed as described in Example 8. An aliquot of the reaction mix was analysed on a 1.4% agarose gel. The result is shown in Figure 38.

Lane 6 520 bp product resulting from amplification between oligonucleotides 79 and 84.

(f) An aliquot of the library described in (4) above was amplified with oligonucleotide 63 and 79. Amplification was performed as described in Example 8. An aliquot of the reaction mix was analysed on a

1.4% agarose gel. The result is shown in Figure 38.

Lane 7 600 bp product resulting from amplification between oligonucleotides 63 and 79.

(g) A Hind III vectorette library was prepared from Hind III digested genomic DNA (cell line 7387) and oligonucleotides 30 and 81 according to Method X. An aliquot of the library (1 ng) was amplified with oligonucleotides 76 and 79. Amplification was performed as described in Example 8. An aliquot of the reaction mix (15 µl) was analysed on a 1.4% agarose gel. The result are shown in Figure 38.

Lane 8 230 bp product resulting from amplification between oligonucleotides 76 and 79.

(h) A Hind III vectorette library was prepared from Hind III digested genomic DNA (cell line 7387) and oligonucleotides 82 and 81 according to Method X. An aliquot of the library (1 ng) was amplified with oligonucletides 76 and 79. Amplification was performed as described in Example 8. An aliquot of the reaction mix (15 µl) was analysed on a 1.4% agarose gel. The results are shown in Figure 38.

Lane 9 230 bp product resulting from amplification between oligonucleotides 76 and 79.

(i) A Hind III vectorette library was prepared from Hind III digested genomic DNA (cell line 7387) and oligonucleotides 83 and 81 according to Method X. An aliquot of the library (1 ng) was amplified with oligonucleotides 76 and 79. Amplification was performed as described in Example 8. An aliquot of the reaction mix (15 µl) was analysed on a 1.4% agarose gel. The results are shown in Figure 38.

Lane 9 230 bp product resulting from amplification between oligonucleotides 76 and 79.

Lane 10 0x174 Hae III DNA markers.

Example 24

The following oligonucleotides were synthesised:

85 5′ ATTCTGTCCAGGAAACTTTGTGTTTTGTCA
85 5′ AGCCGAAGACAAAGGGCATAATCTC
87 5′ TCACTACCAGCTTTCCCCCACTTCCCTGGC
88 5′ CCCTAAATTAGTCTCAGCTCCAGGTAAGGT

DNA was extracted from cell line 7382 (previously demonstrated to possess the -+ haplotype for the KM19 PstI polymorphism) and digested with PstI. After extraction with phenol/chloroform, DNA was recovered by ethanol precipitation (see Figure 39(a)).

Circularisation

The following were added to a 0.5 ml Sarstedt tube:

| | |
|---|---|
| 100 ng | PstI digested 7382 DNA |
| 10 µl | 10X ligation buffer |
| 10 µl | 10 mM ATP |
| 0.5 µl | $T_4$ DNA ligase (Boehringer Mannheim 8 units/µl). |

The volume was adjusted to 100 µl with sterile, double distilled water and incubated for 2 hours at 25°C. 5 identical reactions were performed and pooled after 2 hours at 25°C. The pooled mixture was extracted with phenol/chloroform and DNA was recovered by ethanol precipitation. The DNA pellet was dissolved in 100 µl water and purified by gel filtrations on prepacked columns (G-50 Sephadex Nick columns, Pharmacia). The DNA was eluted in 400 µl water. The solution was lyophilised and redissolved in water (9 µl) to give a final concentration of 50 ng/µl. 1 µl of this solution was diluted to 50 ul with water (see Figure 39(b)).

Construction of vectorette library

Hind III Digestion

| | |
|---|---|
| 2 µl | PstI circles (100 ng) |
| 0.5 µl | 10X buffer |
| 1.5 µl | $H_2O$ |
| 1 µl | Hind III (2 units) |

The reaction mixture was incubated at 37°C for 30 minutes.

Ligation

The following were added to the restriction digest

| | |
|---|---|
| 1 µl | 10X buffer |
| 1 µl | 10 mM ATP |
| 1 µl | Annealed oligonucleotides 81 and 82 (0.25 pmole) or 81 and 83 (0.25 pmole) |
| 1 µl | ligase (2 units) |
| 0.5 µl | 10X Hind III buffer |

The mixture was incubated for 30 minutes at 37°C.
Annealed oligonucleotides (81/82 or 81/83 0.25 p mole) were added and the reaction was incubated for a further hour at 37°C. The volume of the reaction mixture was then adjusted to 100 µl with water (see Figure 39(c)).

1. The following were added to a 0.5 ml Sarstedt tube:
PstI circles (5 ng)
oligonucleotide 85 (100 pmole)
oligonucleotide 86 (100 pmole)
10 µl 10X PCR buffer
100 µM dNTPs
2 units Taq polymerase (Cetus)

The volume was adjusted to 100 µl with water and amplification was performed as described in Example 8. The predicted 710 bp product was obtained confirming that circles had been formed. The results are shown in Figure 40.

Lane 1 φX174 Hae III DNA marker
2 710 bp product of amplification between oligonucleotides 85 and 86.

2. The following were placed in a 0.5 ml Sarstedt tube:
10 ng PstI circle-Hind III vectorette-oligonucleotides 81/82
oligonucleotide 85 (100 pmole)
oligonucleotide 79 (100 pmole)
10 µl 10 x PCR buffer
100 µM dNTPs
2 units Taq polymerase (Cetus)

The volume was adjusted to 100 µl with water and amplification was performed as described in Example 8.
The predicted 720 bp product was obtained. The results are shown in Figure 40.

Lane 3 720 bp product of amplification between oligonucleotide 85 and 79.

3. The amplification reaction mixture described in (2) above was diluted by a factor of $10^7$. 1 µl of the diluted mixture was placed in a 0.5 ml Sarstedt tube.
The following were also added to the Sarstedt tube.
oligonucleotide 79 (100 pmole)
oligonucleotide 88 (100 pmole)
10 µl 10X PCR buffer
100 µM dNTPs
2 units Taq polymerase (Cetus)

The volume was adjusted to 100 µl and amplification was performed as described in Example 8. The

predicted 500 bp product was obtained. The results are shown in Figure 40.

Lane 4 500 bp product of amplification between oligonucleotides 79, 88.

4. The amplification product from (3) above was digested with PstI. The predicted products (340 bp, 160 bp) was obtained. The result is shown in Figure 40.

Lane 5 Products resulting from digestion of PCR product (Lane 4) with PstI. Products of 340 bp and 160 bp are obtained.

5. The following were place in a 0.5 ml Sarstedt tube.

10 ng PstI circle-Hind III vectorette-oligonucleotide 83/82
oligonucleotide 85 (100 pmole)
oligonucleotide 79 (100 pmole)
10 µl 10X PCR buffer
100 µM dNTPS
2 units Taq polymerase (Cetus).

The volume was adjusted to 100 µl with sterile, double distilled water and amplification was performed as described in Example 8. The predicted 720 bp product was obtained. The results are shown in Figure 40.

Lane 6 720 bp product of amplification between oligonucleotide 79 and 85.

6. The amplification reaction mixture described in (5) above was diluted by a factor of $10^5$. 1 µl of the diluted mixture was placed in a 0.5 ml Sarstedt tube. The following were also added to the Sarstedt tube.

oligonucleotide 79 (100 pmole)
oligonucleotide 88 (100 pmole)
10 µl 10X PCR buffer
100 µM dNTPS
2 units Taq polymerase (Cetus)

The volume was adjusted to 100 µl with sterile, double distilled water and amplification was performed as described in Example 8. The predicted 500 bp product was obtained. The results are shown in Figure 40.

lane 7 500 bp product of amplification between oligonucleotide 79, 88.

7. The amplification product from (6) above was digested with PstI. The predicted products (340 bp, 160 bP) were obtained. The results are shown in Figure 40.

Lane 8 Products resulting from digestion of PCR product (Lane 6) with PstI. Products of 340 bp and 160 bp are obtained.

8. The amplification reaction mixture described in (3) above was diluted by a factor of $10^7$. 1 µl of the diluted mixture was placed in a 0.5 ml Sarstedt tube. The following were added to the Sarstedt tube.

oligonucleotide 79
oligonucleotide 85
10 µl 10X PCR buffer
100 µM dNTPs
2 units Taq polymerase (Cetus)

The volume was adjusted to 100 µl with sterile, double distilled water and amplification was performed as described in Example 1. The predicted 720 bp product was obtained. The results are shown in Figure 40.

Lane 9 720 bp product of amplification between oligonucleotide 79, 85.

9. The amplification product from (8) above was digested with PstI. The predicted products (370 bp, 350 bp).

Lane 10 Products resulting from digestion of PCR product (Lane 9) with PstI.

Products of 370 bp and 350 bp were obtained.

Lane 11 ϕX174 Hae III DNA markers.

Sample from lane 3 of the agarose gel shown in Figure 40 was precipitated and loaded onto a fresh agarose gel, the photograph of which appears as Figure 41. The lanes of the photograph in Figure 41 are as follows:-

Lane 1 ϕX174 Hae III DNA marker
Lane 2 Sample form lane 3 of Figure 40
Lane 3 ϕX174 Hae III DNA marker
Lane 4 Amplification from lane 9 of Figure 40.

Example 25

Examples 3, 4, 5, 6 and 7 were repeated except that no dephosphorylation step was effected. The

results are set out in Figure 42 in which the lanes of the photograph of the agarose gel are as follows:-

Lane 1 $\phi$X174 Hae III DNA marker
Lane 2 the amplification product of Example 3(i)
Lane 3 the amplification product of Example 3(ii)
Lane 4 the amplification product of Example 4
Lane 5 the amplification product of Example 5
Lane 6 the amplification product of Example 6(ii)
Lane 7 the amplification product of Example 7(i)
Lane 8 this lane was omitted
Lane 9 $\phi$X174 Hae III marker.

## Claims

A method for the amplification of a nucleic acid fragment, comprising unknown sequence, by primer extension which method comprises cleaving a target nucleic acid to obtain target nucleic acid fragments, one of said fragments containing an initiating priming region of known nucleotide sequence for hybridisation with an initiating primer, preparing target nucleic acid fragment/vectorette units from the target nucleic acid fragments by ligation each unit having a vectorette priming region of known sequence for hybridisation with a vectorette primer, and treating the target nucleic acid fragment/vectorette units, together or sequentially, with appropriate nucleoside triphosphates and an agent for polymerisation of nucleoside triphosphates under hybridising conditions, such that an extension product of an initiating primer is synthesised complementary to a single stranded target nucleic acid/vectorette unit having an initiating priming region to which is hybridised an initiating primer selected so as to be substantially complementary to the initiating priming region, whereas no such extension product is synthesised complementary to single stranded target nucleic acid fragment/vectorette units having no such initiating priming region.

2. A method as claimed in claim 1 wherein the extension product is subjected to amplification in the presence of a vectorette primer which primer is selected so as to be substantially complementary to a vectorette priming region.

3. A method as claimed in claim 2 wherein the synthesis of vectorette primer amplification products is dependent upon the initial synthesis of an extension product of the initiating primer.

4. A method as claimed in claim 3 wherein the vectorette portion of the target nucleic acid fragment/vectorette unit comprises a double stranded portion having first and second strands, the second strand of the vectorette portion being ligated to that strand of the target nucleic acid fragment which contains the initiating priming region, and the nucleotide sequence of the first strand, second strand and vectorette primer being selected such that the vectorette primer is capable of hybridising to the complement of the second strand but not to the first strand under the same hybridisation conditions.

5. A method as claimed in claim 3 wherein the vectorette portion of the target nucleic acid fragment/vectorette unit comprises a double stranded portion having first and second strands, the first strand having a terminal polymerisation blocking moeity and the second strand, which in use is ligated to that strand of the target nucleic acid fragment containing the initiating priming region, carrying a single stranded portion, the terminal polymerisation blocking moiety being effective to prevent extension of the first strand to form a complement to the said single stranded portion of the second strand in the presence of appropriate nucleoside triphosphates and an agent for the polymerisation of the nucleoside triphosphates under hybridising conditions.

6. A method as claimed in any one of the preceding claims wherein a plurality of different vectorette libraries is prepared for use with the same single initiating primer, each vectorette library comprising target nucleic acid fragment/vectorette units obtained by ligation of nucleic acid fragments prepared by cleaving target nucleic acid at different cleavage sites; treating each vectorette library either separately or together with appropriate nucleoside triphosphates and an agent for polymerisation of nucleoside triphosphates under hybridising conditions whereby to obtain a plurality of initiating primer extension products, based on the use of the same single initiating primer.

7. A method as claimed in claim 1 or claim 2 wherein a nucleic acid having termini capable of ligation to each other is circularised, the nucleic acid containing a portion there of being capable of serving as an initiating priming region for hybridisation with an initiating primer; cleaving the circularised nucleic acid outside the known nucleotide sequenoe to form a linear molecule containing the known nucleotide sequence and having a known cleavage site pattern at at least one terminus for ligation to form a target nucleic acid fragment/vectorette unit; forming said target nucleic acid fragment/vectorette unit by ligation and treating the

target nucleic acid fragment/vectorette unit, together or sequentially, with initiating primer, appropriate nucleoside triphosphates and an agent for polymerisation of the nucleoside triphosphates under hybridising conditions.

8. A method as claimed in claim 7 wherein the target nucleic acid fragment/vectorette unit is treated, together or sequentially with initiating primer, vectorette primer, appropriate nucleoside triphosphates and an agent for polymerisation of the nucleoside triphosphates.

9. A method as claimed in any one of the preceding claims wherein the target nucleic acid fragment/vectorette units are prepared from target nucleic acid fragments by ligation such that the vectorette cannot be cleaved from the target nucleic acid fragment/vectorette units obtained therefrom by the same agent used to cleave the target nucleic acid to yield target nucleic acid fragments.

10. A method as claimed in claim 9 wherein the target nucleic acid is cleaved with a restriction endonuclease to yield a target nucleic acid fragment for ligation to a vectorette, the sequence of the vectorette being selected such that the restriction endonuclease recognition sequence of the said restriction endonuclease is absent in the target nucleic acid fragment/vectorette unit.

11. A method as claimed in any one of the preceding claims wherein any or all of the initiating primer extension products obtained is (are) sequenced at least at the end(s) distal to a given initiating primer so as to determine the sequence of a further initiating primer whereby to obtain further initiating primer extension products based on primer extension of the further initiating primer.

12. A method as claimed in any one of the preceding claims wherein an initiating primer extension product or portion thereof is sequenced whereby to characterise the said extension product or portion thereof

13. A method as claimed in claim 11 or claim 12 for the identification of a genotype responsible for a given phenotype wherein the sequence of nucleic acid containing the genotype is compared with the sequence of nucleic acid containing no such genotype whereby to identify the genotype responsible for the given phenotype.

14. A method as claimed in claim 13 wherein the comparison is effected by the use of a single sample of nucleic acid from an obligate heterozygote for the given phenotype.

15. A method as claimed in claim 11 or claim 12 for the identification of a genotype responsible for or contributory to a predisposition to a given phenotype if such be present wherein the sequences of nucleic acid from a plurality of individuals affected by the phenotype to be investigated are compared with the sequences of nucleic acid from a plurality of individuals in which no evidence of the phenotype to be investigated is present whereby to identify a genotype responsible for or contributory to a predisposition to the phenotype to be investigated if such be present.

16. A method as claimed in claim 15 wherein the sequence differences between 1) a pool of nucleic acids from a plurality of individuals affected by the phenotype to be investigated; and 2) a pool of nucleic acids from a plurality of individuals presenting no evidence of the phenotype to be investigated; are compared.

17. A kit for the amplification of a nucleic acid fragment of unknown sequence by primer extension, which kit comprises:-

1) means for cleaving a target nucleic acid at a specific site to obtain a target nucleic acid fragment;

2) a vectorette adapted for ligation to a target nucleic acid fragment obtained by use of the means for cleaving a target nucleic acid defined in (1) whereby to form in use a target nucleic acid fragment/vectorette unit said vectorette having a vectorette priming region of known sequence for hybridisation with a vectorette primer;

3) each of four different nucleoside triphosphates; and

4) an agent for polymerisation of the nucleoside triphosphates in (3).

18. A kit as claimed in claim 12 which additionally comprises a vectorette primer and if desired at least one nested primer, said primer(s) having a nucleotide sequence substantially complementary to a vectorette priming region of a target nucleic acid fragment/vectorette unit.

19. A kit as claimed in claim 17 or claim 18 wherein the vectorette adapted for ligation to a target nucleic acid fragment comprises a double stranded portion having first and second stands, the second strand of the vectorette being adapted for ligation to that strand of the nucleic acid fragment which contains the initiating priming region, and the nucleotide sequence of the first strand and second strand being selected such that in use a vectorette primer is capable of hybridising to the complement of the second strand but not to the first strand under the same hybridisation conditions.

20. A vectorette library kit for the amplification of a nucleic acid fragment of unknown sequence by primer extension, which kit comprises:-

1) at least one vectorette library each library comprising a set of target nucleic acid

fragment/vectorette units obtained from nucleotide sequences of an individual member of a species of animal, plant or microorganism; and

2) an initiating primer or primers for hybridisation to an initiating priming region of the target nucleic acid fragment/vectorette units.

21. A kit as claimed in claim 20 for the analysis of a genotype responsible for or contributory to a predisposition to a given phenotype wherein the set of target nucleic acid fragment/vectorette units comprises:-

1) a pool of target nucleic acid fragment/vectorette units from a plurality of individuals affected by the phenotype to be investigated; and

2) a pool of target nucleic acid fragment/vectorette units from a plurality of individuals presenting no evidence of the phenotype to be investigated.

Neu eingereicht / Newly filed
Nouvellement déposé

Fig.1.

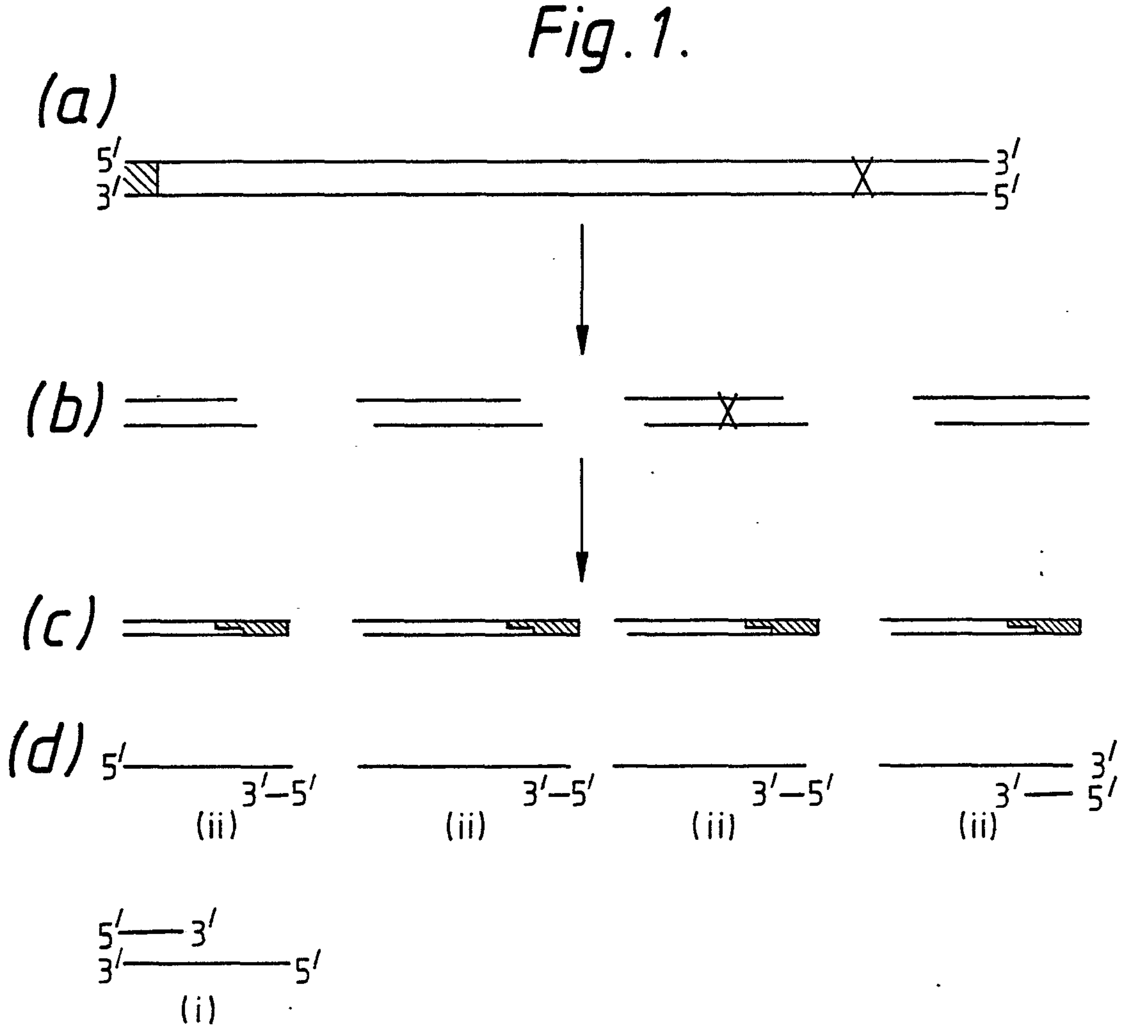

13.10.89

Neu eingereicht / New
Nouvellement dépo...

Fig. 2.

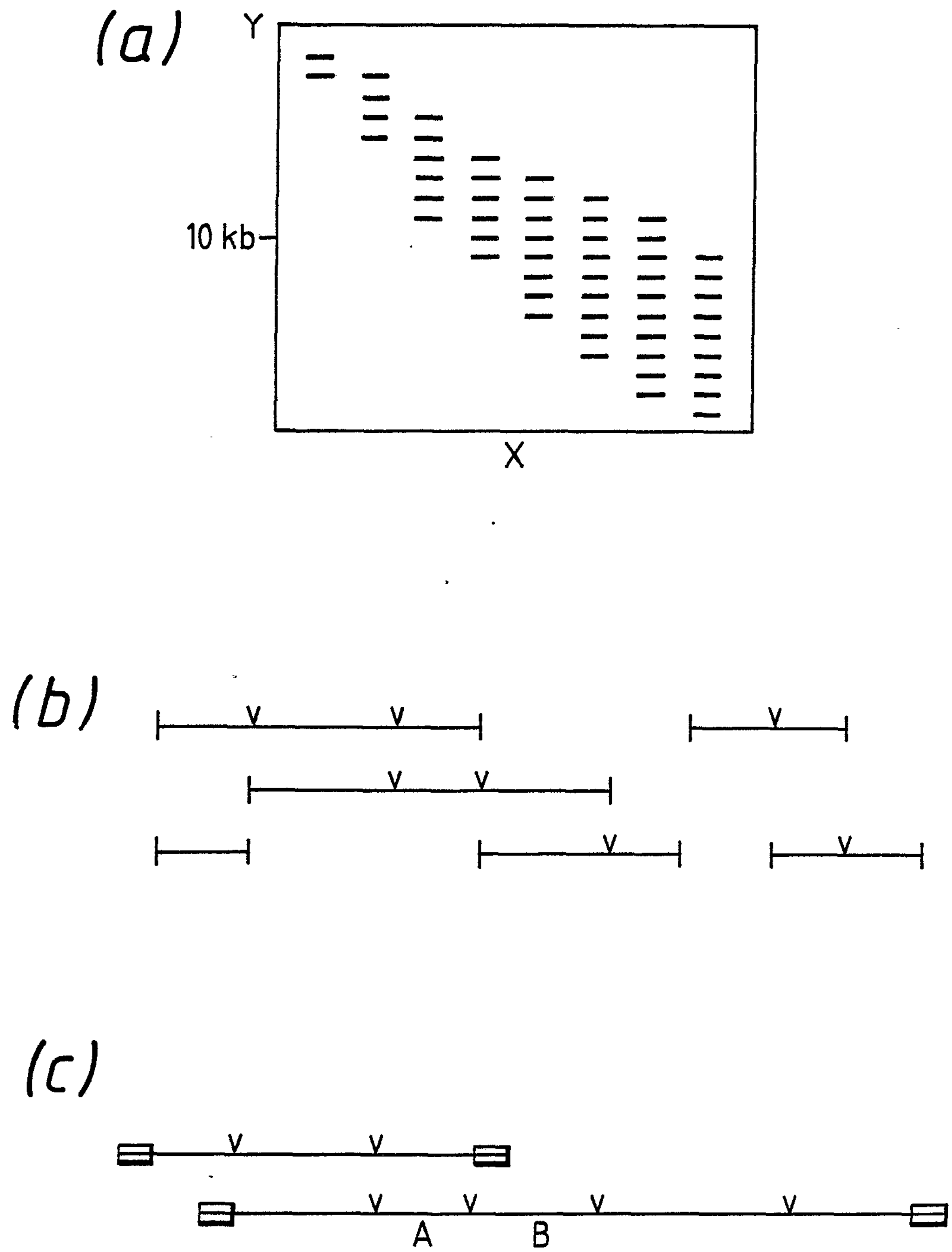

Neu eingereicht / Newl
Nouvellement dépo

Fig.3.

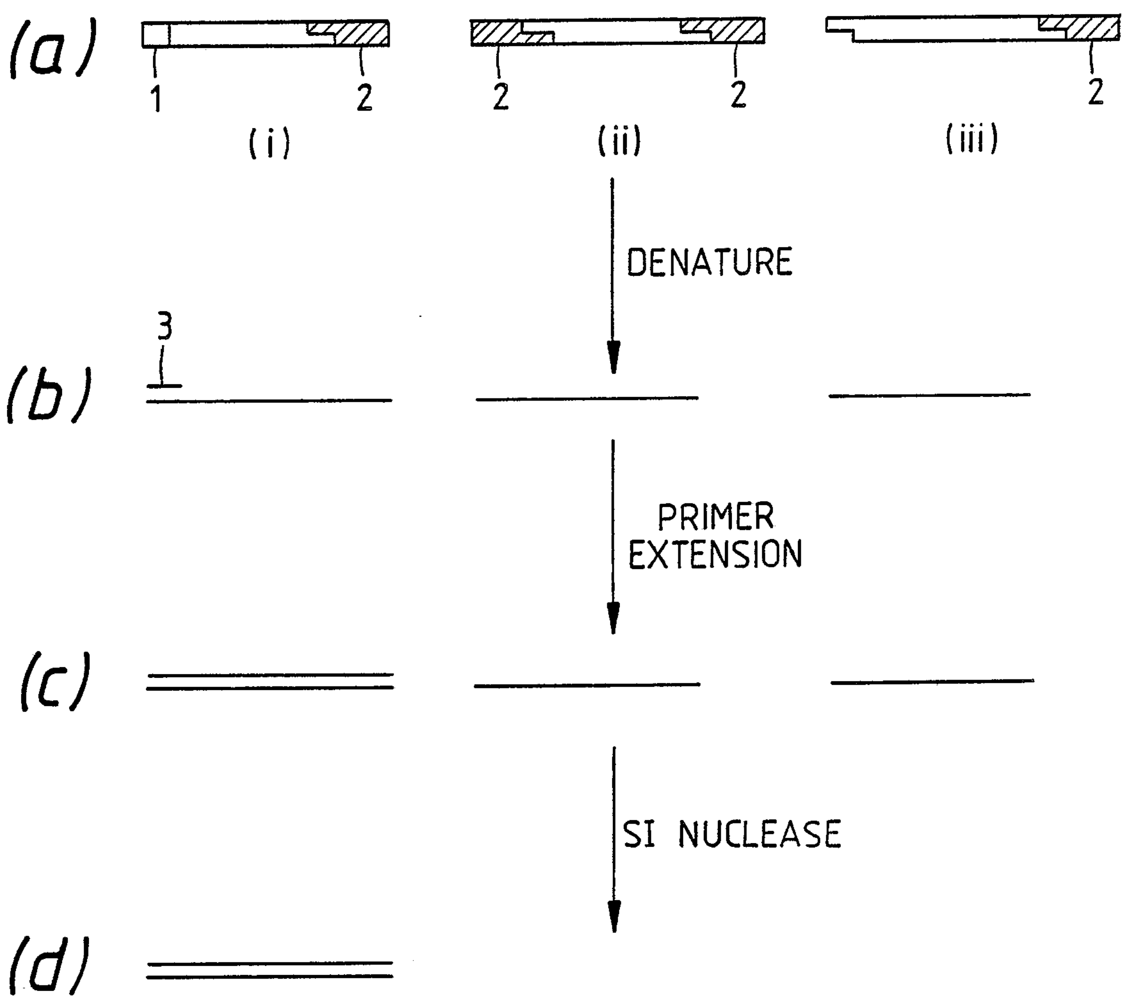

Neu eingereicht / Newly filed
Nouvellement déposé

(a) Fig.4.

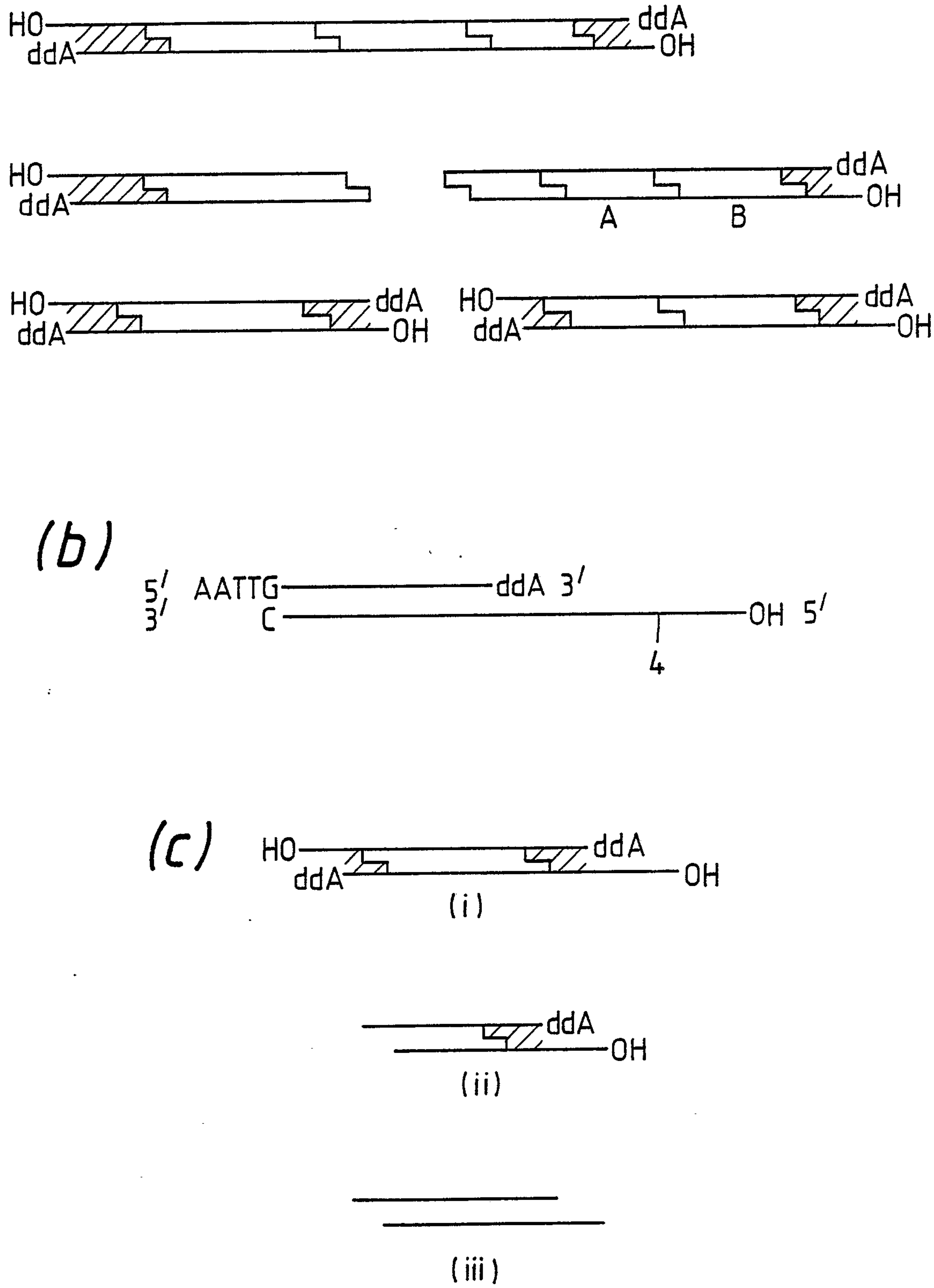

Neu eingereicht / Newly filed
Nouvellement déposé

# Fig. 5.

i) Blocking vectorette

5' $N^1N^2N^3N^4N^5$ AAGGAGAGGX 3'

||||||||||

3' $N^{5'}$ TTCCTCTCCTGTCGCTAAGAGCATGCTTGCCAATGCTAAGC 5'

ii) Non-complementary vectorette

GCTGTCTGTCGAAGGTAAGGAACGGACGA

5' $N^1N^2N^3N^4N^5$ AAGGAGAGGAC | | ||| | | | | || || GAGAAGGGAGAG 3'

||||||||||| | | ||| | | | | || || ||||||||||||

3' $N^{5'}$ TTCCTCTCCTG | | ||| | | | | || || CTCTTCCCTCTC 5'

TCGCTAAGAGCATGCTTGCCAATGCTAAG

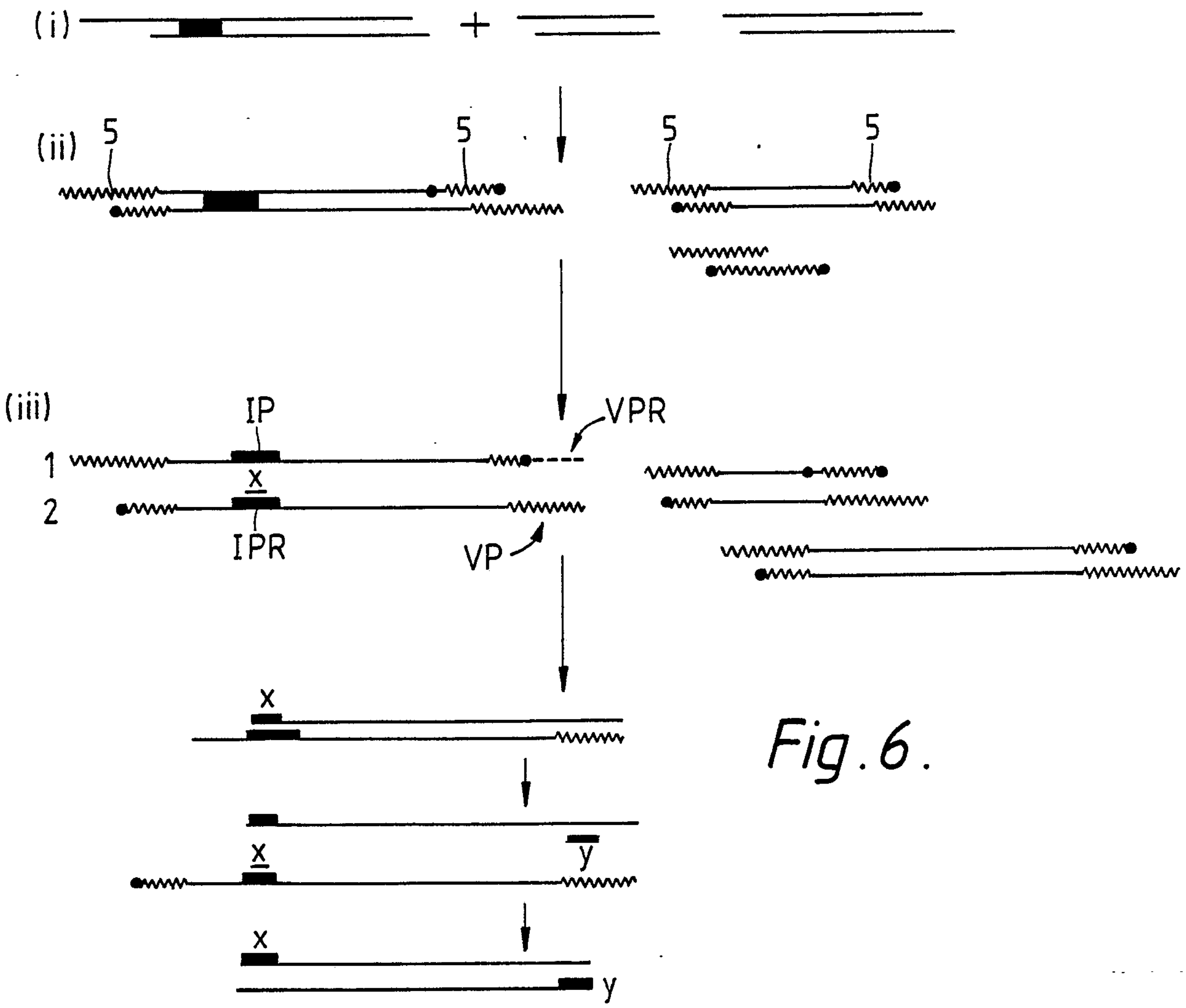


Fig. 6.

Neu eingereicht / Newly
Nouvellement déposé

Fig. 7.

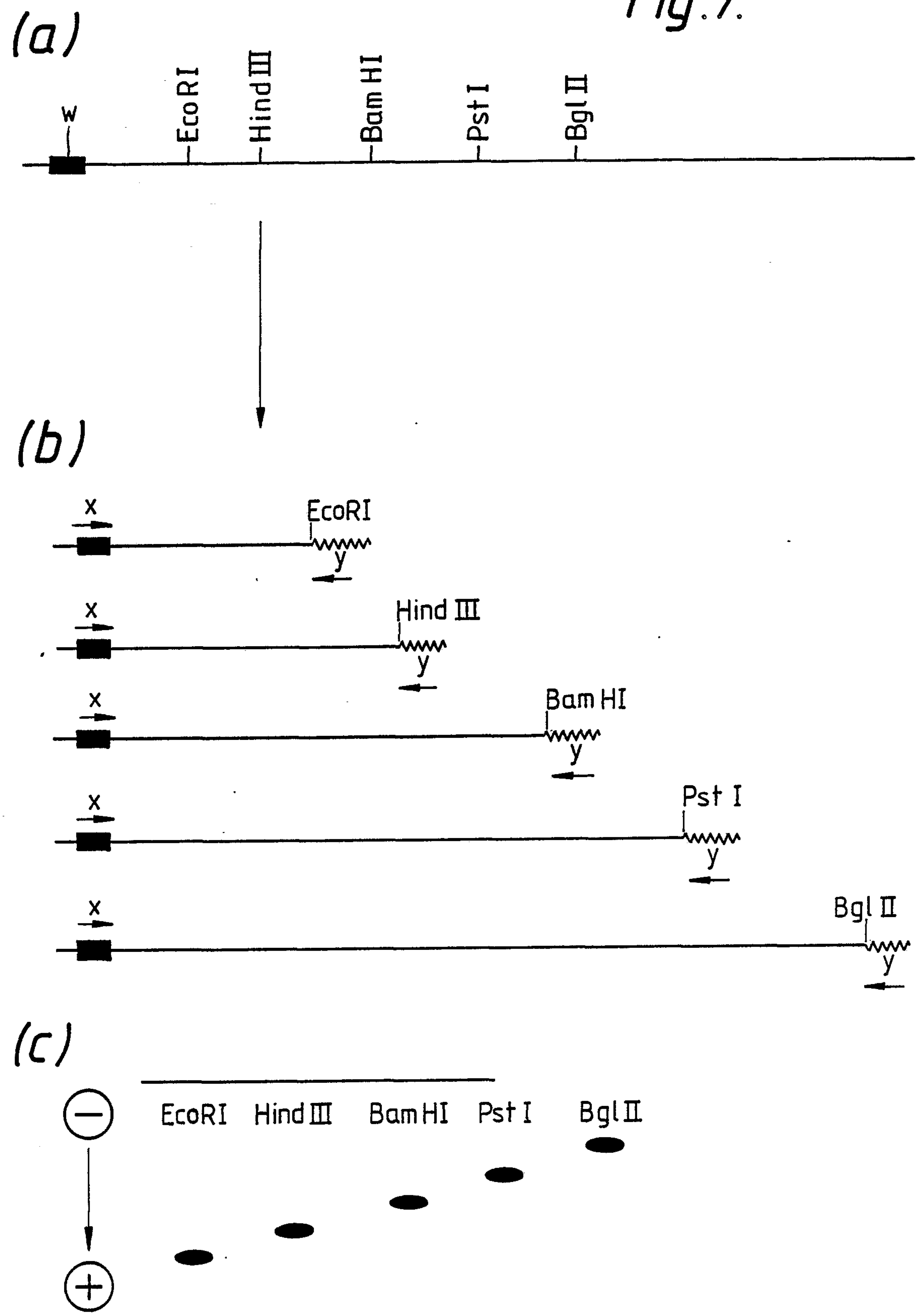

Neu eingereicht / Newl
Nouvellement dépo



Fig.8.

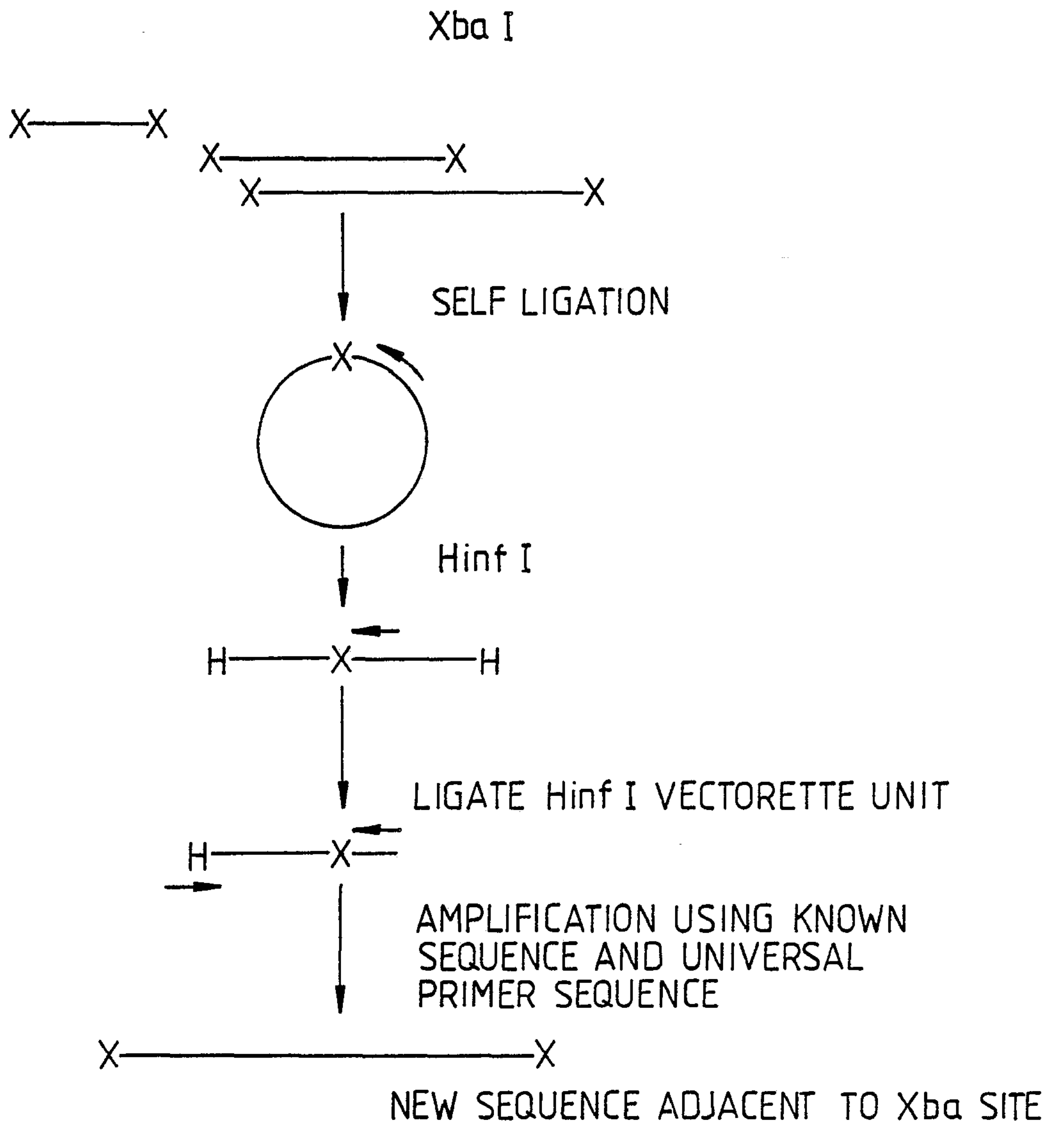


Fig.9.

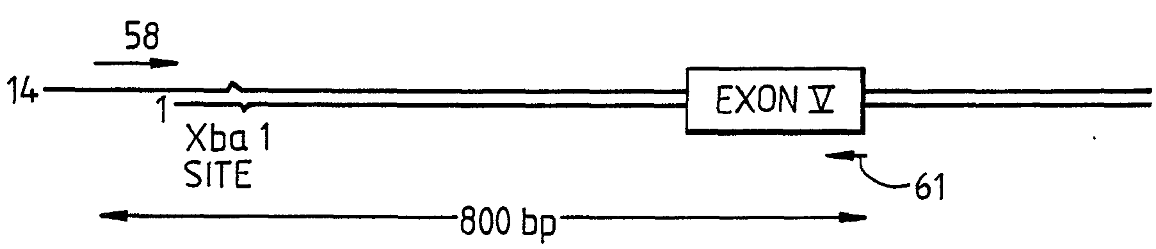

Fig. 10.

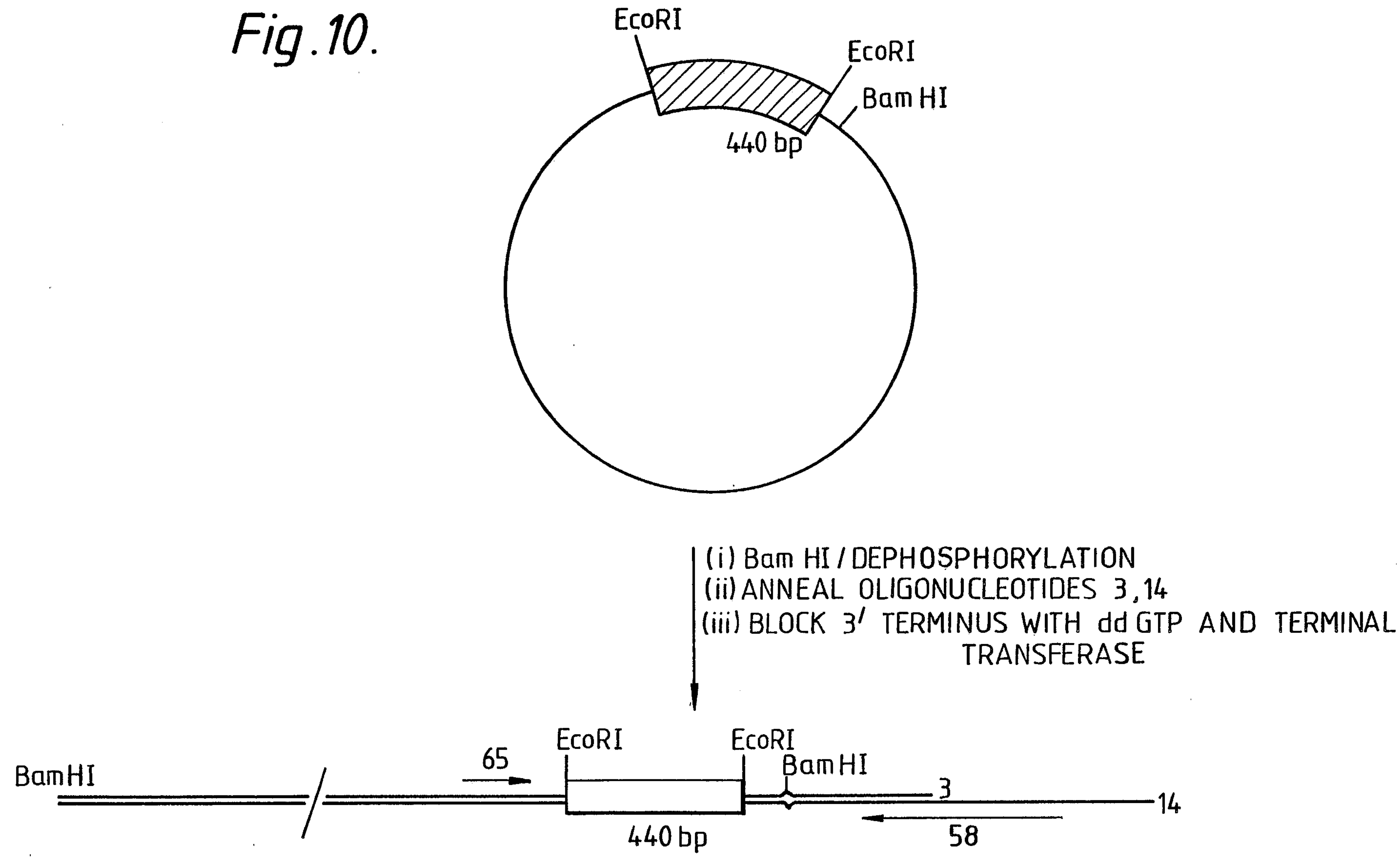
EcoRI
EcoRI
Bam HI
440 bp
(i) Bam HI / DEPHOSPHORYLATION
(ii) ANNEAL OLIGONUCLEOTIDES 3,14
(iii) BLOCK 3′ TERMINUS WITH dd GTP AND TERMINAL TRANSFERASE
BamHI
65
EcoRI
EcoRI
BamHI
3
14
440 bp
58

Fig. 11.

1 9

(i)

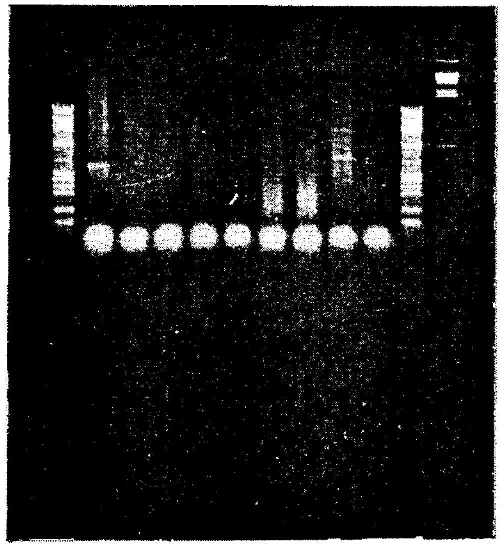

1 2 3 4 5 6 7 8 9 10 11 12

1 2 3 10 12 13

(ii)

(iii)

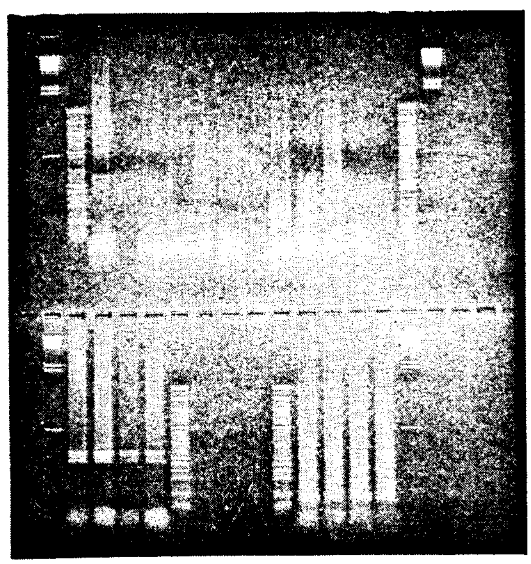

1 2 3 4 5 6 6 7 8 9 10 11

Neu eingereicht / Newl
Nouvellement dépo

Fig.12.

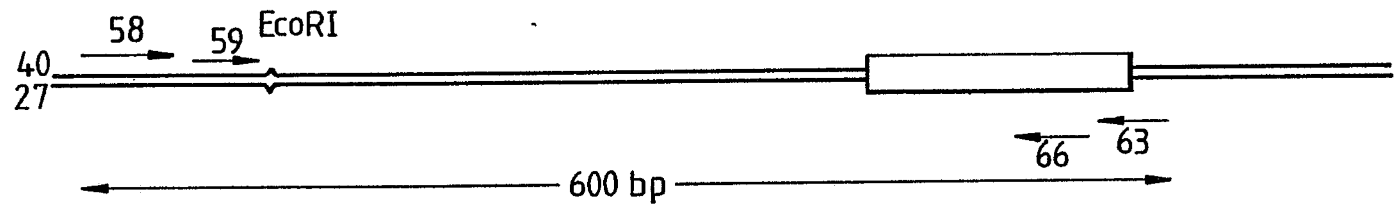


Fig.22.

```
           10          20          30          40          50
5' TGTAATTGGG  GGAAAATAGA  ACCTGTTCTG  TTCCTGTAAT  TGGAACCACA
           60          70          80          90         100
   GAACCAACCT  AGTACTTGGC  CATAAAAAGG  TGATGGGTGG  CCAGATGCCT
          110         120         130         140         150
   TCAGAACAGC  CCACATAGAC  CCTGAGTGTG  TTATCAAGTC  TTTCCTGCCC
          160         170         180         190         200
   ATATGTTATA  GAATCACAGA  TCTCCAGGGT  AGGGGGATCC  TTCCACCCTC
          210         220         230         240         250
   ACTTGGCCCT  TTGTTTTCAC  TTTATAAGTG  AGGAAATTGA  GGCACAGAGA
          260         270         280         290         300
   AGGGAAGTGA  CTTGCTCATG  ATCACAGGGT  CGGTTAGTAT  CTAAAGCTTC
          310         320         330         340         350
   TTCTCTTGAC  TCCAGCTCAG  TGATTATTCA  TCAGCAGAAC  ATTCCTGGAA
          360         370         380         390         400
   TGTTAAATGA  GTCTTAATCA  TGTACCCTGG  AAACATCACA  GGAGAACTGC
          410         420         430         440         450
   AGATAAGTGG  TGACATCGGT  CCAGGCATAT  ATATAGCTGG  AGAGTCTAAC  AC  3'
```

Neu eingereicht / New...
Nouvellement dépo...

Fig. 13.

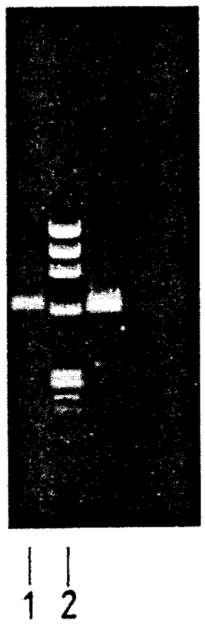


Fig. 14.

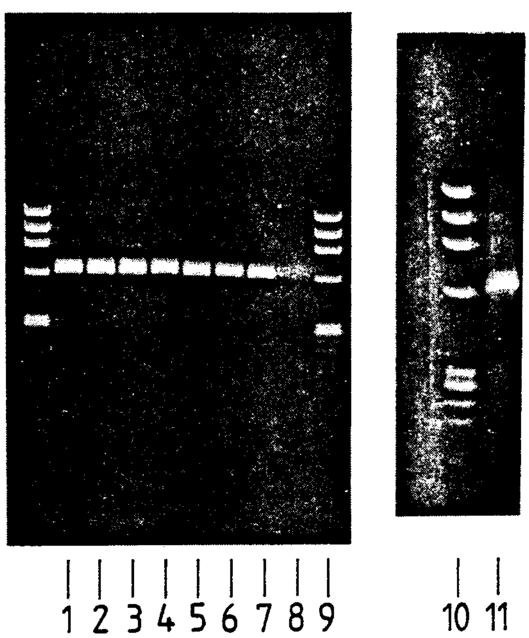

Neu eingereicht / Newly filed
Nouvellement déposé

13.10.89

Fig. 15.

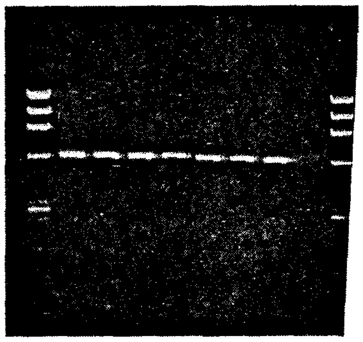

1 2 3 4 5 6 7 8 9 10

Fig. 16.

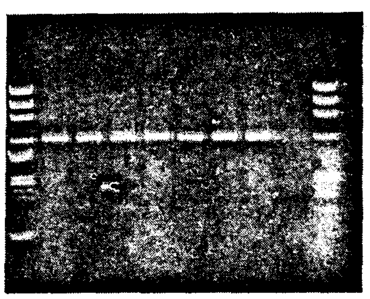

1 2 3 4 5 6 7 8 9 10

Neu eingereicht / Newly file
Nouvellement déposé

Fig. 17.

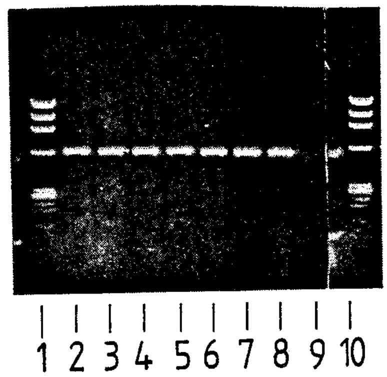


Fig. 18.

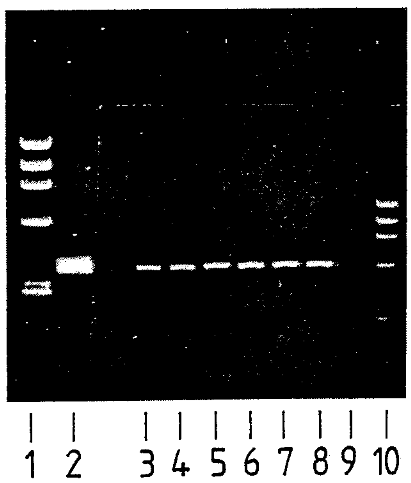


Fig. 19.

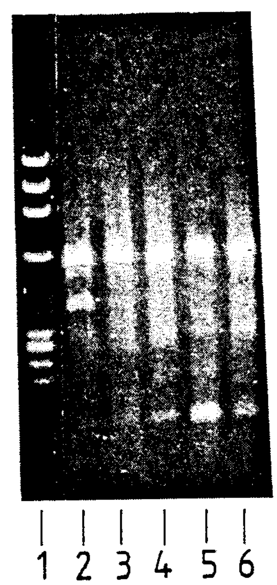

13·10·89

Neu eingereicht / Newly fi...
Nouvellement déposé

Fig. 20.

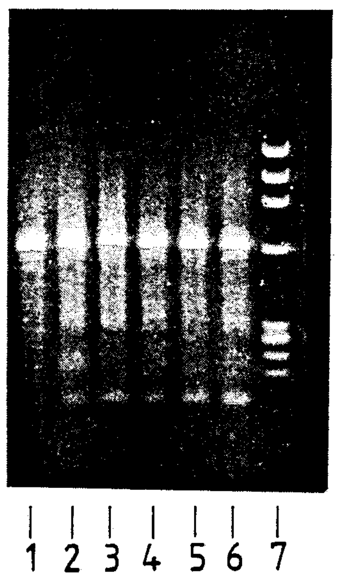

Neu eingereicht / Newly file
Nouvellement déposé



13-10-89

Fig. 21.

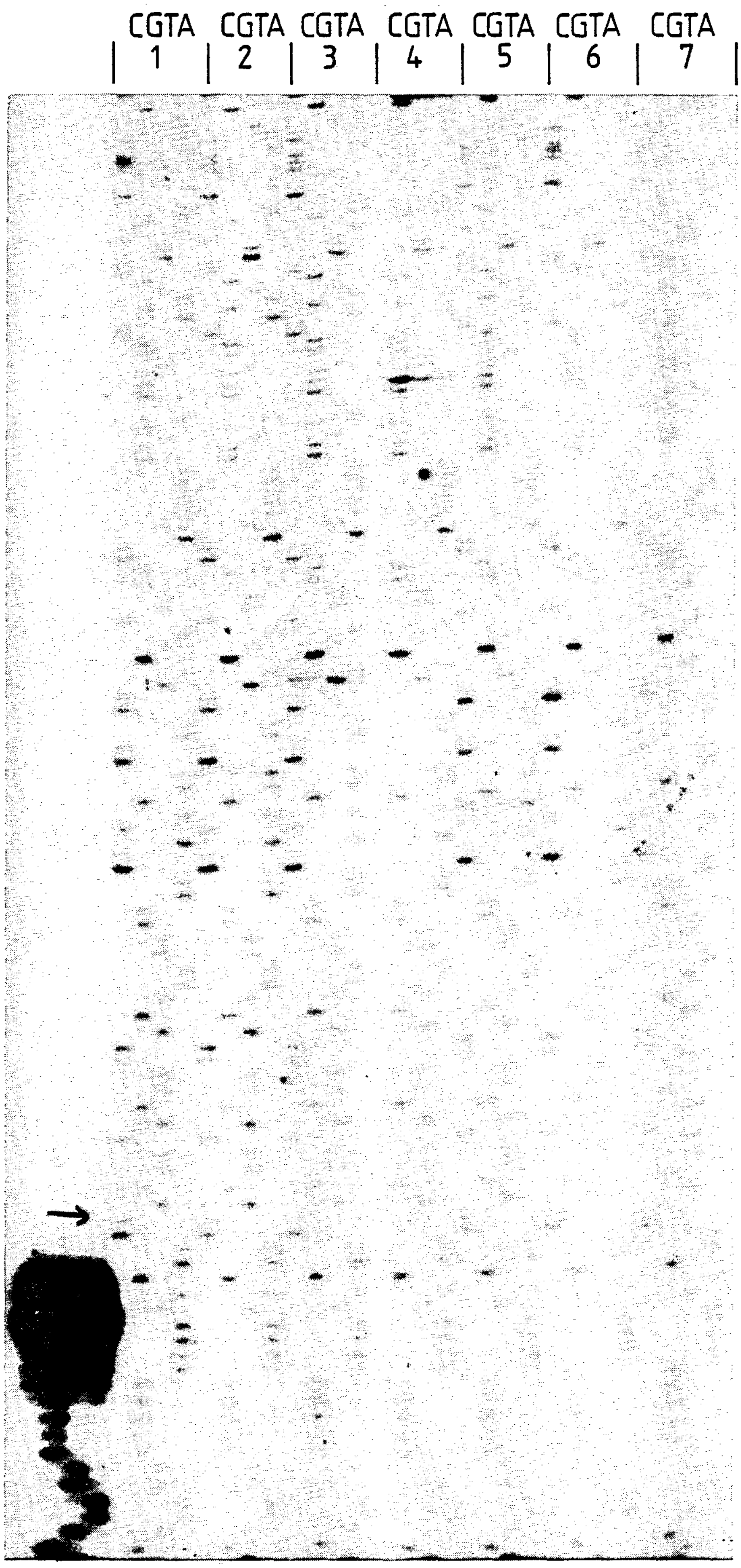

Neu eingereicht / Newly filed
Nouvellement déposé

Fig. 23.

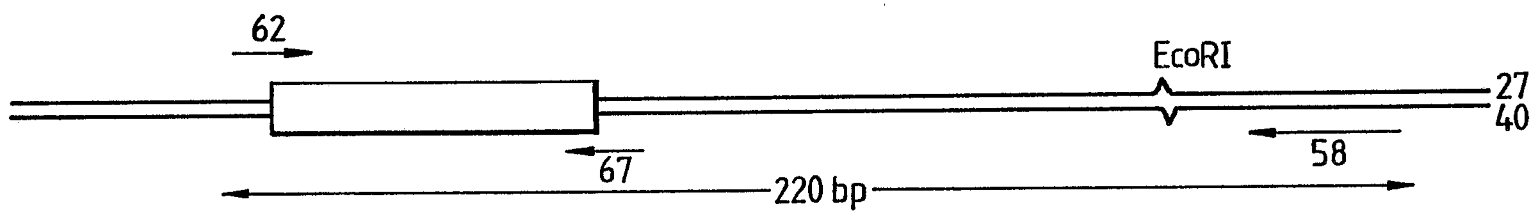


Fig. 25.

a) CTG GAA AAC CCA GGC TTG GGC AGG AAA CTC TCT GAC TTT GGA CAG GTG AGC
CAC GGC AGC CTG AGC TGT CAG TTA GGG GAA TTT GGG CCT CCA GAG AAA GAG ATC
CGA AGA CTG CTG GTG CTT CTG GTT TAA TAA

b) GGG TTT TCC AGG ACC GCA GTG GAC ATG CTG GCT CCC CGG GAG TGA GGT CTC
TGG CTT T

c) —AAA GCCAG AGACC TCACT$^{20}$ CCCGG GGAGC CAGCA TGTCC$^{40}$ ACTGC

GGTCC TGGAA AACCC$^{60}$ AGGCT TGGGC AGGAA ACTCT$^{80}$ CTGAC TTTGG

ACAGG TGAGC$^{100}$ CACGG CAGCC TGAGC TGTCA$^{120}$ GTTAG GGGAA TTTGG

GCCTC$^{140}$ CAGAG AAAGA GATCC GAAGA$^{160}$ CTGCT GGTGC TTCTG

GTTTA$^{180}$ ATAA – – – – – etc.

Neu eingereicht / Newly file
Nouvellement déposé

03.10.89

Fig. 24.

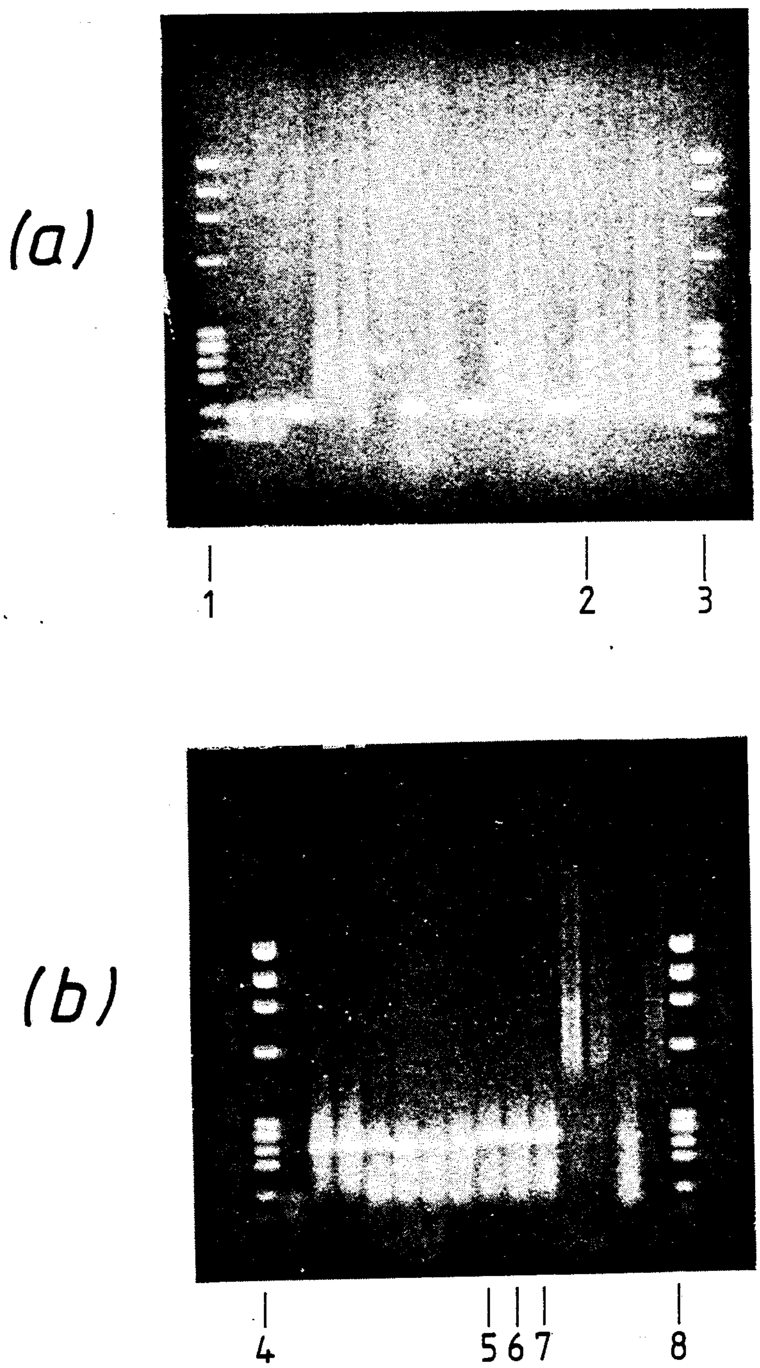

Fig.26.

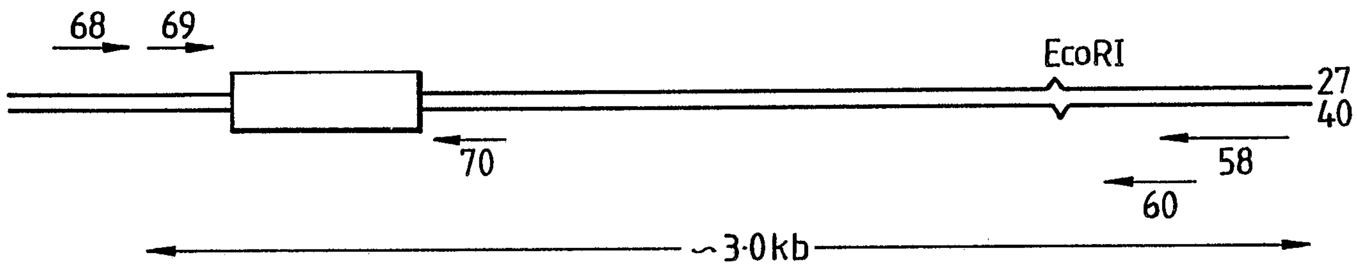


Fig.31.

```
 HindIII              HinfI                 HaeIII
 AAGCTTTGAT GTCATCCTTG ACTCTTCTCT TNNTCAAAGG CCACATCTAA TCAGTTAGCA AACCCTGACT
1 ---------+ ---------+ ---------+ ---------+ ---------+ ---------+ ---------+ 70
 TTCGAAACTA CAGTAGGAAC TGAGAAGAGA ANNAGTTTCC GGTGTAGATT AGTCAATCGT TTGGGACTGA
            <<<<<<<<< Oligo 73    <<<<<<<<<<<

  GCACTATTTC CAAAATATAA ACAGAATTTG ACCACTCCTG TCAATGTATC CA
71 ---------+ ---------+ ---------+ ---------+ ---------+ --  122
  CGTGATAAAG GTTTTATATT TGTCTTAAAC TGGTGAGGAC AGTTACATAG GT
```

3-10-89

Neu eingereicht / Newly filed
Nouvellement déposé

Fig. 27.

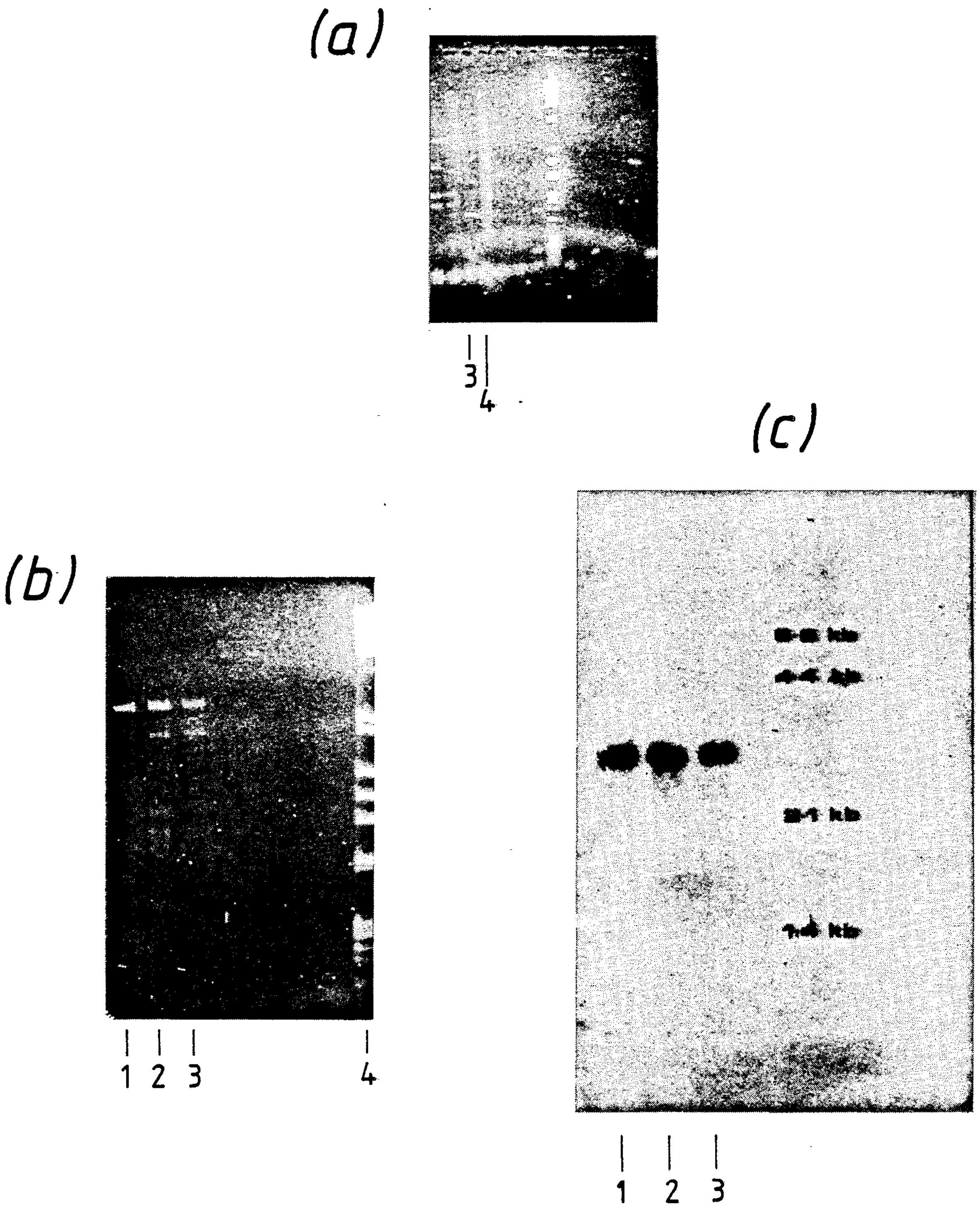

Neu eingereicht / Newly filed
Nouvellement déposé



# Fig. 28.

```
1     ATGCCTGCAG  GTCGACTCTA  GAGGATTCCC  CTAGAGCATA  TAAAATTATT
51    TTCAAGGGAA  GATGTAAAAA  TAGGTATGAA  GAAGTTCTGG  TACTTTTTTC
101   CCCACCCAGC  AGATCACTGT  TTTTTTTTTT  TTTTTTTTTT  TTTTTTTTTT
151   TATCACTTGA  GTGTTATGCA  CTGCTCTTTA  AAGATTCCTG  CTCTCAGGAG
201   CACCTAAGGG  TAGAATAGGA  ACTGGCACTT  CCATGGCCAG  GACATGCCTT
251   CTGGAGGTGC  TTATGGAGAT  GGACTGGAGT  CTGGGTCTTG  GAAAGTAGGC
301   ATGTTTGATG  GGCCAGTTTT  ACATTAAGTT  CTGTAGTCTC  ATTGCATCTG
351   GCACCCTCAA  ATTTCTTATT  AAATACTACA  GCCTGGTTTT  TAGCTTTGTT
401   CCATAGTAGG  AAAAGTTTGA  GCATAGGAAA  AGTTCTGTGC  CCAAAAATTGC
                                   71
451   ATCCAAGAAT  TGGGTACCGA  GCTC                        72
```

# Fig. 33.

```
1     AGATGCTGTA  CACATAAAAG  GACAAGCTGA  ACTCCTGCAA  CTCACTTAAC

                  HinfI                                HinfI
51    GAGAACTATT  AGAATCGGCA  CATTTGAACA  GACAAGTCGC  TGAATCCAAG

101   ATGAAGCCTT  TTATTTTTAA  GAAAAAAATG  GCCTTTACAT  CACCGACAAA
151   TTAGGTTCAG  ATGAAAAAGG  CTGTTTC
```

Neu eingereicht / Newly filed
Nouvellement déposé



*Fig. 29.*

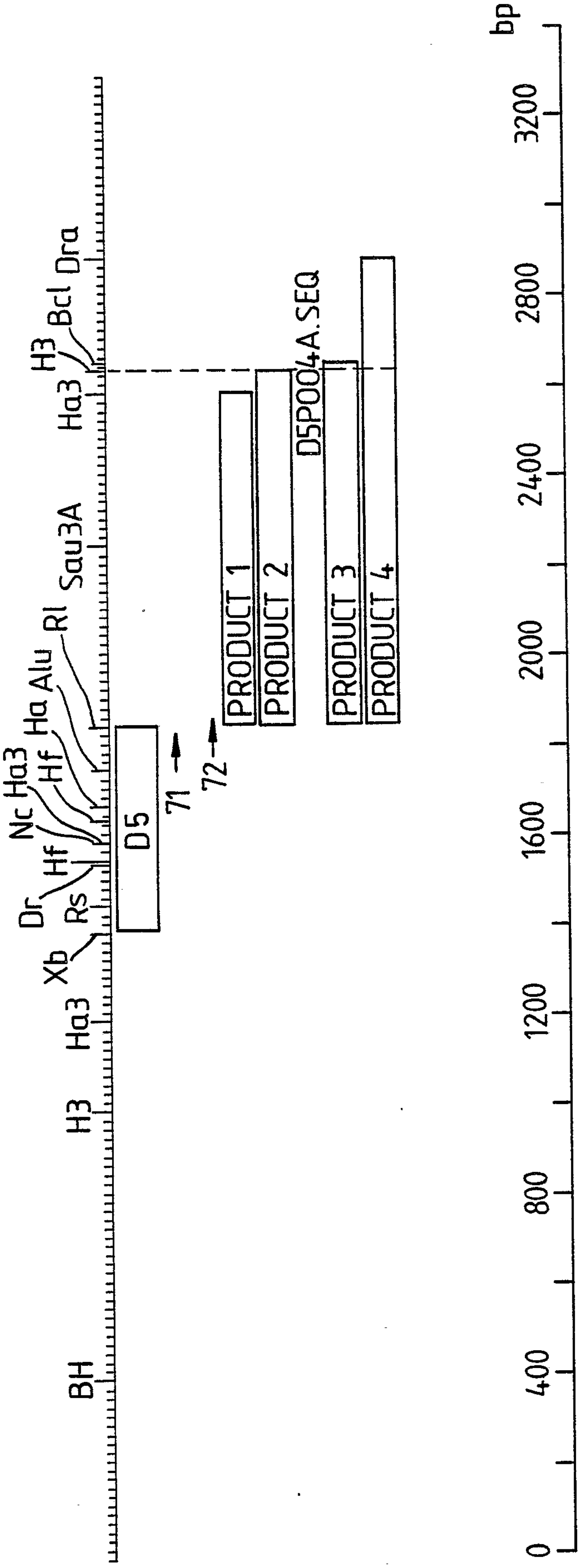

Neu eingereicht / Newly filed
Nouvellement déposé

Fig. 30.

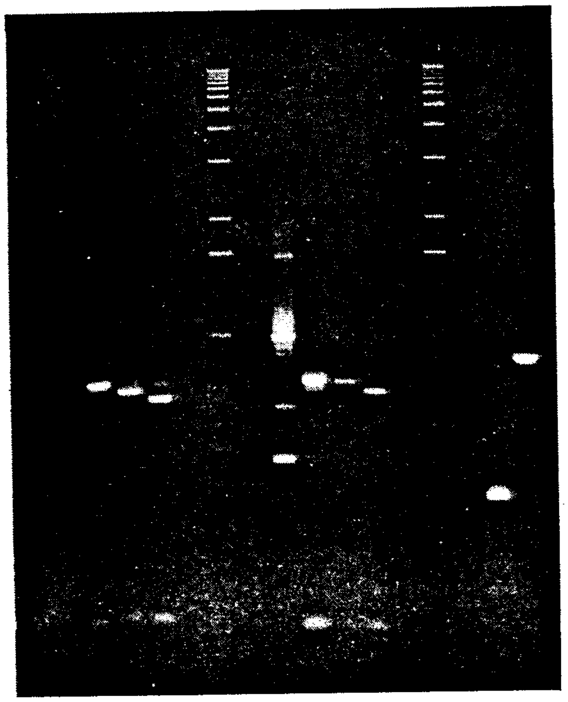


Fig. 32.

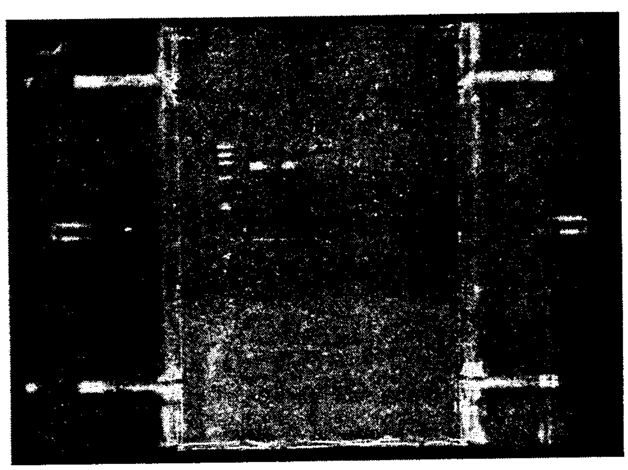

Neu eingereicht / Newly filed
Nouvellement déposé

Fig. 34.

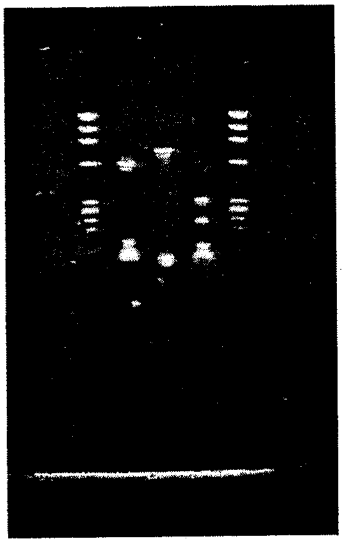

1 2 3 4 5

Fig. 35.

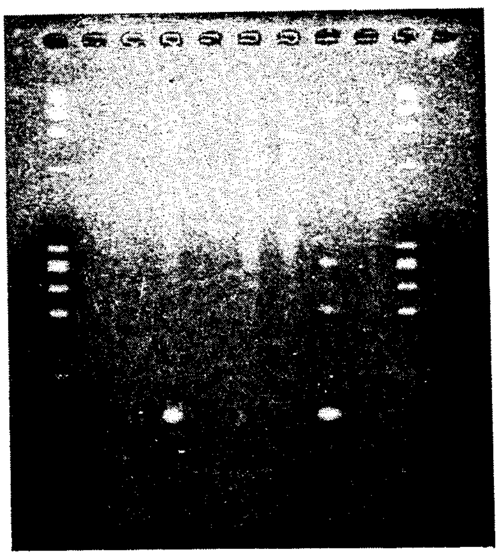

1 2 3 4 5

# Fig. 36.

(a)

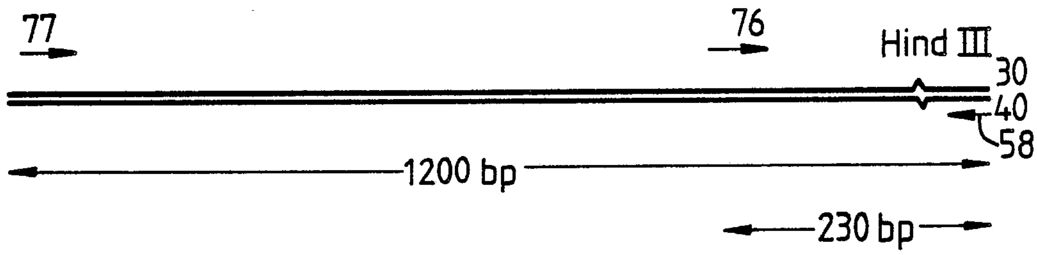


(b)

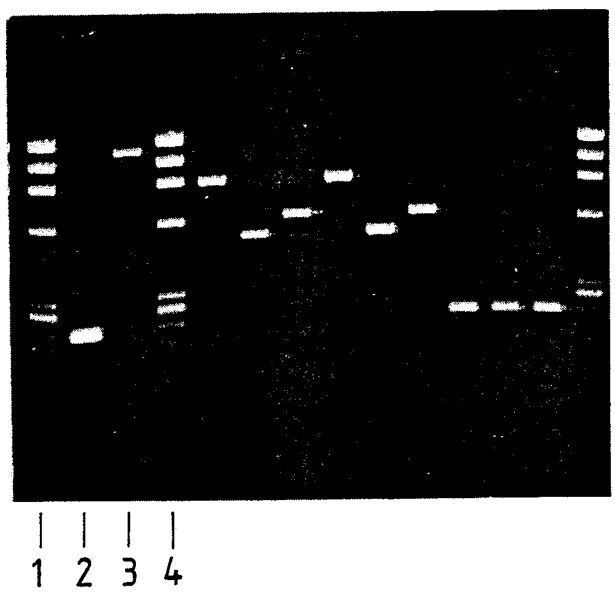

13.10.89

Neu eingereicht / Newly filed
Nouvellement déposé

Fig. 37.

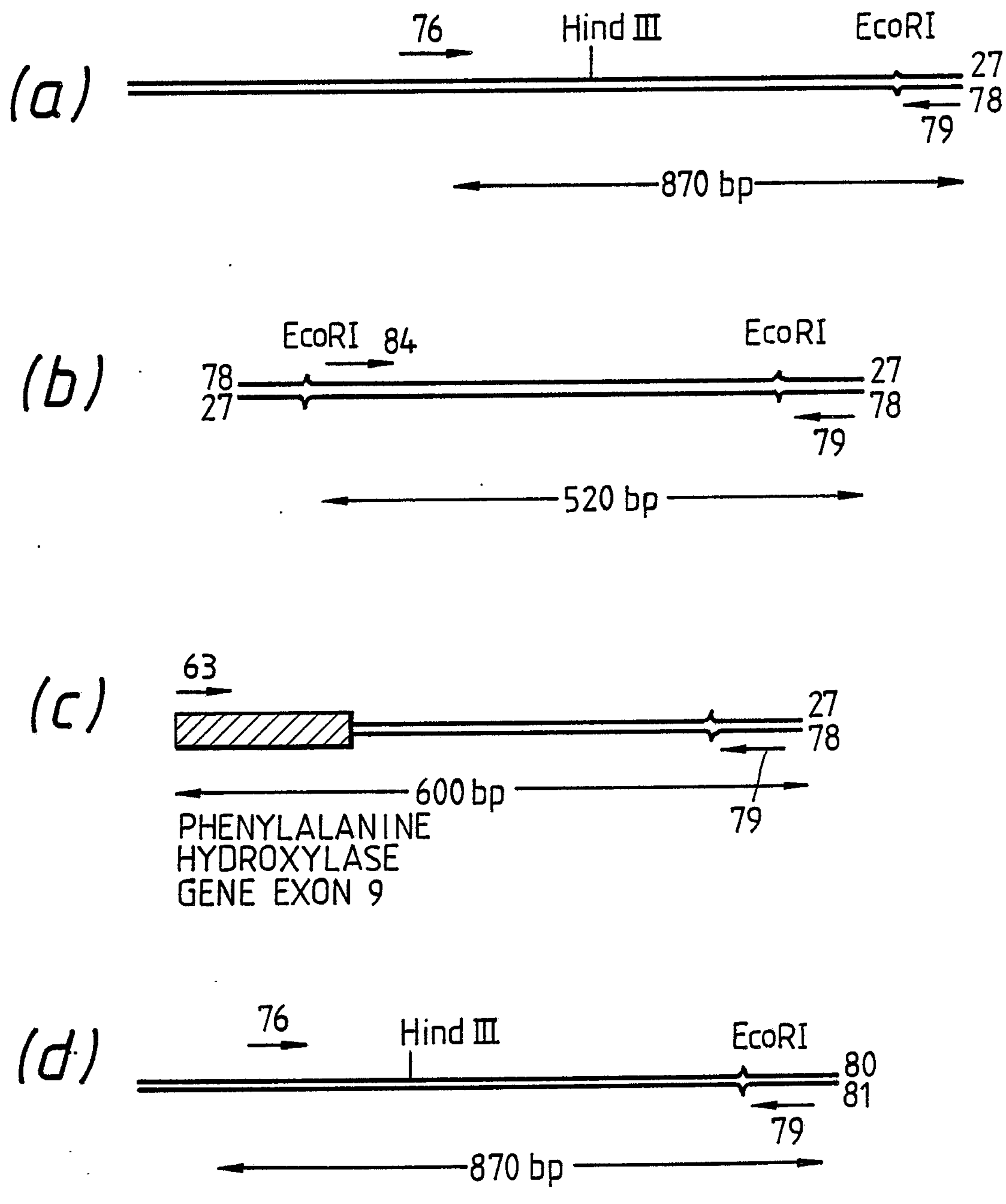

Neu eingereicht / Newly filed
Nouvellement déposé

Fig. 37.

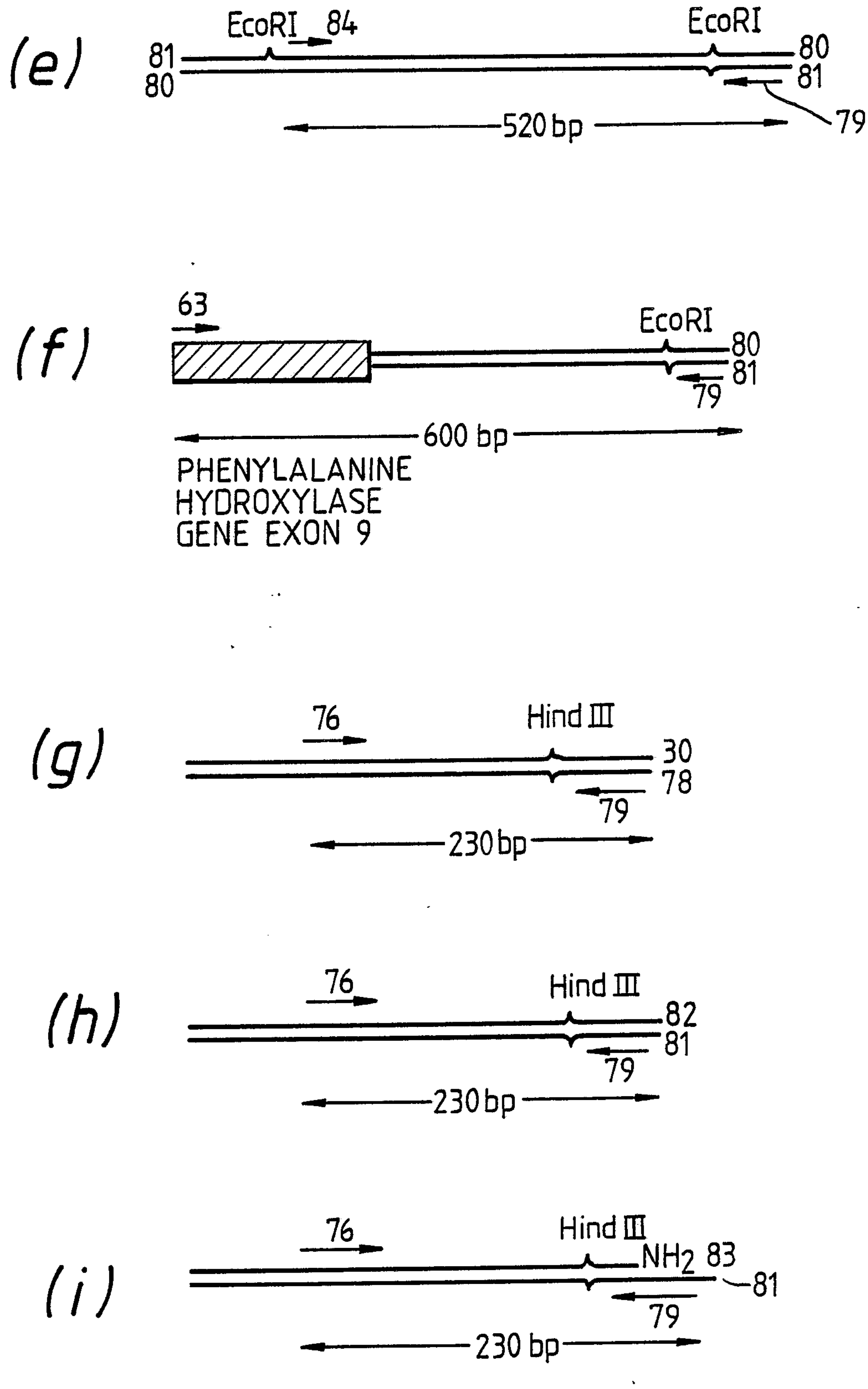

Neu eingereicht / Newly filed
Nouvellement déposé

Fig. 38.

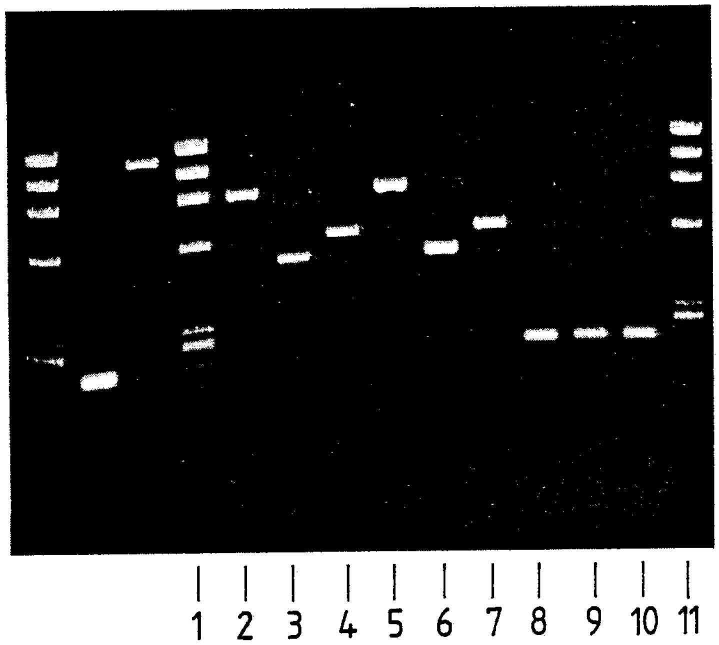


Fig. 40.

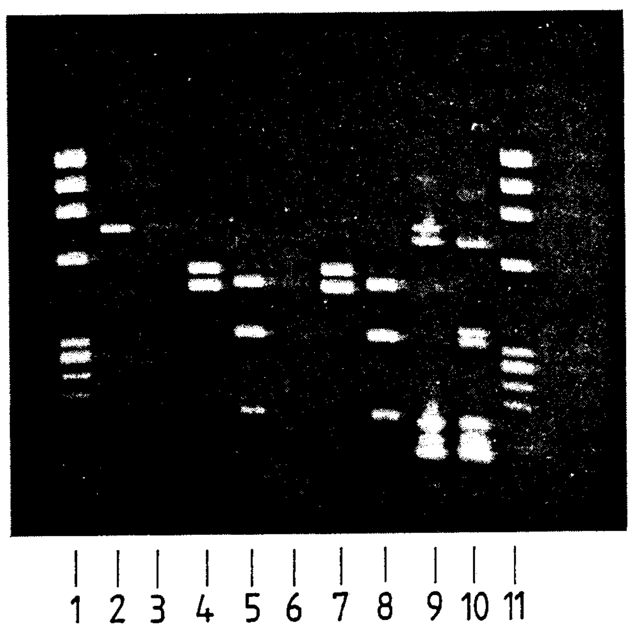

Neu eingereicht / Newly filed
Nouvellement déposé

Fig. 39.

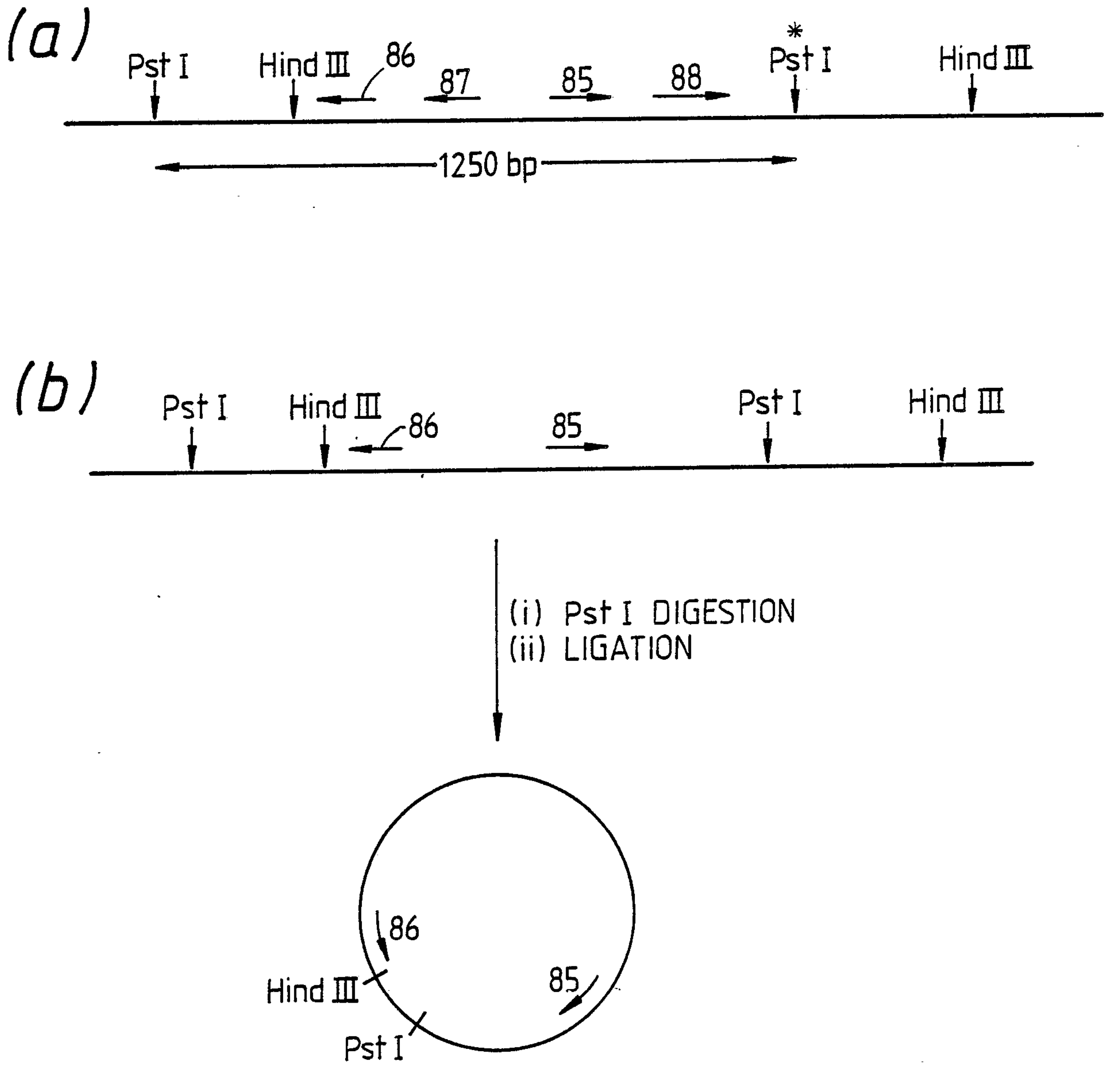

Neu eingereicht / Newly fi.
Nouvellement déposé

Fig. 39.

(c)

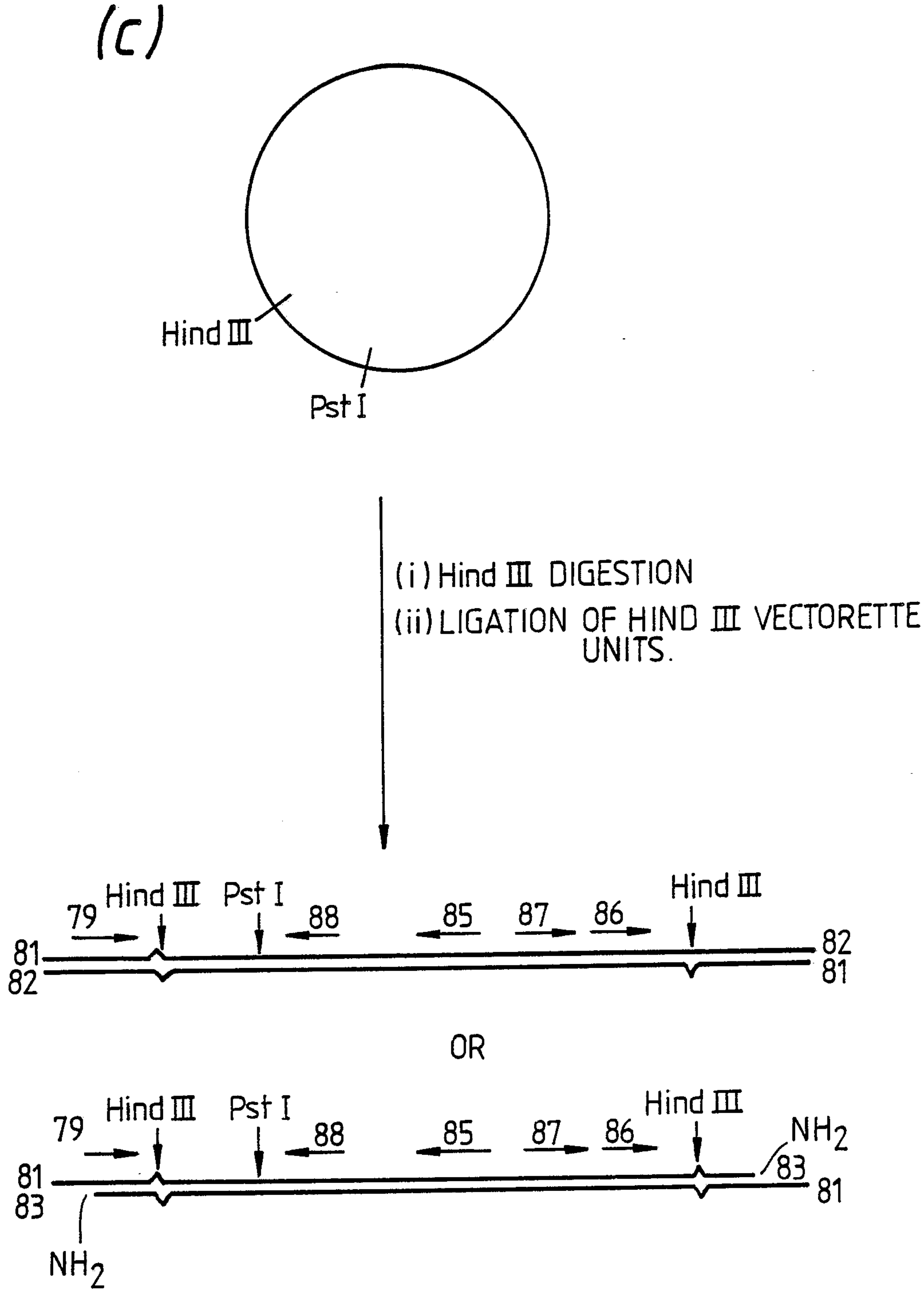

Neu eingereicht / Newly file
Nouvellement déposé

Fig. 41.

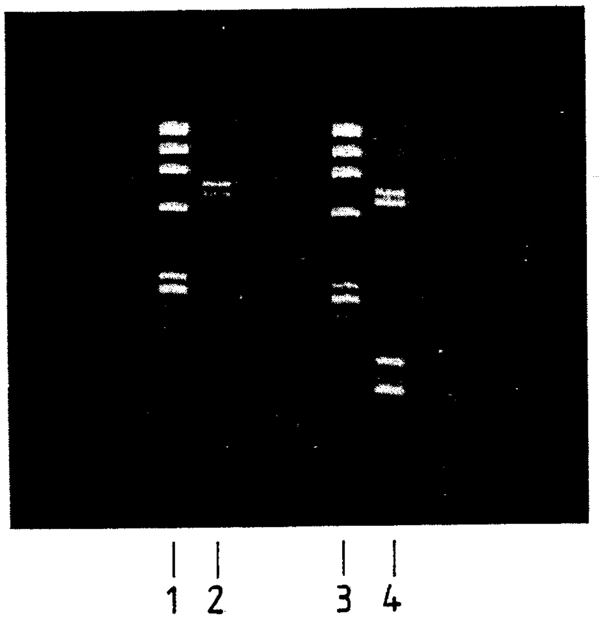


Fig. 42.